(19) Europäisches Patentamt
European Patent Office
Office européen des brevets

(11) EP 4 559 919 A1

(12) EUROPEAN PATENT APPLICATION
published in accordance with Art. 153(4) EPC

(43) Date of publication:
**28.05.2025 Bulletin 2025/22**

(21) Application number: **23842442.8**

(22) Date of filing: **21.07.2023**

(51) International Patent Classification (IPC):
***C07D 498/14*** (2006.01) ***C07D 491/18*** (2006.01)
***A61K 31/5383*** (2006.01) ***A61K 31/5386*** (2006.01)
***A61P 35/00*** (2006.01)

(52) Cooperative Patent Classification (CPC):
**A61K 31/5383; A61K 31/5386; C07D 491/18; C07D 498/14;** A61P 35/00

(86) International application number:
**PCT/CN2023/108708**

(87) International publication number:
**WO 2024/017380 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.07.2022 CN 202210872155**
**03.11.2022 CN 202211369098**
**13.12.2022 CN 202211601752**
**08.03.2023 CN 202310219452**
**16.05.2023 CN 202310562000**

(71) Applicant: **Medshine Discovery Inc.**
**Nanjing, Jiangsu 210032 (CN)**

(72) Inventors:
- **YU, Tao**
  **Shanghai 200131 (CN)**
- **WANG, Lu**
  **Shanghai 200131 (CN)**
- **LIU, Ning**
  **Shanghai 200131 (CN)**
- **ZHANG, Yang**
  **Shanghai 200131 (CN)**
- **CHEN, Shuhui**
  **Shanghai 200131 (CN)**

(74) Representative: **Dehns**
**10 Old Bailey**
**London EC4M 7NG (GB)**

# (54) TRICYCLIC-CONTAINING MACROCYCLIC COMPOUND AND USE THEREOF

(57) Disclosed in the present invention are a tricyclic-containing macrocyclic compound and a use thereof, and particularly disclosed are a compound as shown in formula (II) and a pharmaceutically acceptable salt thereof.

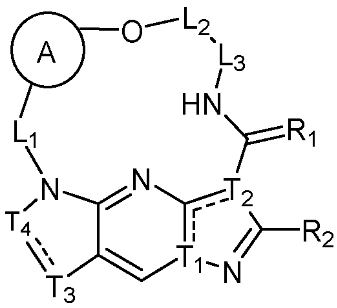

( II )

EP 4 559 919 A1

## Description

SPECIFICATION

The present application claims the right of the following priorities: CN2022108721559, application date: July 22, 2022; CN2022113690989, application date: November 3, 2022; CN2022116017524, application date: December 13, 2022; CN2023102194528, application date: March 8, 2023; CN202310562000X, application date: May 16, 2023.

TECHNICAL FIELD

The present disclosure relates to a tricyclic-containing macrocyclic compound and a use thereof, specifically to a compound of formula (II) and a pharmaceutically acceptable salt thereof.

BACKGROUND

Kinases are associated with proliferation, metabolism, differentiation, and apoptosis of cells, and abnormal activation of various kinases will lead to the occurrence of cancer.

Anaplastic lymphoma kinase (ALK) is a receptor tyrosine kinase (RTK), which belongs to the insulin receptor family. It is expressed primarily in the central and peripheral nervous systems, playing a role in the development and function of the nervous system.

ALK was originally found in a class of anaplastic large-cell lymphoma (ALCL). The N-terminus of the normally expressed protein nuclear phosphate NPM is fused with the ALK kinase domain to form a fusion protein NPM-ALK, and chromosomal translocation leads to continuous activation of cells, and finally forms a tumor. Currently, a variety of ALK fusion proteins have been identified. Among them, the EML4-ALK fusion protein is found in 3-7% NSCLC patients and is considered as a potent factor driving tumor, so EML4-ALK is established as a target that can effectively treat cancer.

Currently, a variety of ALK inhibitors have entered clinical trials and are approved for marketing. Among them, Crizotinib and Lorlatinib have been approved by the FDA for the treatment of ALK-positive NSCLC patients. Unfortunately, while ALK inhibitors have been proved to be effective in the initial clinic, ALK acquired resistance and brain metastases (metastatic brain cancer) can occur in treated patients. Mutations of ALK (EML4 -ALK-L1196M, EML4 -ALK-L1198F, EML4 -ALK-G1202R, EML4 -ALK-G1202 R-L1198F, and EML4-ALK-G1202 R-L1196M, etc.) are the main reasons for the drug resistance mechanism of ALK at present stage.

Therefore, for the treatment of NSCLC patients, there is an urgent need for a new generation of inhibitors that can overcome the acquired drug resistance of existing ALK inhibitors.

CONTENT OF THE PRESENT INVENTION

The present disclosure provides a compound of formula (II) or a pharmaceutically acceptable salt thereof,

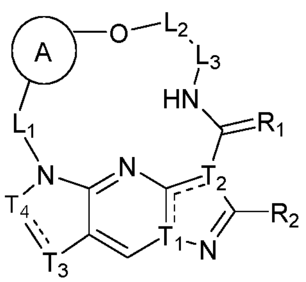

(II)

,

wherein

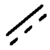 is selected from a single bond and a double bond;

$T_1$ is selected from C, and $T_2$ is selected from N;

or, $T_1$ is selected from N, and $T_2$ is selected from C;

$T_3$ is selected from O, N, NH, C(=O), $C(R_3)_2O$, $C(R_3)$, and $C(R_3)_2$;

$T_4$ is selected from N, NH, C(=O), $C(R_3)_2O$, $C(R_3)$, and $C(R_3)_2$;

$L_1$ is selected from $CH_2$ and C(=O);

$L_2$ and $L_3$ are each independently selected from a single bond and $C_{1-3}$ alkyl, and the $C_{1-3}$ alkyl is optionally substituted with 1, 2, or 3 $R_a$;
$R_1$ is selected from O, S, and $NR_4$;
$R_2$ is selected from H, F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
$R_3$ is selected from H, F, Cl, Br, I, and $C_{1-3}$ alkyl, and the $C_{1-3}$ alkyl is optionally substituted with 1, 2, or 3 $R_b$;
$R_4$ is selected from OH, CN, and $-(OCH_2CH_2)_n-C_{1-3}$ alkoxy;
n is 0 or 1;
ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;
each $R_a$ and each $R_b$ are independently selected from H, F, Cl, Br, I, and OH;
or, two $R_a$ on the same carbon atom are connected to form a $C_{3-6}$ cycloalkyl group or a 4- to 6-membered heterocycloalkyl group, and the $C_{3-6}$ cycloalkyl and the 4- to 6-membered heterocycloalkyl are each independently and optionally substituted with 1, 2, or 3 halogens;
or, two $R_a$ on different carbon atoms are connected to form a $C_{3-6}$ cycloalkyl group, and the $C_{3-6}$ cycloalkyl is optionally substituted with 1, 2, or 3 halogens;
each $R_c$ is independently selected from H, F, Cl, Br, I, and $C_{1-3}$ alkyl, and the $C_{1-3}$ alkyl is optionally substituted with 1, 2, or 3 halogens;
each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;
provided that: when $T_4$ is selected from $C(R_3)_2$, $T_3$ is selected from $C(R_3)_2O$, $T_2$ is selected from C, $T_1$ is selected from N, $L_1$ is selected from $CH_2$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
"hetero" in the 5- to 6-membered heteroaryl or 4- to 6-membered heterocycloalkyl represents 1, 2, or 3 heteroatoms or heteroatom groups independently selected from O, NH, S, S(=O), N, and C(=O).

The present disclosure provides a compound of formula (II) or a pharmaceutically acceptable salt thereof,

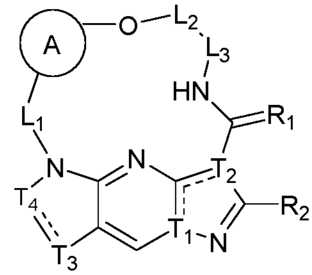

(II)

,

wherein

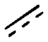 is selected from a single bond and a double bond;
$T_1$ is selected from C, and $T_2$ is selected from N;
or, $T_1$ is selected from N, and $T_2$ is selected from C;
$T_3$ is selected from O, N, NH, C(=O), $C(R_3)_2O$, $C(R_3)$, and $C(R_3)_2$;
$T_4$ is selected from N, NH, C(=O), $C(R_3)_2O$, $C(R_3)$, and $C(R_3)_2$;
$L_1$ is selected from $CH_2$ and C(=O);
$L_2$ and $L_3$ are each independently selected from a single bond and $C_{1-3}$ alkyl, and the $C_{1-3}$ alkyl is optionally substituted with 1, 2, or 3 $R_a$;
$R_1$ is selected from O, S, and $NR_4$;
$R_2$ is selected from H, F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
$R_3$ is selected from H, F, Cl, Br, I, and $C_{1-3}$ alkyl, and the $C_{1-3}$ alkyl is optionally substituted with 1, 2, or 3 $R_b$;

$R_4$ is selected from OH, CN, and $-(OCH_2CH_2)_n-C_{1-3}$ alkoxy;

n is 0 or 1;

ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;

each $R_a$ and each $R_b$ are independently selected from H, F, Cl, Br, I, and OH;

or, two $R_a$ on the same carbon atom are connected to form a $C_{3-6}$ cycloalkyl group or a 4- to 6-membered heterocycloalkyl group, and the $C_{3-6}$ cycloalkyl and the 4- to 6-membered heterocycloalkyl are each independently and optionally substituted with 1, 2, or 3 halogens;

or, two $R_a$ on different carbon atoms are connected to form a $C_{3-6}$ cycloalkyl group, and the $C_{3-6}$ cycloalkyl is optionally substituted with 1, 2, or 3 halogens;

each $R_c$ is independently selected from H, F, Cl, Br, I, and $C_{1-3}$ alkyl, and the $C_{1-3}$ alkyl is optionally substituted with 1, 2, or 3 halogens;

each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;

provided that: when $T_4$ is selected from $C(R_3)_2$, $T_3$ is selected from $C(R_3)_2O$, $T_2$ is selected from C, $T_1$ is selected from N, $L_1$ is selected from $CH_2$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl are independently and optionally substituted with 1, 2, or 3 $R_d$;

"hetero" in the 5- to 6-membered heteroaryl represents 1, 2, or 3 heteroatoms or heteroatom groups independently selected from O, NH, S, S(=O), N, and C(=O).

The present disclosure further provides a compound of formula (II) or a pharmaceutically acceptable salt thereof,

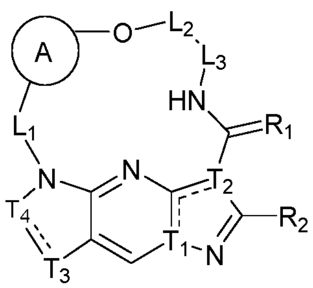

(II) ,

wherein

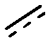 is selected from a single bond and a double bond;

$T_1$ is selected from C, and $T_2$ is selected from N;

or, $T_1$ is selected from N, and $T_2$ is selected from C;

$T_3$ is selected from O, N, NH, C(=O), $C(R_3)_2O$, $C(R_3)$, and $C(R_3)_2$;

$T_4$ is selected from N, NH, C(=O), $C(R_3)_2O$, $C(R_3)$, and $C(R_3)_2$;

$L_1$ is selected from $CH_2$ and C(=O);

$L_2$ and $L_3$ are each independently selected from $C_{1-3}$ alkyl, and the $C_{1-3}$ alkyl is optionally substituted with 1, 2, or 3 $R_a$;

$R_1$ is selected from O, S, and $NR_4$;

$R_2$ is selected from H, F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl, are each independently and optionally substituted with 1, 2, or 3 $R_d$;

$R_3$ is selected from H, F, Cl, Br, I, and $C_{1-3}$ alkyl, and the $C_{1-3}$ alkyl is optionally substituted with 1, 2, or 3 $R_b$;

$R_4$ is selected from OH, CN, and $-(OCH_2CH_2)_n-C_{1-3}$ alkoxy;

n is 0 or 1;

ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;

each $R_a$ and each $R_b$ are independently selected from H, F, Cl, Br, I, and OH;

or, two $R_a$ on the same carbon atom are connected to form a $C_{3-6}$ cycloalkyl group or a 4- to 6-membered heterocycloalkyl group, and the $C_{3-6}$ cycloalkyl and the 4- to 6-membered heterocycloalkyl are each independently and optionally substituted with 1, 2, or 3 halogens;

each $R_c$ is independently selected from H, F, Cl, Br, I, and $C_{1-3}$ alkyl, and the $C_{1-3}$ alkyl is optionally substituted with 1, 2,

or 3 halogens;

each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;

provided that: when $T_4$ is selected from $C(R_3)_2$, $T_3$ is selected from $C(R_3)_2O$, $T_2$ is selected from C, $T_1$ is selected from N, $L_1$ is selected from $CH_2$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl are independently and optionally substituted with 1, 2, or 3 $R_d$;

"hetero" in the 5- to 6-membered heteroaryl represents 1, 2, or 3 heteroatoms or heteroatom groups independently selected from O, NH, S, S(=O), N, and C(=O).

The present disclosure further provides a compound of formula (II) or a pharmaceutically acceptable salt thereof,

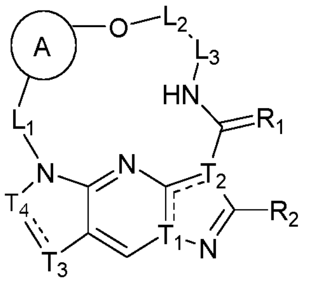

( II ) ,

wherein

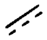 is selected from a single bond and a double bond;

$T_1$ is selected from C, and $T_2$ is selected from N;

or, $T_1$ is selected from N, and $T_2$ is selected from C;

$T_3$ is selected from O, N, NH, C(=O), $C(R_3)_2O$, $C(R_3)$, and $C(R_3)_2$;

$T_4$ is selected from N, NH, C(=O), $C(R_3)_2O$, $C(R_3)$, and $C(R_3)_2$;

$L_1$ is selected from $CH_2$ and C(=O);

$L_2$ and $L_3$ are each independently selected from $C_{1-3}$ alkyl, and the $C_{1-3}$ alkyl is optionally substituted with 1, 2, or 3 $R_a$;

$R_1$ is selected from O, S, and $NR_4$;

$R_2$ is selected from H, F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl, are each independently and optionally substituted with 1, 2, or 3 $R_d$;

$R_3$ is selected from H, F, Cl, Br, I, and $C_{1-3}$ alkyl, and the $C_{1-3}$ alkyl is optionally substituted with 1, 2, or 3 $R_b$;

$R_4$ is selected from OH, CN, and $-(OCH_2CH_2)_n-C_{1-3}$ alkoxy;

n is 0 or 1;

ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;

each $R_a$ and each $R_b$ are independently selected from H, F, Cl, Br, I, and OH;

or, two $R_a$ on the same carbon atom are connected to form a $C_{3-6}$ cycloalkyl group, and the $C_{3-6}$ cycloalkyl is optionally substituted with 1, 2, or 3 halogens;

each $R_c$ is independently selected from H, F, Cl, Br, I, and $C_{1-3}$ alkyl, and the $C_{1-3}$ alkyl is optionally substituted with 1, 2, or 3 halogens;

each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;

provided that: when $T_4$ is selected from $C(R_3)_2$, $T_3$ is selected from $C(R_3)_2O$, $T_2$ is selected from C, $T_1$ is selected from N, $L_1$ is selected from $CH_2$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl are independently and optionally substituted with 1, 2, or 3 $R_d$;

"hetero" in the 5- to 6-membered heteroaryl represents 1, 2, or 3 heteroatoms or heteroatom groups independently selected from O, NH, S, S(=O), N, and C(=O).

The present disclosure further provides a compound of formula (II) or a pharmaceutically acceptable salt thereof,

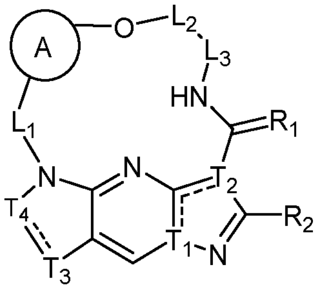

( II )

,

wherein

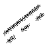 is selected from a single bond and a double bond;
$T_1$ is selected from C, and $T_2$ is selected from N;
or, $T_1$ is selected from N, and $T_2$ is selected from C;
$T_3$ is selected from O, N, NH, C(=O), $C(R_3)_2O$, $C(R_3)$, and $C(R_3)_2$;
$T_4$ is selected from N, NH, C(=O), $C(R_3)_2O$, $C(R_3)$, and $C(R_3)_2$;
$L_1$ is selected from $-CH_2-$ and-C(=O)-;
$L_2$ and $L_3$ are each independently selected from $C_{1-3}$ alkyl, and the $C_{1-3}$ alkyl is optionally substituted with 1, 2, or 3 $R_a$;
$R_1$ is selected from O, S, and $NR_4$;
$R_2$ is selected from H, F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl, are each independently and optionally substituted with 1, 2, or 3 $R_d$;
$R_3$ is selected from H, F, Cl, Br, I, and $C_{1-3}$ alkyl, and the $C_{1-3}$ alkyl is optionally substituted with 1, 2, or 3 $R_b$;
$R_4$ is selected from OH, CN, and $-(OCH_2CH_2)_n-C_{1-3}$ alkoxy;
n is 0 or 1;
ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;
each $R_a$ and each $R_b$ are independently selected from H, F, Cl, Br, I, and OH;
each $R_c$ is independently selected from H, F, Cl, Br, I, and $C_{1-3}$ alkyl, and the $C_{1-3}$ alkyl is optionally substituted with 1, 2, or 3 halogens;
each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;
provided that: when $T_4$ is selected from $C(R_3)_2$, $T_3$ is selected from $C(R_3)_2O$, $T_2$ is selected from C, $T_1$ is selected from N, $L_1$ is selected from $-CH_2-$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, and $C_{2-5}$ alkynyl, and the $C_{1-4}$ alkyl, $C_{2-5}$ alkenyl, and $C_{2-5}$ alkynyl are independently and optionally substituted with 1, 2, or 3 $R_d$;
"hetero" in the 5- to 6-membered heteroaryl represents 1, 2, or 3 heteroatoms or heteroatom groups independently selected from -O-, -NH-, -S-, -S(=O)-, -N-, and -C(=O)-.

The present disclosure further provides a compound of formula (I) or a pharmaceutically acceptable salt thereof,

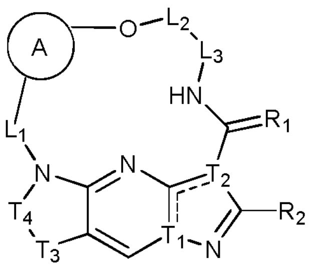

( I )

wherein

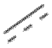 is selected from a single bond and a double bond;
$T_1$ is selected from C, and $T_2$ is selected from N;
or, $T_1$ is selected from N, and $T_2$ is selected from C;
$T_3$ is selected from -O-, $-C(R_3)_2O-$, and $=CR_3-$;
$T_4$ is selected from $-C(R_3)_2-$, -N=, and -C(=O)-;
$L_1$ is selected from $-CH_2-$ and -C(=O)-;
$L_2$ and $L_3$ are each independently selected from $C_{1-3}$ alkyl, and the $C_{1-3}$ alkyl is optionally substituted with 1, 2, or 3 $R_a$;
$R_1$ is selected from O and $NR_4$;
$R_2$ is selected from H, F, Cl, Br, I, OH, $NH_2$, CN, and $C_{1-4}$ alkyl;
$R_3$ is selected from H, F, Cl, Br, I, and $C_{1-3}$ alkyl, and the $C_{1-3}$ alkyl is optionally substituted with 1, 2, or 3 $R_b$;
$R_4$ is selected from OH, CN, and $-(OCH_2CH_2)_n-C_{1-3}$ alkoxy;
n is 0 or 1;
ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;
$R_a$ and $R_b$ are each independently selected from F, Cl, Br, I, and OH;
each $R_c$ is independently selected from F, Cl, Br, I, and $C_{1-3}$ alkyl, and the $C_{1-3}$ alkyl is optionally substituted with 1, 2, or 3 halogens;
provided that:
when $T_4$ is selected from $-C(R_3)_2-$, $T_3$ is selected from $-C(R_3)_2O$, $T_2$ is selected from C, $T_1$ is selected from N, L is selected from $-CH_2-$, ring A is selected from phenyl or 5- to 6-membered heteroaryl, and $R_1$ is selected from =O, then $R_2$ is selected from F, Cl, Br, I, OH, $NH_2$, CN, and $C_{1-4}$ alkyl;
"hetero" in the 5- to 6-membered heteroaryl represents 1, 2, or 3 heteroatoms or heteroatom groups independently selected from -O-, -NH-, -S-, -S(=O)-, -N-, and -C(=O)-.

In some embodiments of the present disclosure, 2 $R_a$ on the same carbon atom are connected to form a cyclopropyl, cyclobutyl, oxetanyl, azetidinyl, oxolanyl, or pyrrolidinyl group, and the cyclopropyl, cyclobutyl, oxetanyl, azetidinyl, oxolanyl, or pyrrolidinyl are optionally substituted with 1, 2, or 3 halogens, and other variables are as defined in the present disclosure.

In some embodiments of the present disclosure, two $R_a$ on the same carbon atom are connected to form a cyclopropyl, cyclobutyl, oxetanyl, or azetidinyl group, and other variables are as defined in the present disclosure.

In some embodiments of the present disclosure, two $R_a$ on the same carbon atom are connected to form a cyclopropyl or cyclobutyl group, and the cyclopropyl or cyclobutyl is optionally substituted with 1, 2, or 3 halogens, and other variables are as defined in the present disclosure.

In some embodiments of the present disclosure, 2 $R_a$ on the same carbon atom are connected to form a cyclopropyl group, and other variables are as defined in the present disclosure.

In some embodiments of the present disclosure, two $R_a$ on the same carbon atom are connected to form a cyclopropyl, cyclobutyl, or oxetanyl group, and other variables are as defined in the present disclosure.

In some embodiments of the present disclosure, 2 $R_a$ on different carbon atoms are connected to form a cyclopropyl or cyclobutyl group, and the cyclopropyl or cyclobutyl is optionally substituted with 1, 2, or 3 halogens, and other variables are as defined in the present disclosure.

In some embodiments of the present disclosure, two $R_a$ on different carbon atoms are connected to form a cyclobutyl group, and other variables are as defined in the present disclosure.

In some embodiments of the present disclosure, each of the above $R_c$ is independently selected from F, Cl, Br, I, and $CH_3$, the $CH_3$ is optionally substituted with 1, 2, or 3 halogens, and other variables are as defined in the present disclosure.

In some embodiments of the present disclosure, each of the above $R_c$ is independently selected from F, Cl, Br, I, $CH_3$, and $CF_3$, and other variables are as defined in the present disclosure.

In some embodiments of the present disclosure, each of the above $R_c$ is independently selected from H, F, Cl, Br, I, and $CH_3$, the $CH_3$ is optionally substituted with 1, 2, or 3 halogens, and other variables are as defined in the present disclosure.

In some embodiments of the present disclosure, each of the above $R_c$ is independently selected from H, F, Cl, Br, I, $CH_3$, and $CF_3$, and other variables are as defined in the present disclosure.

In some embodiments of the present disclosure, each of the above $R_d$ is independently selected from F, OH, $NH_2$, $CH_3$, and cyclopropyl, and other variables are as defined in the present disclosure.

In some embodiments of the present disclosure, each of the above $R_d$ is independently selected from H, F, OH, $NH_2$, $CH_3$, and cyclopropyl, and other variables are as defined in the present disclosure.

In some embodiments of the present disclosure, the above $L_2$ is selected from $CH_2$, $CH_2CH_2$, and $C(CH_3)_2$, and

the $CH_2$, $CH_2CH_2$, and $C(CH_3)_2$ are each independently and optionally substituted with 1, 2, or 3 $R_a$, and other variables are as defined in the present disclosure.

In some embodiments of the present disclosure, the above $L_2$ is selected from $CH_2$ and $C(CH_3)_2$, and the $CH_2$ and $C(CH_3)_2$ are each independently and optionally substituted with 1, 2, or 3 $R_a$, and other variables are as defined in the present disclosure.

In some embodiments of the present disclosure, the above $L_2$ is selected from $CH_2$, $CF_2$, $C(CH_3)_2$, and $C(CF_3)_2$, and other variables are as defined in the present disclosure.

In some embodiments of the present disclosure, the above $L_2$ is selected from $-CH_2-$ and $-C(CH_3)_2-$, and the $-CH_2-$ and $-C(CH_3)_2-$ are each independently and optionally substituted with 1, 2, or 3 $R_a$, and other variables are as defined in the present disclosure.

In some embodiments of the present disclosure, the above $L_2$ is selected from $-CH_2-$, $-CF_2-$, $-C(CH_3)_2-$, and $-C(CF_3)_2-$, and other variables are as defined in the present disclosure.

In some embodiments of the present disclosure, the above $L_2$ is selected from $CH_2$, $CF_2$, $CH_2CH_2$, $C(CH_3)_2$, $C(CF_3)_2$,

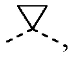, 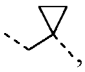, 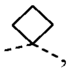, and 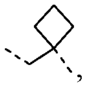,

and other variables are as defined in the present disclosure.

In some embodiments of the present disclosure, the above $L_2$ is selected from $CH_2$, $CF_2$, $CH_2CH_2$, $C(CH_3)_2$, $C(CF_3)_2$,

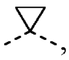, 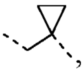, 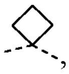, 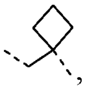, 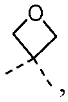, and 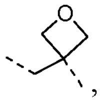,

and other variables are as defined in the present disclosure.

In some embodiments of the present disclosure, the above $L_2$ is selected from a single bond, $CH_2$, $CH_2CH_2$, and $C(CH_3)_2$, and the $CH_2$, $CH_2CH_2$, and $C(CH_3)_2$ are each independently and optionally substituted with 1, 2, or 3 $R_a$, and other variables are as defined in the present disclosure.

In some embodiments of the present disclosure, the above $L_2$ is selected from a single bond, $CH_2$, $CF_2$, $CH_2CH_2$, $C(CH_3)_2$, $C(CF_3)_2$,

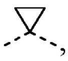, 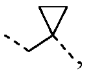, 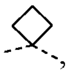, 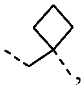, 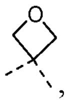, 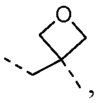,

and

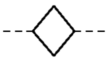,

and other variables are as defined in the present disclosure.

In some embodiments of the present disclosure, the above $L_3$ is selected from $CH_2$, $CH_2CH_2$, and $C(CH_3)_2$, and the $CH_2$, $CH_2CH_2$, and $C(CH_3)_2$ are each independently and optionally substituted with 1, 2, or 3 $R_a$, and other variables are as defined in the present disclosure.

In some embodiments of the present disclosure, the above $L_3$ is selected from $CH_2$ and $C(CH_3)_2$, and the $CH_2$ and $C(CH_3)_2$ are each independently and optionally substituted with 1, 2, or 3 $R_a$, and other variables are as defined in the present disclosure.

In some embodiments of the present disclosure, the above $L_3$ is selected from $CH_2$, $CF_2$, $C(CH_3)_2$, and $C(CF_3)_2$, and other variables are as defined in the present disclosure.

In some embodiments of the present disclosure, the above $L_3$ is selected from $-CH_2-$ and $-C(CH_3)_2-$, and the $-CH_2-$ and $-C(CH_3)_2-$ are each independently and optionally substituted with 1, 2, or 3 $R_a$, and other variables are as defined in the present disclosure.

In some embodiments of the present disclosure, the above $L_3$ is selected from $-CH_2-$, $-CF_2-$, $-C(CH_3)_2-$, and $-C(CF_3)_2-$, and other variables are as defined in the present disclosure.

In some embodiments of the present disclosure, the above $L_3$ is selected from $CH_2$, $CF_2$, $CH_2CH_2$, $C(CH_3)_2$, $C(CF_3)_2$,

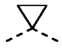, 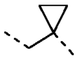, 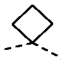, and 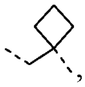,

and other variables are as defined in the present disclosure.

In some embodiments of the present disclosure, the above $L_3$ is selected from $CH_2$, $CF_2$, $CH_2CH_2$, $C(CH_3)_2$, $C(CF_3)_2$,

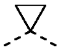, 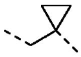, 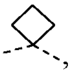, 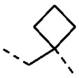, 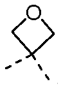, and 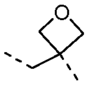,

and other variables are as defined in the present disclosure.

In some embodiments of the present disclosure, the above $L_3$ is selected from a single bond, $CH_2$, $CH_2CH_2$, and $C(CH_3)_2$, and the $CH_2$, $CH_2CH_2$, and $C(CH_3)_2$ are each independently and optionally substituted with 1, 2, or 3 $R_a$, and other variables are as defined in the present disclosure.

In some embodiments of the present disclosure, the above $L_3$ is selected from a single bond, $CH_2$, $CF_2$, $CH_2CH_2$, $C(CH_3)_2$, $C(CF_3)_2$,

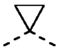, 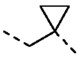, 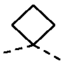, 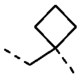, 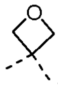, 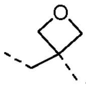,

and

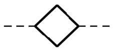,

and other variables are as defined in the present disclosure.

In some embodiments of the present disclosure, the structural moiety $-L_2-L_3-$ is selected from $-CH_2C(CH_3)_2-$,

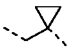, 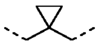, 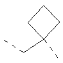, and 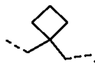,

and other variables are as defined in the present disclosure.

In some embodiments of the present disclosure, the structural moiety $-L_2-L_3-$ is selected from $-CH_2C(CH_3)_2-$,

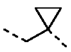, 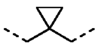, 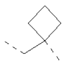, 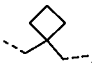, and 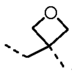,

and other variables are as defined in the present disclosure.

In some embodiments of the present disclosure, the structural moiety $-L_2-L_3-$ is selected from $-CH_2C(CH_3)_2-$,

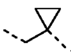, 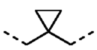, 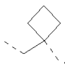, 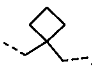, 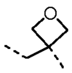, and 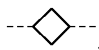,

and other variables are as defined in the present disclosure.

In some embodiments of the present disclosure, the above $R_1$ is selected from O, S, NOH, NCN, $NOCH_3$, $NOCH_2CH_3$, and $NOCH_2CH_2OCH_3$, and other variables are as defined in the present disclosure.

In some embodiments of the present disclosure, the above $R_1$ is selected from O, NOH, NCN, $NOCH_3$, $NOCH_2CH_3$, and $NOCH_2CH_2OCH_3$, and other variables are as defined in the present disclosure.

In some embodiments of the present disclosure, the above $R_1$ is selected from O and S, and other variables are as defined in the present disclosure.

In some embodiments of the present disclosure, the above $R_1$ is selected from NCN and $NOCH_3$, and other variables are as defined in the present disclosure.

In some embodiments of the present disclosure, the above $R_2$ is selected from H, F, Cl, Br, I, OH, $NH_2$, CN, $CH_3$, and 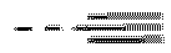 , and the $CH_3$ and 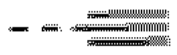 are independently and optionally substituted with 1, 2, or 3 $R_d$, and other variables are as defined in the present disclosure.

In some embodiments of the present disclosure, the above $R_2$ is selected from H, F, Cl, Br, I, OH, $NH_2$, CN, $CH_3$, 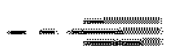, and

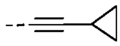,

and other variables as defined in the present disclosure.

In some embodiments of the present disclosure, the above $R_2$ is selected from H, F, Cl, Br, I, OH, $NH_2$, CN, and $CH_3$, and other variables are as defined in the present disclosure.

In some embodiments of the present disclosure, the above $R_2$ is selected from H, F, Cl, Br, I, OH, $NH_2$, CN, $CH_3$, cyclopropyl, 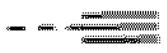, and 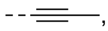, and the $CH_3$, cyclopropyl, 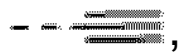, and  are each independently and optionally substituted with 1, 2, or 3 $R_d$, and other variables are as defined in the present disclosure.

In some embodiments of the present disclosure, the above $R_2$ is selected from H, F, Cl, Br, I, OH, $NH_2$, CN, $CH_3$, 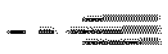,

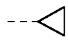, 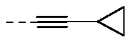,

and 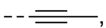, and other variables are as defined in the present disclosure.

In some embodiments of the present disclosure, the above $R_2$ is selected from H, F, Cl, Br, I, OH, $NH_2$, CN, $CH_3$, 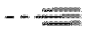,

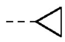, 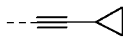,

, and

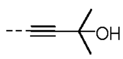,

and other variables are as defined in the present disclosure.

In some embodiments of the present disclosure, the above $R_2$ is selected from F, Cl, Br, I, OH, $NH_2$, CN, $CH_3$, 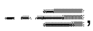,

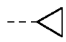, 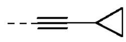,

, and

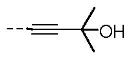,

and other variables are as defined in the present disclosure.

In some embodiments of the present disclosure, the above $R_2$ is selected from $C_{2-5}$ alkynyl, and the $C_{2-5}$ alkynyl is independently and optionally substituted with 1, 2, or 3 $R_d$, and other variables are as defined in the present disclosure.

In some embodiments of the present disclosure, the above $R_2$ is selected from ethynyl and propynyl, and the ethynyl and propynyl are each independently and optionally substituted with 1, 2, or 3 $R_d$, and other variables are as defined in the present disclosure. In some embodiments of the present disclosure, the above $R_3$ is selected from H, F, Cl, Br, I, $CH_3$, $CH_2F$, $CHF_2$, $CF_3$, and

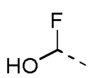,

and other variables are as defined in the present disclosure.

In some embodiments of the present disclosure, the above $R_3$ is selected from H, $CH_3$, and $CHF_2$, and other variables are as defined in the present disclosure.

In some embodiments of the present disclosure, the above $R_3$ is selected from $CHF_2$, and other variables are as defined in the present disclosure.

In some embodiments of the present disclosure, the above $T_3$ is selected from -O-, - $CH_2O$-, =CH-, and =$CCH_3$-, and other variables are as defined in the present disclosure.

In some embodiments of the present disclosure, the above $T_3$ is selected from O, $CH_2O$, CH, and $C(CH_3)$, and other variables are as defined in the present disclosure.

In some embodiments of the present disclosure, the above $T_4$ is selected from - $CH(CRF_2)$-,-N=, and -C(=O)-, and other variables are as defined in the present disclosure.

In some embodiments of the present disclosure, the above $T_4$ is selected from $CH(CHF_2)$, N, and C(=O), and other variables are as defined in the present disclosure.

In some embodiments of the present disclosure, the above ring A is selected from phenyl, pyridyl, and cyclohexenyl, and the phenyl, pyridyl, and cyclohexenyl are each independently and optionally substituted with 1, 2, or 3 $R_c$, and other variables are as defined in the present disclosure.

In some embodiments of the present disclosure, the above ring A is selected from phenyl and pyridyl, and the phenyl and pyridyl are each independently and optionally substituted with 1, 2, or 3 $R_c$, and other variables are as defined in the present disclosure.

In some embodiments of the present disclosure, the above ring A is selected from

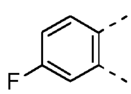, 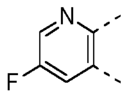, and 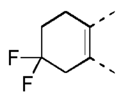,

and other variables are as defined in the present disclosure.

In some embodiments of the present disclosure, the above ring A is selected from

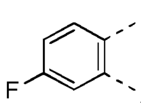, 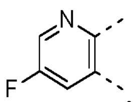, 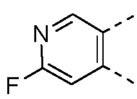, and 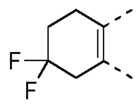,

and other variables are as defined in the present disclosure.

In some embodiments of the present disclosure, the structural moiety

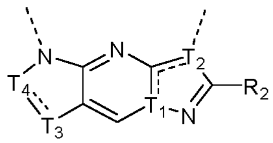

is selected from

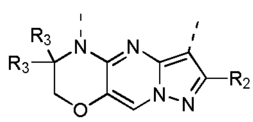, 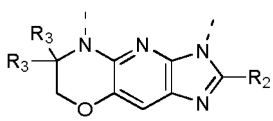,

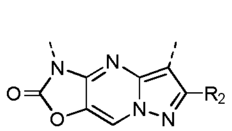, 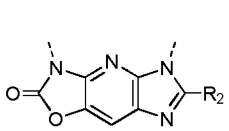, 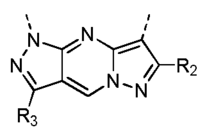, and 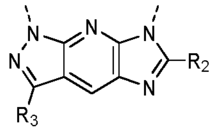,

and other variables are as defined in the present disclosure.

In some embodiments of the present disclosure, the structural moiety

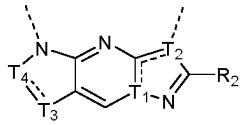

is selected from

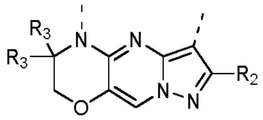 and 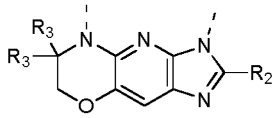 ,

and other variables are as defined in the present disclosure.

In some embodiments of the present disclosure, the structural moiety

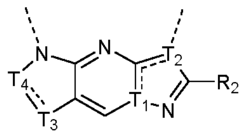

is selected from

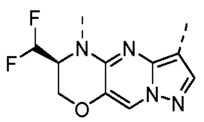 , 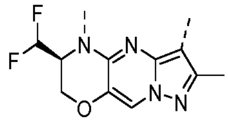 ,

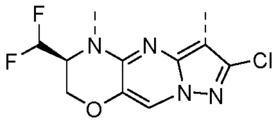 , 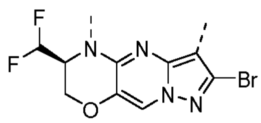 , 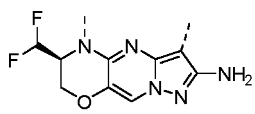 , 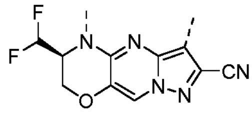 ,

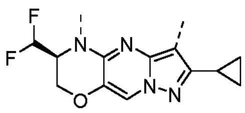 , 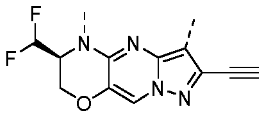 , 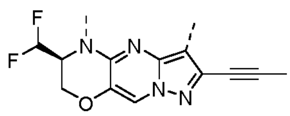 , 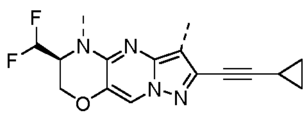 ,

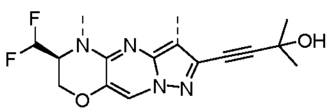 , 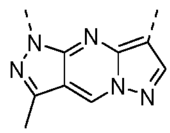 , 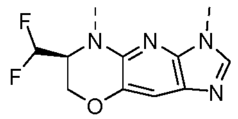 , 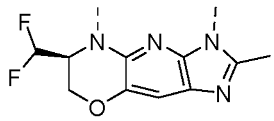 ,

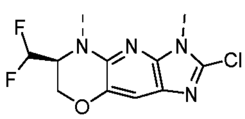 , 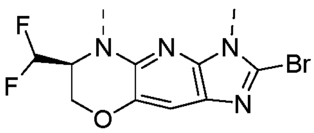 , 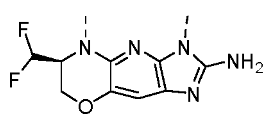 , 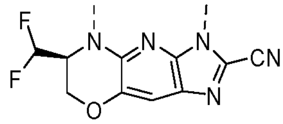 ,

and

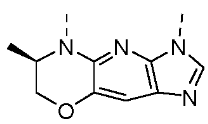 ,

and other variables are as defined in the present disclosure.

In some embodiments of the present disclosure, the structural moiety

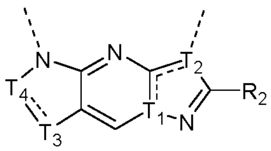

is selected from

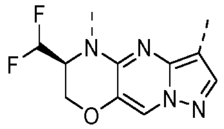,
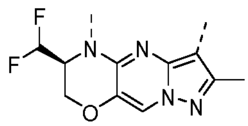,
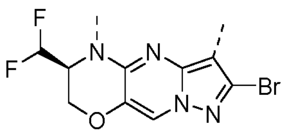,
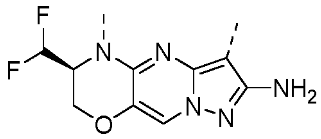,
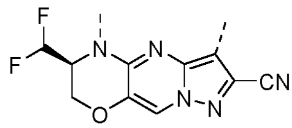,
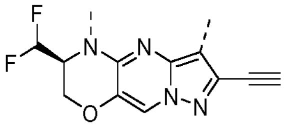,
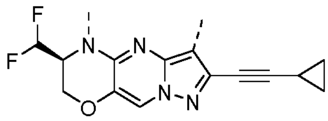, and
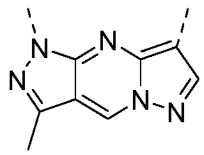,

and other variables are as defined in the present disclosure.

In some embodiments of the present disclosure, the structural moiety

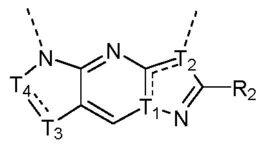

is selected from

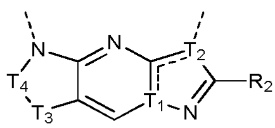,

and other variables are as defined in the present disclosure.

In some embodiments of the present disclosure, the structural moiety

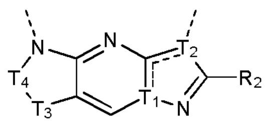

is selected from

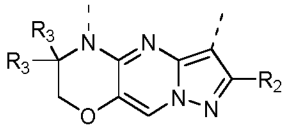, 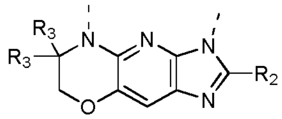,

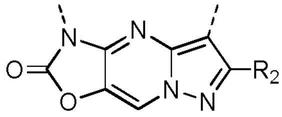, 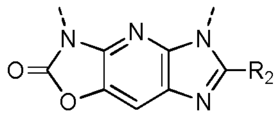, 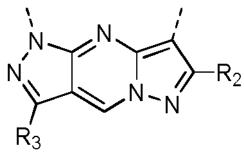, and 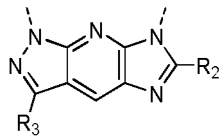,

and other variables are as defined in the present disclosure.

In some embodiments of the present disclosure, the structural moiety

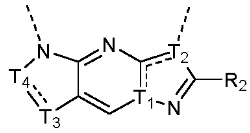

is selected from

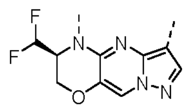, 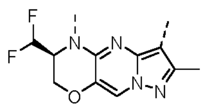, 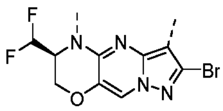,

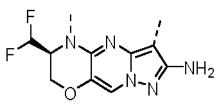, 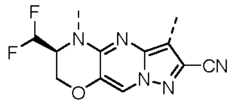, 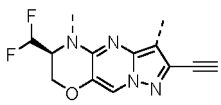, 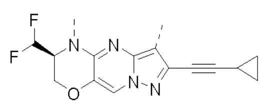, 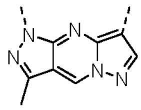,

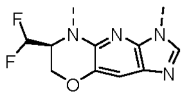, 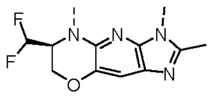, 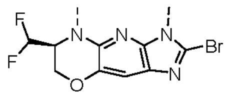, 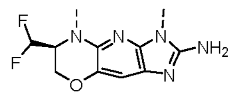, 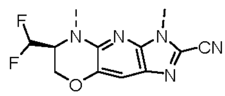,

and

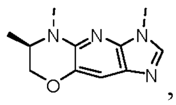,

and other variables are as defined in the present disclosure.

In some embodiments of the present disclosure, the structural moiety

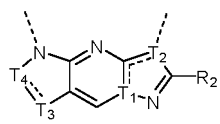

is selected from

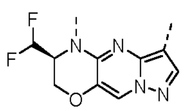, 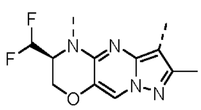, 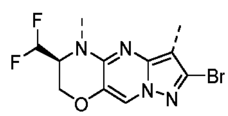,

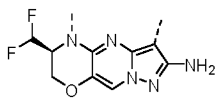, 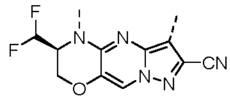, 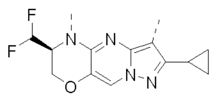, 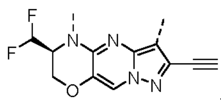, 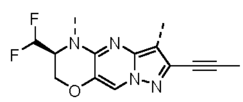,

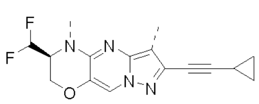, 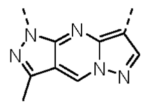, 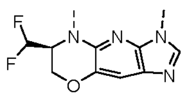, 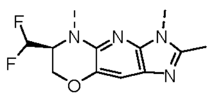, 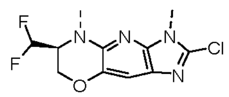,

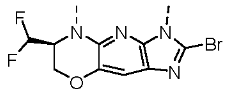, 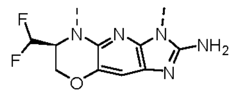, 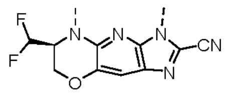, and 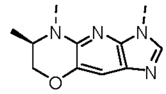,

and other variables are as defined in the present disclosure.

In some embodiments of the present disclosure, the compound or the pharmaceutically acceptable salt thereof is selected from:

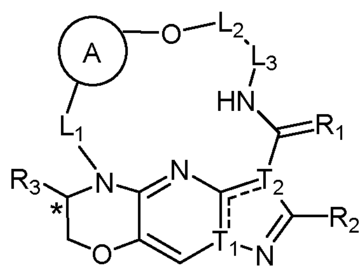

( II-1 ) ,

wherein

$R_1$, $R_2$, $R_3$, $T_1$, $T_2$, $L_1$, $L_2$, $L_3$, ring A, and ⁄⁄ are as defined in the present disclosure;
when $R_3$ is not H, the carbon atom with "*" is a chiral carbon atom, which exists in an (R) or (S) single enantiomer form or an (R) or (S) single enantiomer-rich form.

In some embodiments of the present disclosure, the compound or the pharmaceutically acceptable salt thereof is selected from:

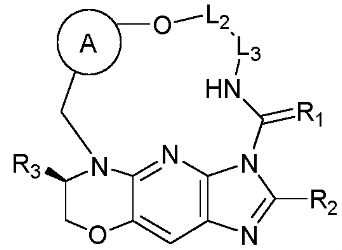

( III-1 ) ,

wherein

$R_3$ is selected from F, Cl, Br, I, and $C_{1-3}$ alkyl, and the $C_{1-3}$ alkyl is optionally substituted with 1, 2, or 3 $R_b$;
$R_1$, $R_2$, $L_2$, $L_3$, ring A, and each $R_b$ are as defined in the present disclosure.

In some embodiments of the present disclosure, the compound or the pharmaceutically acceptable salt thereof is selected from:

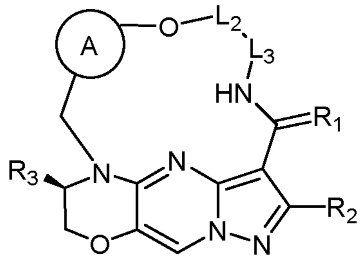

( P-1 ) ,

wherein

- $R_3$ is selected from F, Cl, Br, I, and $C_{1-3}$ alkyl, and the $C_{1-3}$ alkyl is optionally substituted with 1, 2, or 3 $R_b$;
- $R_1$, $R_2$, $L_2$, $L_3$, ring A, and each $R_b$ are as defined in the present disclosure.

In some embodiments of the present disclosure, the compound or the pharmaceutically acceptable salt thereof is selected from:

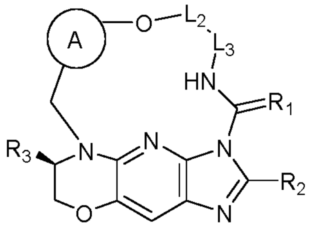

( III-1 ) and

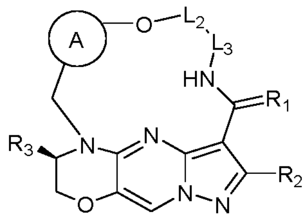

( P-1 ) ,

wherein

- $R_1$ is selected from O, NOH, NCN, $NOCH_3$, $NOCH_2CH_3$, and $NOCH_2CH_2OCH_3$;
- $R_2$ is selected from F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
- $R_3$ is selected from F, Cl, Br, I, and $C_{1-3}$ alkyl, and the $C_{1-3}$ alkyl is optionally substituted with 1, 2, or 3 $R_b$;
- ring A is selected from phenyl and pyridyl, and the phenyl and pyridyl are each independently and optionally substituted with 1, 2, or 3 $R_c$;
- the structural moiety $-L_2-L_3-$ is selected from $-CH_2C(CH_3)_2-$,

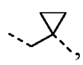 , 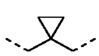 , 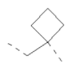 ,

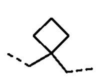 , 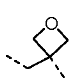 , and 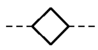 ;

- each $R_b$ is independently selected from H, F, Cl, Br, I, and OH;
- each $R_c$ is independently selected from H, F, Cl, Br, I, and $C_{1-3}$ alkyl, and the $C_{1-3}$ alkyl is optionally substituted with 1, 2, or 3 halogens;
- each $R_d$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens.

In some embodiments of the present disclosure, the compound of formula (III-1) or (P-1), or a pharmaceutically acceptable salt thereof, wherein $R_d$ is as defined in formula (II) of the present disclosure.

In some embodiments of the present disclosure, the compound of formula (III-1) or (P-1), or a pharmaceutically

acceptable salt thereof, wherein $R_1$ is selected from O, and other variables are as defined in formula (III-1) or (P-1) of the present disclosure.

In some embodiments of the present disclosure, the compound of formula (III-1) or (P-1), or a pharmaceutically acceptable salt thereof, wherein $R_2$ is selected from F, Cl, Br, CN, $C_{1-4}$ alkyl, $C_{2-5}$ alkynyl, and $C_{3-6}$ cycloalkyl, and the $C_{1-4}$ alkyl, $C_{2-5}$ alkynyl, and $C_{3-6}$ cycloalkyl are each independently and optionally substituted with 1, 2, or 3 $R_d$, and other variables are as defined in formula (III-1) or (P-1) of the present disclosure.

In some embodiments of the present disclosure, the compound of formula (III-1) or (P-1), or a pharmaceutically acceptable salt thereof, wherein $R_2$ is selected from $C_{2-5}$ alkynyl, and the $C_{2-5}$ alkynyl is optionally substituted with 1, 2, or 3 $R_d$, and other variables are as defined in formula (III-1) or (P-1) of the present disclosure.

In some embodiments of the present disclosure, the compound of formula (III-1) or (P-1), or a pharmaceutically acceptable salt thereof, wherein $R_3$ is selected from $CHF_2$, and other variables are as defined in formula (III-1) or (P-1) of the present disclosure.

There are still some embodiments of the present disclosure which are obtained by any combination of the above variables.

The present disclosure further provides a compound of the following formula or a pharmaceutically acceptable salt thereof,

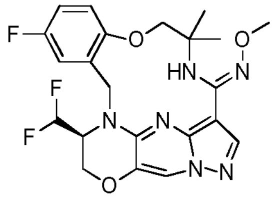 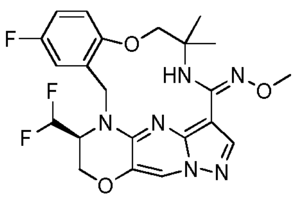 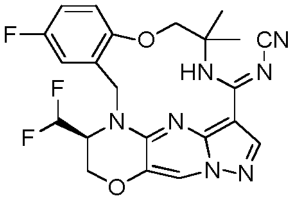 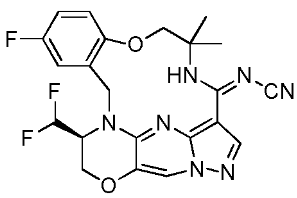

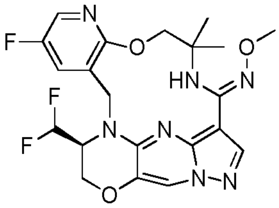 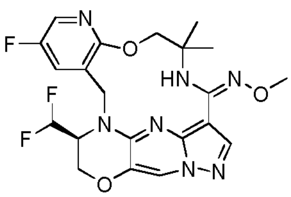 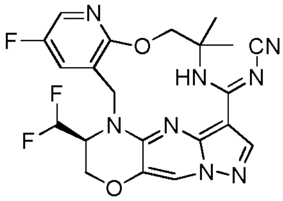 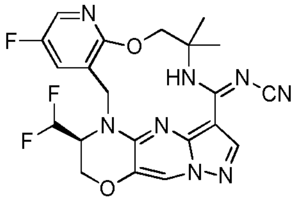

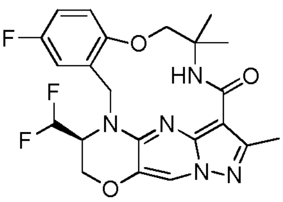 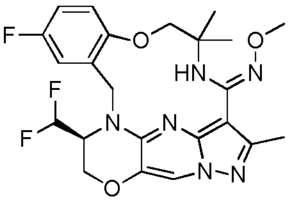 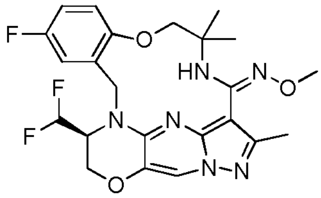 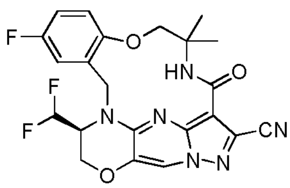

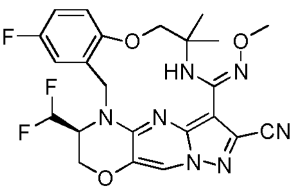 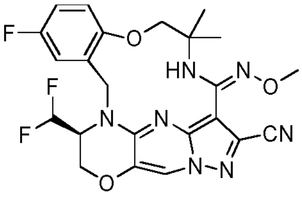 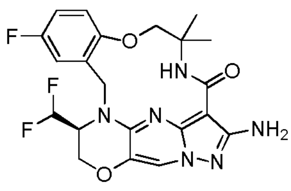 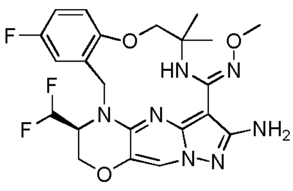

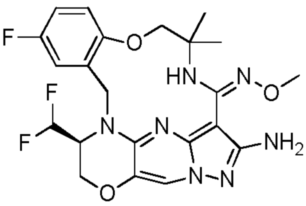 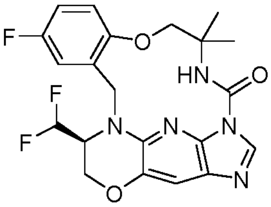 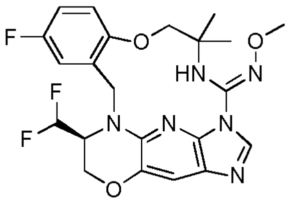 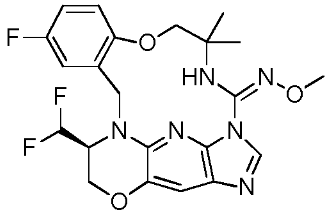

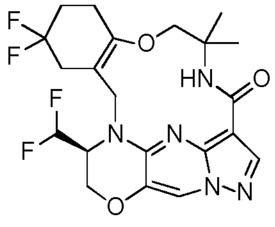 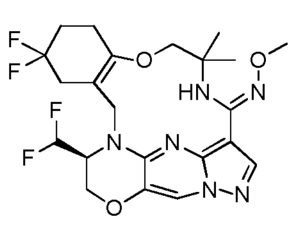 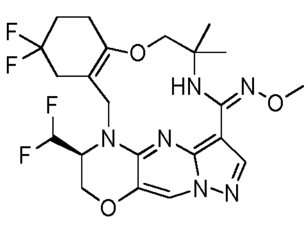 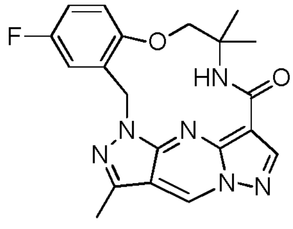

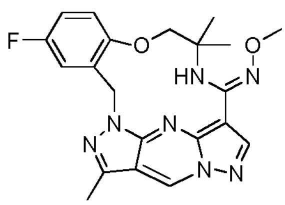 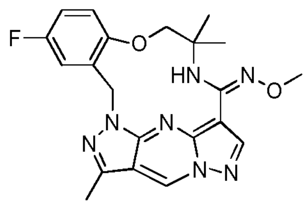 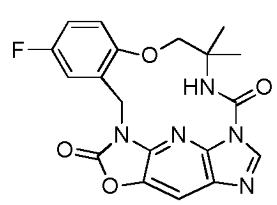 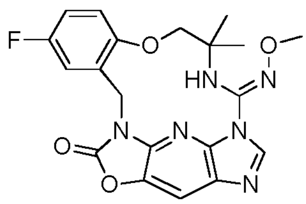

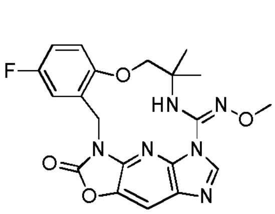 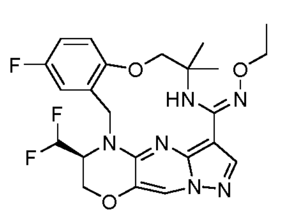 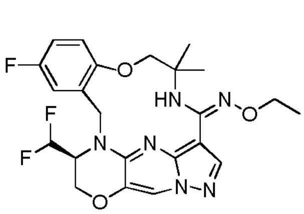 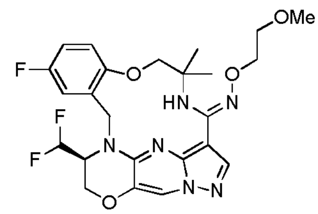

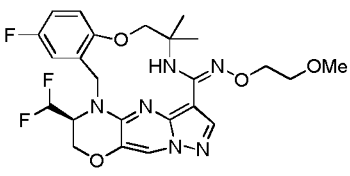 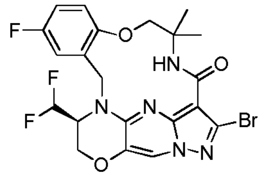 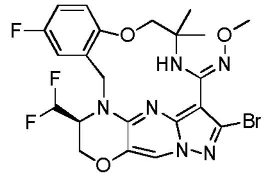 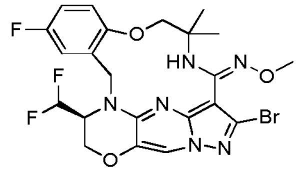

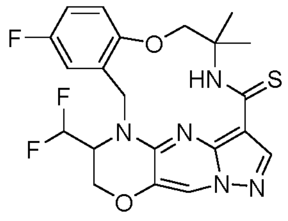 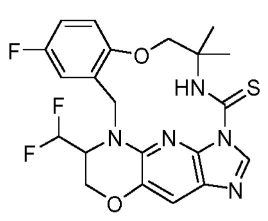 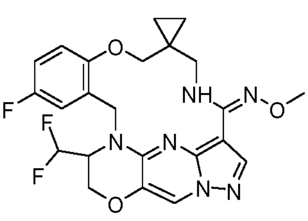 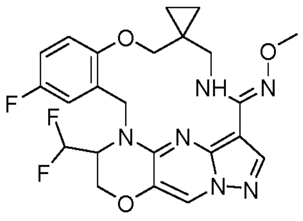

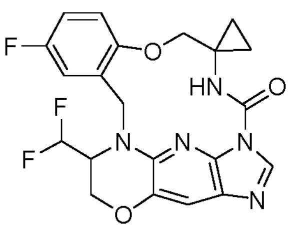 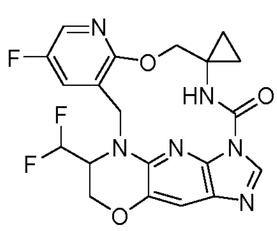 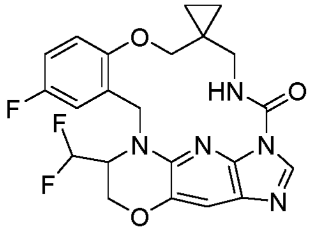 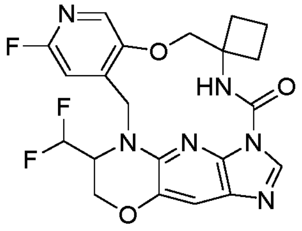

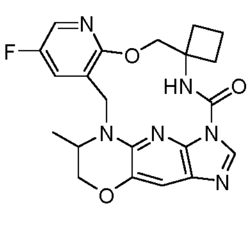 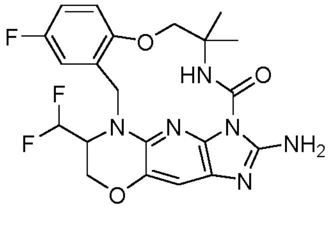 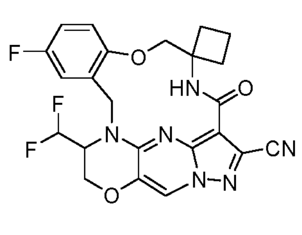 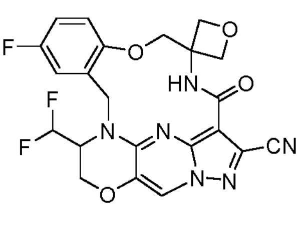

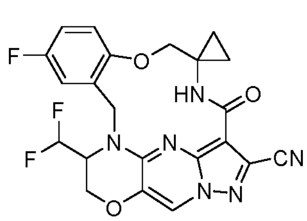
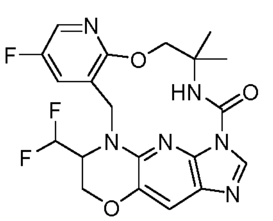
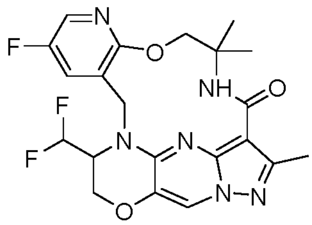
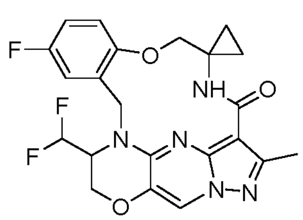
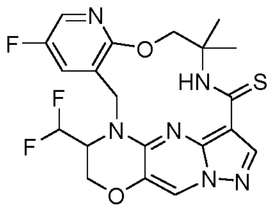
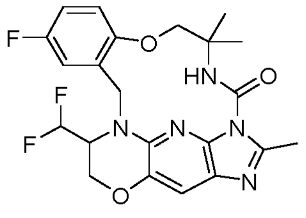
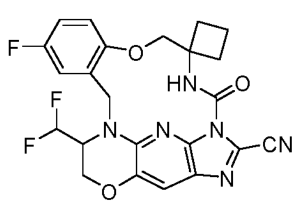
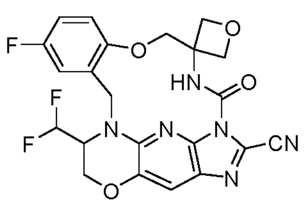
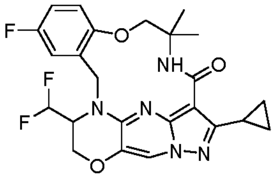
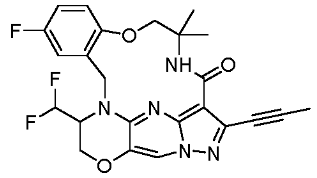
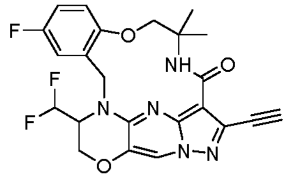
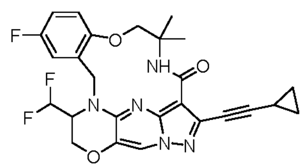
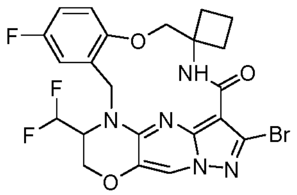
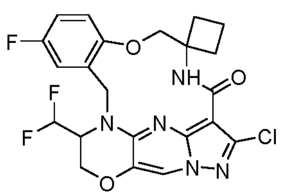
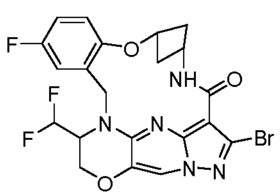
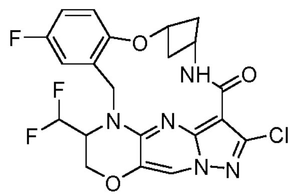
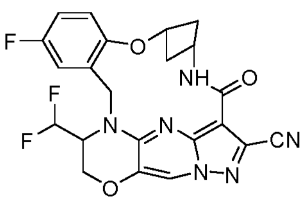
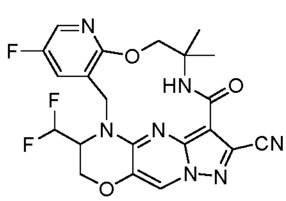
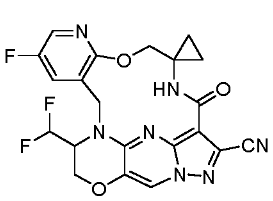
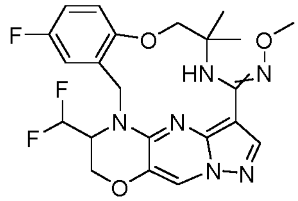
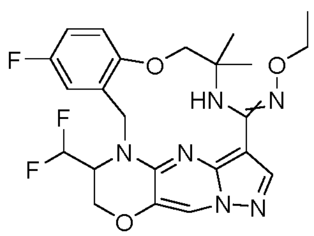
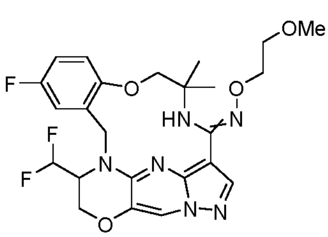
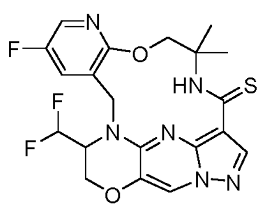
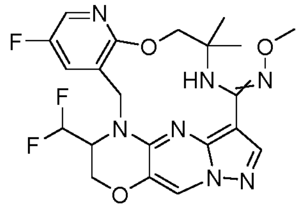
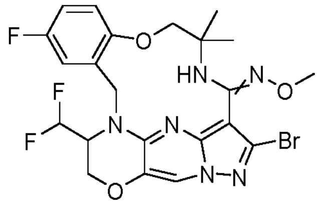
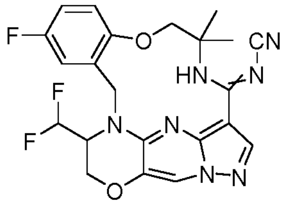
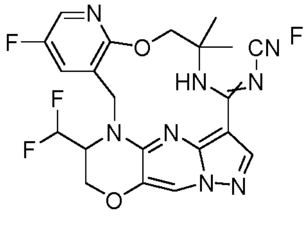
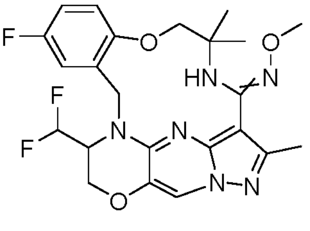

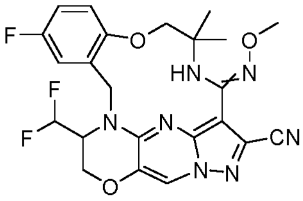 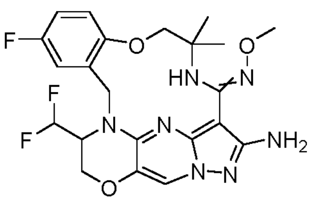 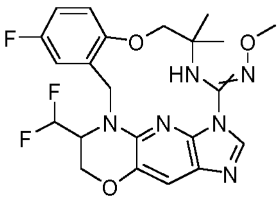 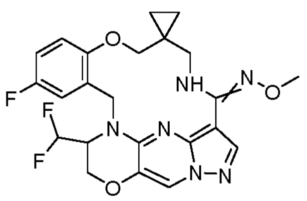

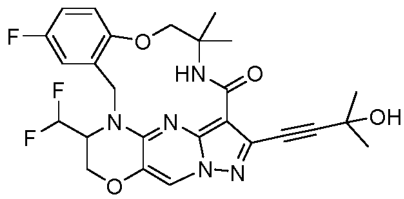.

The present disclosure further provides a compound of the following formula or a pharmaceutically acceptable salt thereof,

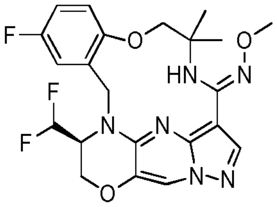 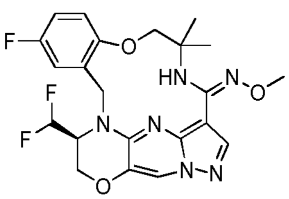 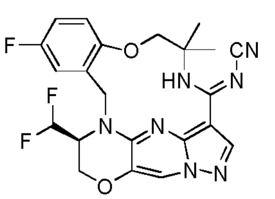 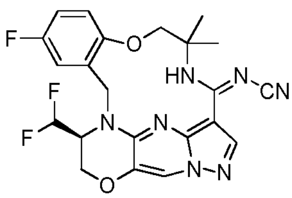

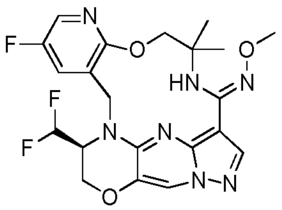 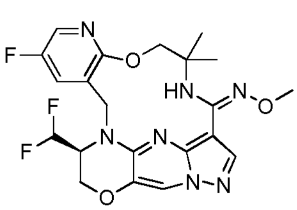 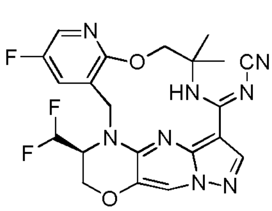 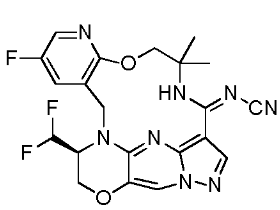

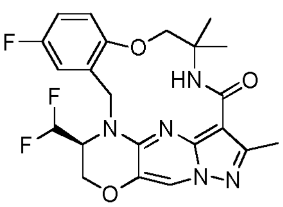 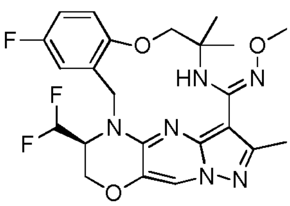 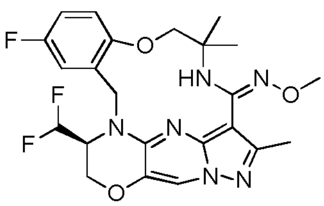 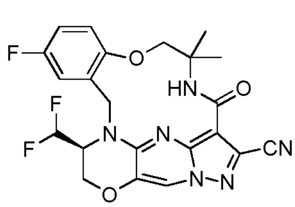

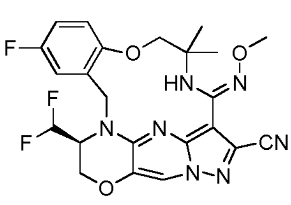 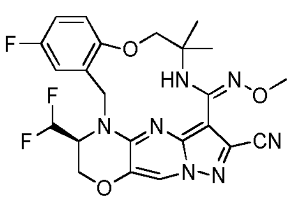 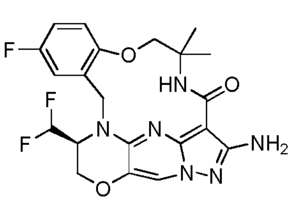 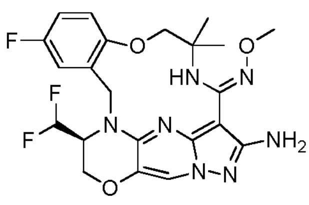

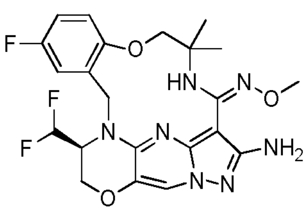 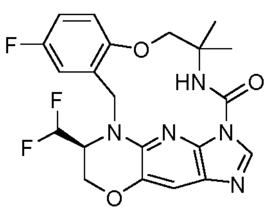 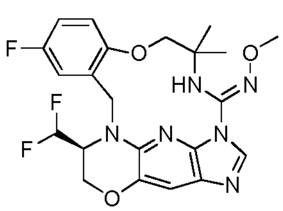 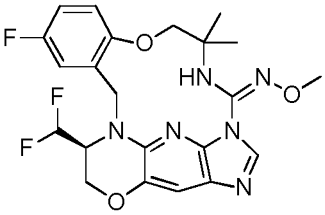

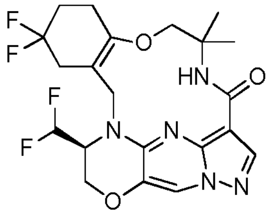 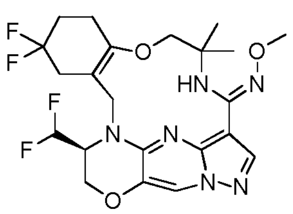 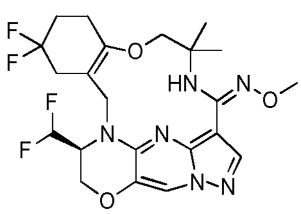 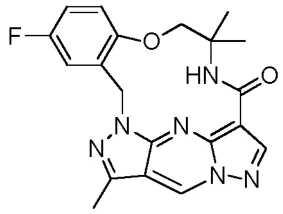

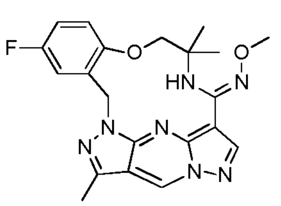 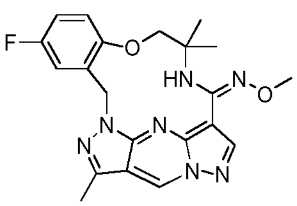 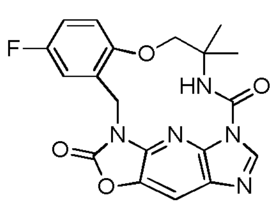 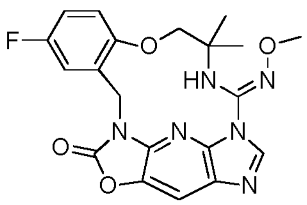

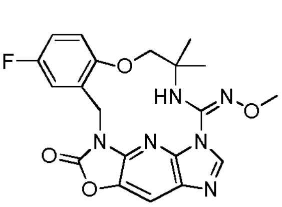 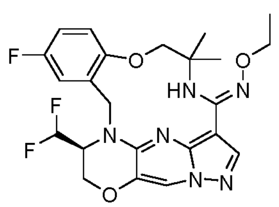 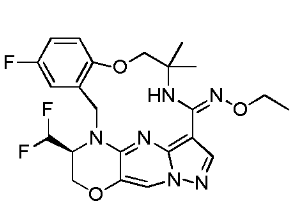 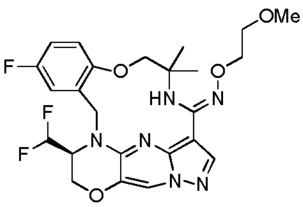

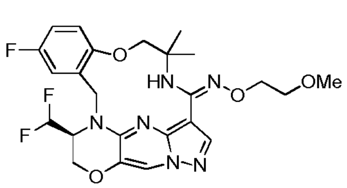 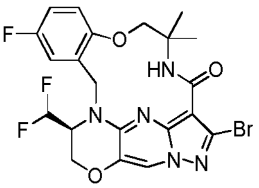 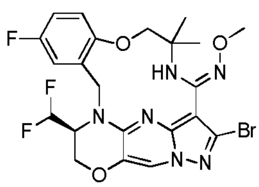 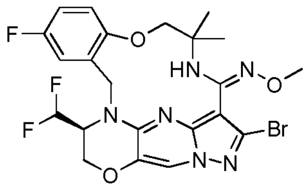

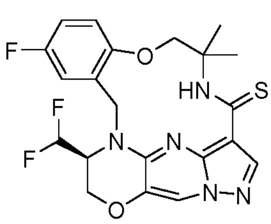 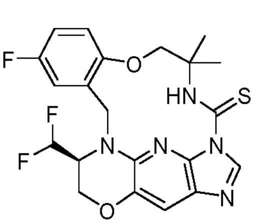 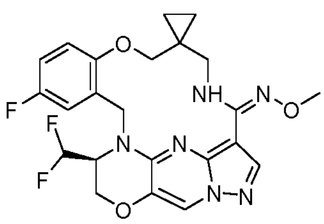 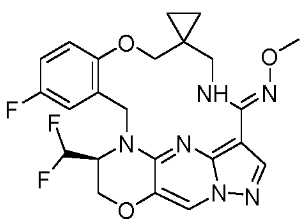

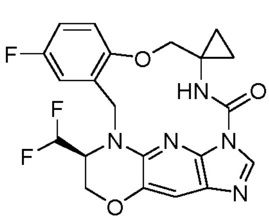 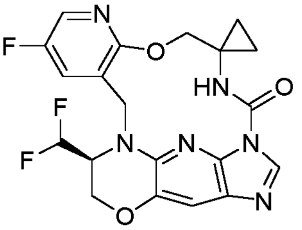 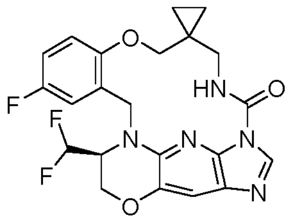 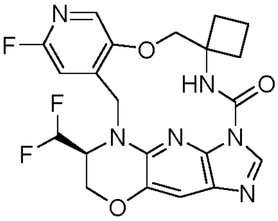

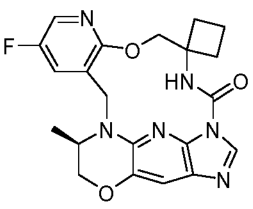 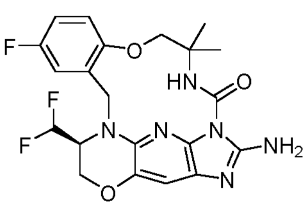 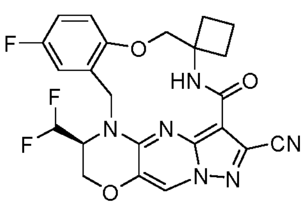 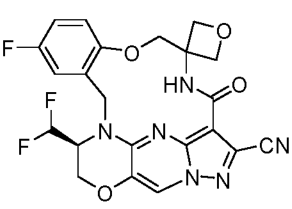

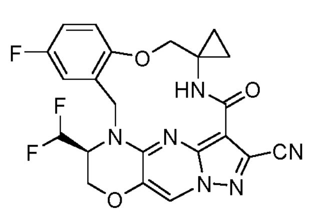 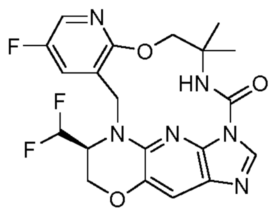 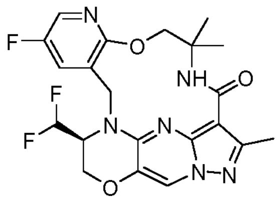 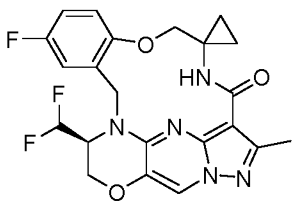

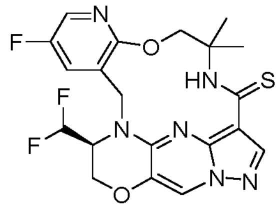 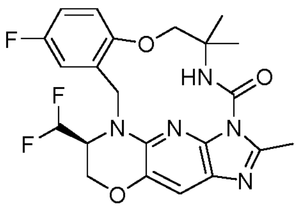 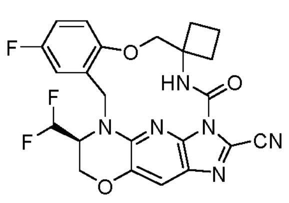 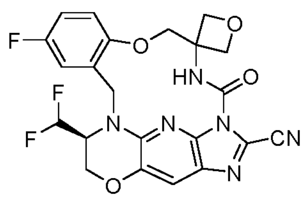

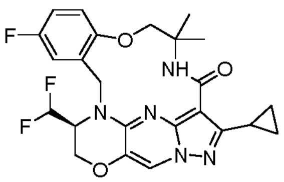 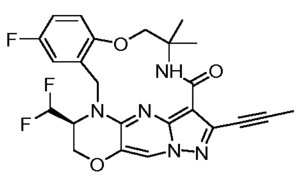 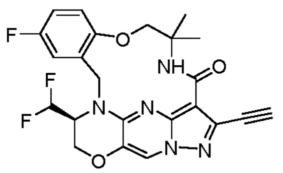 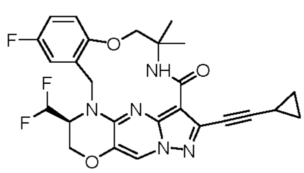

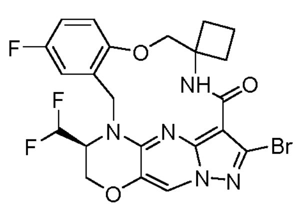 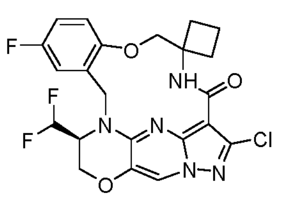 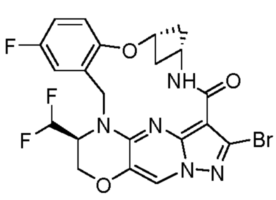 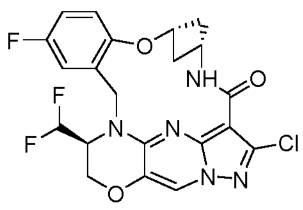

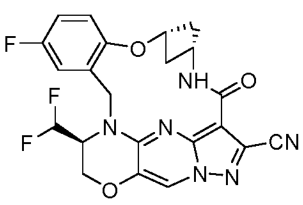 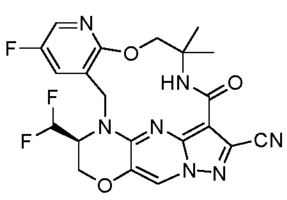 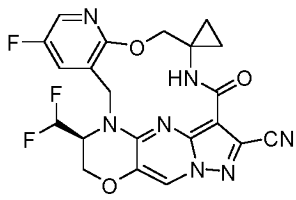 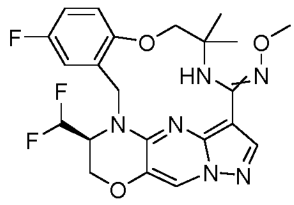

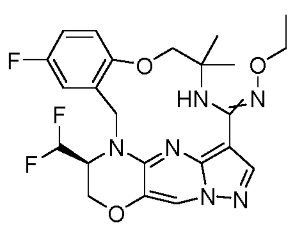 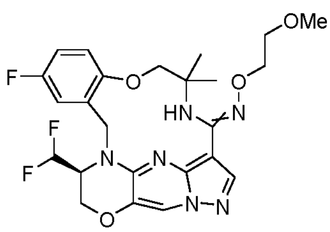 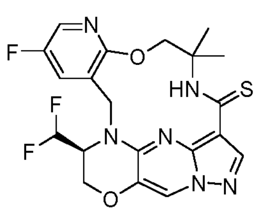 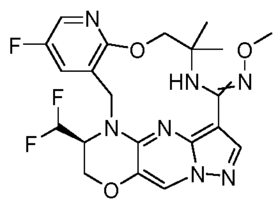

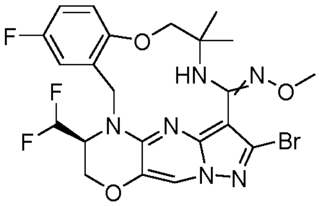 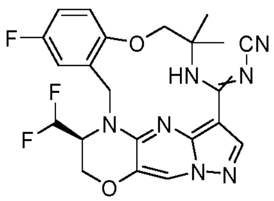 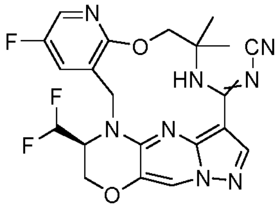 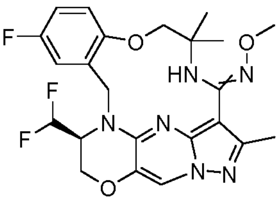

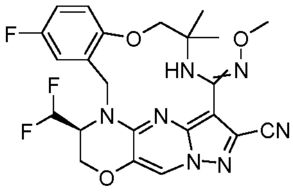
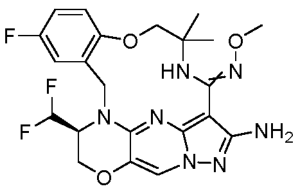
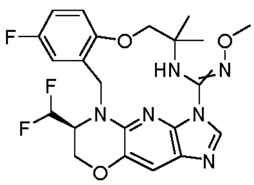
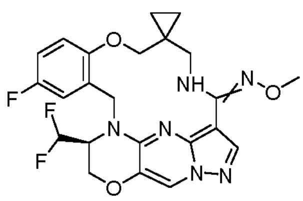

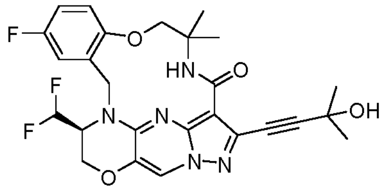

.

The present disclosure further provides a use of the compound or the pharmaceutically acceptable salt thereof in the manufacture of a medicament for treating ALK inhibitor-related diseases.

Technical effect

The compounds of the present disclosure show higher kinase inhibitory activity in ALK kinases and mutants thereof, ROS1 and TRKB kinases, and also have potent inhibitory effect on ALK mutant cells and TRKB phenotype cells, wherein the compounds have stronger inhibition on ALK, relatively weaker inhibition on TRKB, and excellent selectivity; the compounds of the present disclosure are hypertonic and low-efflux compounds, do not significantly inhibit different subtypes of P450 isozymes, demonstrate better hepatocyte metabolic stability, and have excellent pharmacokinetic properties.

Definition and description

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting such compounds with a sufficient amount of acid in a pure solution or a suitable inert solvent. Certain specific compounds of the present disclosure contain both basic and acidic functional groups, and thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (R)- and (S)-enantiomers, diastereoisomers, (D)-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the absolute configuration of a stereogenic center is represented by a wedged solid bond ( ) and a wedged dashed bond ( ), and the relative configuration of a stereogenic center is represented by a straight solid bond ( ) and a straight dashed bond ( ), a wave line ( ) is used to represent a wedged solid bond ( )

or a wedged dashed bond (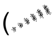) or a mixture of the two isomers, or the wave line (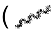) is used to represent a straight solid bond (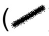)or a straight dashed bond (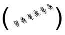) or a mixture of the two isomers.

Unless otherwise specified, when a double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound and its substituent are represented by

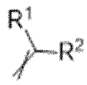,

this refers to the (Z) isomer, (E) isomer or a mixture of two isomers of the compound.

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (R)- and (S)-isomers, *or D* and *L* isomers can be prepared using chiral synthesis, chiral reagents, or other conventional techniques. If one kind of enantiomer of certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more atoms that constitute the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more hydrogen atoms on a specific atom is substituted with the substituent, including deuterium and hydrogen variables, as long as the valence of the specific atom is normal and the substituted compound is stable. When the substituent is an oxygen (i.e., =O), it means that two hydrogen atoms are substituted. When the substituent is $=CH_2$, it means that two hydrogen atoms are substituted.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0 to 2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as $-(CRR)_0-$, it means that the linking group is a single bond.

When a substituent is 0 in number, it means that the substituent is absent. In the case of $-A-(R)_0$, the structure is actually -A.

When a substituent is vacant, it means that the substituent is absent, for example, when X is vacant in A-X, the

structure of A-X is actually A.

When one of the variables is selected from a single bond, it means that the two groups linked by the single bond are connected directly. For example, when L in A-L-Z represents a single bond, the structure of A-L-Z is actually A-Z.

When the enumerative linking group does not indicate the direction for linking, the direction for linking is arbitrary, for example, the linking group L contained in

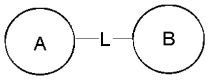

is -M-W-, then -M-W- can link ring A and ring B to form

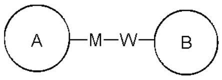

in the direction same as left-to-right reading order, and form

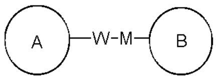

in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond (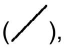), a straight dashed bond (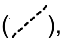), or a wavy line (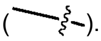). For example, the straight solid bond in $-OCH_3$ means that it is linked to other groups through the oxygen atom in the group; the straight dashed bond in

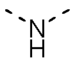

means that it is linked to other groups through the two ends of the nitrogen atom in the group; the wave lines in

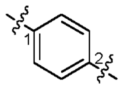

means that the phenyl group is linked to other groups through carbon atoms at position 1 and position 2;

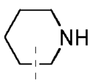

means that it can be linked to other groups through any linkable sites on the piperidinyl by one chemical bond, including at least four types of linkage, including

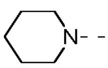, 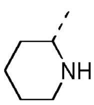, 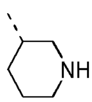, 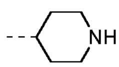.

Even though the H atom is drawn on the -N-,

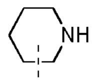

still includes the linkage of

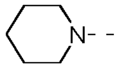

,

merely when one chemical bond was connected, the H of this site will be reduced by one to the corresponding monovalent piperidinyl.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "halogen element" or "halogen" by itself or as part of another substituent refers to fluorine, chlorine, bromine, or iodine atom.

Unless otherwise specified, the term "$C_{1-4}$ alkyl" is used by itself or in combination with other terms to refer to a linear or branched saturated hydrocarbon group consisting of 1 to 4 carbon atoms. The term "$C_{1-4}$ alkyl" refers to a linear or branched saturated hydrocarbon group consisting of 1 to 4 carbon atoms. The $C_{1-4}$ alkyl includes $C_4$, $C_3$, $C_{1-3}$, $C_{1-2}$, and $C_{2-3}$ alkyl, etc.; it can be monovalent, divalent, or multivalent. Examples of $C_{1-4}$ alkyl include, but are not limited to, methyl (Me), ethyl (Et), propyl (including n-propyl and isopropyl), etc.

Unless otherwise specified, the term "$C_{1-3}$ alkyl" is used by itself or in combination with other terms to refer to a linear or branched saturated hydrocarbon group consisting of 1 to 3 carbon atoms. The term "$C_{1-3}$ alkyl" refers to a linear or branched saturated hydrocarbon group consisting of 1 to 3 carbon atoms. The $C_{1-3}$ alkyl includes $C_3$, $C_{1-2}$, and $C_{2-3}$ alkyl, etc.; it can be monovalent, divalent, or multivalent. Examples of $C_{1-3}$ alkyl include, but are not limited to, methyl (Me), ethyl (Et), propyl (including n-propyl and isopropyl), etc.

Unless otherwise specified, "$C_{2-5}$ alkenyl" is used by itself or in combination with other terms to refer to a linear or branched hydrocarbon group consisting of 2 to 5 carbon atoms containing at least one carbon-carbon double bond, wherein the carbon-carbon double bond can be located at any position within the group. The $C_{2-5}$ alkenyl includes $C_{2-4}$, $C_{2-3}$, $C_5$, $C_4$, $C_3$, and $C_2$ alkenyl, etc.; it can be monovalent, divalent, or multivalent. Examples of $C_{2-5}$ alkenyl include, but are not limited to, vinyl, propenyl, butenyl, pentenyl, butadienyl, pentadienyl,etc.

Unless otherwise specified, "$C_{2-5}$ alkynyl" is used by itself or in combination with other terms to refer to a linear or branched hydrocarbon group consisting of 2 to 5 carbon atoms containing at least one carbon-carbon triple bond, wherein the carbon-carbon triple bond can be located at any position within the group. The $C_{2-5}$ alkynyl includes $C_{2-4}$, $C_{2-3}$, $C_5$, $C_4$, $C_3$, and $C_2$ alkynyl, etc. It can be monovalent, divalent, or multivalent. Examples of $C_{2-5}$ alkynyl include, but are not limited to, ethynyl, propynyl, butynyl, pentynyl, etc.

Unless otherwise specified, the term "$C_{1-3}$ alkoxy" by itself or in combination with other terms refers to an alkyl consisting of 1 to 3 carbon atoms that are connected to the rest of the molecule through an oxygen atom. It can be monovalent, divalent, or multivalent. The $C_{1-3}$ alkoxy includes $C_{1-2}$, $C_{2-3}$, $C_3$, $C_2$ alkoxy, etc. Examples of $C_{1-3}$ alkoxy include, but are not limited to, methoxy, ethoxy, propoxy (including n-propoxy and isopropoxy), etc.

Unless otherwise specified, "$C_{3-6}$ cycloalkyl" by itself or in combination with other terms refers to a saturated monocyclic hydrocarbon group consisting of 3 to 6 carbon atoms. The $C_{3-6}$ cycloalkyl includes $C_{3-5}$, $C_{4-5}$, and $C_{5-6}$ cycloalkyl, etc.; it can be monovalent, divalent, or multivalent. Examples of $C_{3-6}$ cycloalkyl include, but are not limited to, cyclopropyl, cyclobutyl, cyclopentyl, cyclohexyl, etc.

Unless otherwise specified, "$C_{5-6}$ cycloalkenyl" by itself or in combination with other terms refers to a partially unsaturated monocyclic hydrocarbon group consisting of 5 to 6 carbon atoms containing at least one carbon-carbon double bond. The $C_{5-6}$ cycloalkenyl includes $C_5$ or $C_6$ cycloalkenyl, etc.; it can be monovalent, divalent, or multivalent. Examples of $C_{5-6}$ cycloalkenyl include, but are not limited to, cyclopentenyl, cyclopentadienyl, cyclohexenyl, cyclohexadienyl, etc.

Unless otherwise specified, the term "4- to 6-membered heterocycloalkyl" by itself or in combination with other terms refers to a saturated monocyclic group consisting of 4 to 6 ring atoms, wherein 1, 2, 3, or 4 ring atoms are heteroatoms independently selected from O, S, and N, and the rest are carbon atoms, wherein the carbon atoms are optionally oxo-substituted (i.e., C(O)), nitrogen atoms are optionally quaternized, and nitrogen and sulfur heteroatoms can be optionally oxidized (i.e., NO and $S(O)_p$, p is 1 or 2). In addition, in the case of the "4- to 6-membered heterocycloalkyl", the heteroatom may occupy the position where the heterocycloalkyl is linked to the rest of the molecule. The 4- to 6-membered heterocycloalkyl includes 5- to 6-membered, 4-membered, 5-membered, 6-membered heterocycloalkyl, etc. It can be monovalent, divalent, or multivalent. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl

(including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, etc.

Unless otherwise specified, the terms "5- to 6-membered heteroaryl ring" and "5- to 6- membered heteroaryl" are used interchangeably. The term "5- to 6-membered heteroaryl" refers to a monocyclic group consisting of 5 to 6 ring atoms with a conjugated π-electron system, in which 1, 2, 3, or 4 ring atoms are heteroatoms independently selected from O, S, and N, and the rest are carbon atoms, Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, and 3-pyrrolyl, etc.), pyrazolyl (including 2-pyrazolyl and 3-pyrazolyl, etc.), imidazolyl (including N-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1*H*-1,2,3-triazolyl, 2*H*-1,2,3-triazolyl, 1*H*-1,2,4-triazolyl, and 4*H*-1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.), pyridyl (including 2-pyridyl, 3-pyridyl, and 4-pyridyl, etc.), pyrazinyl, pyrimidinyl (including 2-pyrimidinyl, 4-pyrimidinyl, etc.).

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuKα radiation as the light source and scanning mode: φ/ω scan, and after collecting the relevant data, the crystal structure is further analyzed by direct method (Shelxs97), so that the absolute configuration can be confirmed.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The present disclosure uses the following abbreviations: aq represents water; DCM represents dichloromethane; PE represents petroleum ether; DMSO represents dimethyl sulfoxide; EA and EtOAc both represent ethyl acetate; EtOH represents ethanol; MeOH represents methanol; DMF represents N,N-dimethylformamide; Boc represents *tert*-butoxycarbonyl, which is an amine protecting group; THF represents tetrahydrofuran; $Boc_2O$ represents di-tert-butyl dicarbonate; TFA represents trifluoroacetic acid; TEA represents triethylamine; HCl represents hydrochloric acid; mp represents melting point; FDPP represents pentafluorophenyl diphenylphosphinate; prep-HPLC represents preparative high-performance liquid chromatography; THP represents 2-chloro-tetrahydro-2*H*-pyran; BRETTPHOS represents 2-(dicyclohexylphosphino)-3,6-dimethoxy-2',4',6'-triisopropyl-1,1'-biphenyl; and TBAF represents tetrabutylammonium fluoride.

The solvents used in the present disclosure are commercially available. The compounds of the present disclosure are named according to the conventional naming principles in the art or by ChemDraw® software, and the commercially available compounds use the supplier catalog names.

## DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred embodiments include but are not limited to the examples of the present disclosure. It will be apparent to those skilled in the art that various variations and improvements can be made to specific embodiments of the present disclosure without departing from the spirit and scope of the present disclosure.

### Reference example 1: Compound B-1

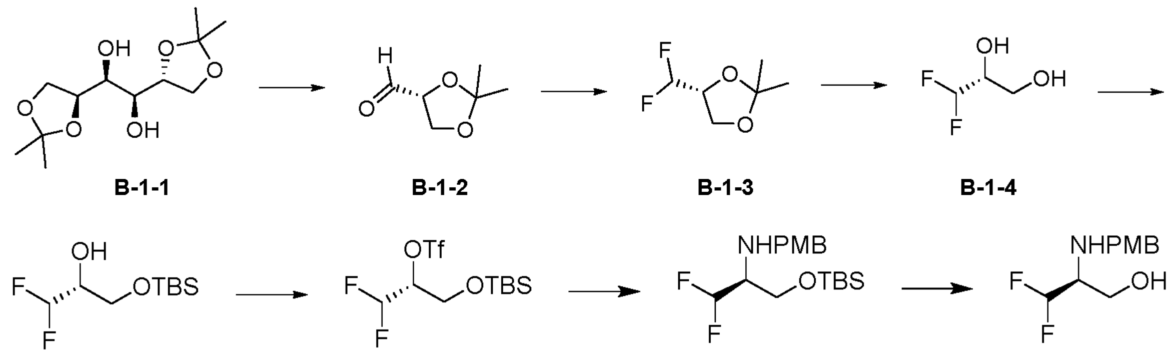

B-1-1 B-1-2 B-1-3 B-1-4

B-1-5 B-1-6 B-1-7 B-1

Step 1

**B-1-1** (90 g, 343.12 mmol) was dissolved in dichloromethane (700 mL), then sodium periodate (169.8 g, 793.86 mmol) and 6 mL of saturated sodium bicarbonate solution were sequentially added at 24°C, and sodium bicarbonate (12 g, 142.85 mmol) was added 5 minutes later. After the addition was completed, the water bath was removed. After the reaction mixture was stirred at room temperature for 2 hours, magnesium sulfate (180 g, 1.50 mol) was added. The reaction mixture was filtered, and the filter cake was washed with dichloromethane (2 * 180 mL). The filtrate was collected to obtain a crude dichloromethane solution of **B-1-2,** which was directly used for the next step.

Step 2

Diethylaminosulfur trifluoride (686.18 mmol, 90.66 mL) was added dropwise to the crude dichloromethane solution of **B-1-2** obtained from step 1 at 24°C, and the reaction mixture was stirred at 20°C for 14 hours. The resulting reaction mixture was poured in small portions repeatedly into a sodium bicarbonate solution dissolved in crushed ice to quench the reaction. The phases were separated, and the organic phase was sequentially washed with water (1000 mL) and saturated brine (500 mL), dried over anhydrous sodium sulfate, filtered, and the filter residue was rinsed with dichloromethane (150 mL). The resulting filtrate was concentrated under reduced pressure to obtain compound **B-1-3.** $^1$H NMR (400 MHz, $CDCl_3$): $\delta$ ppm 5.88 - 5.55 (m, 1H), 4.25 - 4.22 (m, 1H), 4.16 - 4.11 (m, 1H), 4.10 - 4.06 (m, 1H), 1.47 (s, 3H), 1.39 (s, 3H).

Step 3

**B-1-3** (22 g, 144.60 mmol) was dissolved in acetonitrile (190 mL) and $H_2O$ (10 mL), then bis(acetonitrile) dichloropalladium(II) (750.30 mg, 2.89 mmol) was added, and the reaction mixture was stirred at 60°C for 5 hours under $N_2$ atmosphere. After cooling to room temperature, the reaction mixture was filtered, and concentrated under reduced pressure to obtain a crude product of **B-1-4.**

Step 4: Synthesis of**B-1-5**

The crude product of **B-1-4** (22 g) was dissolved in dichloromethane (400 mL), then imidazole (11.81 g, 173.53 mmol) and *tert*-butyldimethylsilyl chloride (23.97 g, 159.07 mmol) were sequentially added at 0°C, and the reaction mixture was warmed to room temperature and stirred for 2 hours. The reaction mixture was diluted with dichloromethane (600 mL) and washed with 400 mL of water. The resulting organic phase was washed with saturated brine (300 mL), dried over anhydrous sodium sulfate, and filtered. The filtrate was concentrated to obtain a crude product. The crude product was purified by column chromatography (petroleum ether: ethyl acetate = 1:0 to 10:1) to obtain compound **B-1-5.** $^1$H NMR (400 MHz, DMSO-$d_6$): $\delta$ ppm 5.99 - 5.67 (m, 1H), 5.57 (d, *J*=5.6 Hz, 1H), 3.74 - 3.53 (m, 3H), 0.84 (s, 9H), 0.02 (s, 6H).

Step 5: Synthesis of**B-1-6**

**B-1-5** (26.2 g, 115.76 mmol) was dissolved in dichloromethane (400 mL), and then pyridine (185.21 mmol, 14.95 mL) and trifluoromethanesulfonic anhydride (138.91 mmol, 22.92 mL) were sequentially added at -30°C to -20°C. After the addition was completed, the reaction mixture was stirred at 0°C for 2 hours. The reaction mixture was diluted with dichloromethane (400 mL) and washed with 300 mL of water. The resulting organic phase was washed with saturated brine (300 mL), dried over anhydrous sodium sulfate, and filtered. The filtrate was concentrated to obtain a crude product.

The crude product was purified by column chromatography (petroleum ether: ethyl acetate = 1:0 to 10:1) to obtain compound **B-1-6.** $^1$H NMR (400 MHz, $CDCl_3$): $\delta$ ppm 6.20 - 5.85 (m, 1H), 4.93 - 4.82 (m, 1H), 4.03 - 3.97 (m, 2H), 0.91 (s, 9H), 0.11 (s, 6H).

Step 6: Synthesis of**B-1-7**

**B-1-6** (28.94 g, 80.75 mmol) was dissolved in 1,4-dioxane (250 mL), and then 4-methoxybenzylamine (96.90 mmol, 12.54 mL) and triethylamine (96.90 mmol, 13.49 mL) were sequentially added, and the reaction mixture was stirred at 90°C for 8 hours. After the reaction mixture was cooled to room temperature, the reaction mixture was added with water (300 mL) and extracted with ethyl acetate (400 mL * 2). The organic phases were combined, washed with saturated brine (300 mL), dried over anhydrous sodium sulfate, filtered, and the resulting filtrate was concentrated to obtain a crude product. The crude product was purified by column chromatography (petroleum ether: ethyl acetate = 1:0 to 10:1) to obtain compound **B-1-7.** $^1$H NMR (400 MHz, $CDCl_3$): $\delta$ ppm 7.22 - 7.20 (m, 2H), 6.86 - 6.81 (m, 2H), 5.90 - 5.58 (m, 1H), 3.87 - 3.75 (m, 2H), 3.75 (s, 3H), 3.68 - 3.64 (m, 2H), 2.89 - 2.84 (m, 1H), 0.83 (s, 9H), 0.02 (s, 6H).

Step 6: Synthesis of **B-1**

**B-1-7** (26.99 g, 78.12 mmol) was dissolved in tetrahydrofuran (280 mL), then tetrabutylammonium fluoride solution (1 M, 78.12 mL) was added, and the reaction mixture was stirred at 24°C for 2 hours. The reaction mixture was diluted with ethyl acetate (500 mL * 3) and washed with 300 mL of water. The resulting organic phase was washed with saturated brine (200 mL), dried over anhydrous sodium sulfate, and filtered. The filtrate was concentrated under reduced pressure to obtain a crude product. The crude product was purified by column chromatography (petroleum ether: ethyl acetate = 1:0 to 0:1) to obtain **B-1.** $^1$H NMR (400 MHz, $CDCl_3$): $\delta$ ppm 7.20 - 7.16 (m, 2H), 6.86 - 6.75 (m, 2H), 5.95 - 5.56 (m, 1H), 3.85 - 3.75 (m, 2H), 3.73 (s, 3H), 3.71 - 3.63 (m, 1H), 3.56 - 3.49 (m, 1H), 2.98 - 2.85 (m, 1H).

**Reference example 2: Compound B-2**

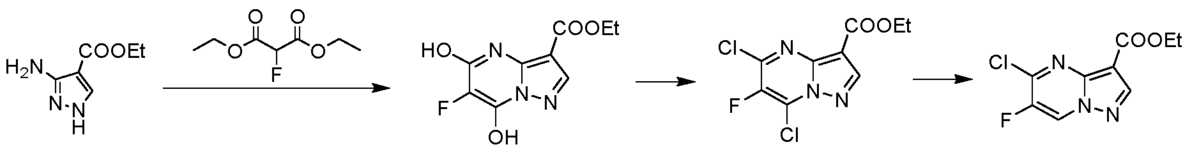

B-2-1 B-2-2 B-2-3 B-2

Step 1: Synthesis of**B-2-2**

**B-2-1** (100 g, 644.52 mmol) was dissolved in ethanol (1000 mL), and then diethyl fluoromalonate (172.24 g, 966.78 mmol) and sodium ethoxide (109.65 g, 1.61 mol) were sequentially added. The reaction mixture was heated to 90°C and stirred for 6 hours. After cooling to room temperature, the reaction mixture was diluted with water (1000 mL), and the pH was adjusted to 3 using 2N hydrochloric acid solution. The solid precipitate was filtered, and the filter cake was collected and stirred with 1.5 L of acetonitrile, then filtered. The filter cake was further stirred with 1.0 L of acetonitrile, followed by filtration. The filter cake was collected to obtain **B-2-2.** $^1$H NMR (400 MHz, DMSO-$d_6$): $\delta$ ppm 7.84 (s, 1H), 4.23(q, J=7.0 Hz, 2H), 1.27(t, J=7.0 Hz, 3H); LCMS: m/z=242.1 $[M+1]^+$.

Step 2: Synthesis of**B-2-3**

Phosphorus oxychloride (11.4 mol, 1.06 L) was added to a reaction flask equipped with a buffer device, and **B-2-2** (110 g, 456.1 mmol) was added in batches. After purging with nitrogen, the reaction mixture was cooled to 0°C, and *N,N*-diethylaniline (684.15 mmol, 109.43 mL) was slowly added dropwise. The reaction was heated to 100°C and stirred for 3 hours. After cooling to room temperature, the reaction mixture was diluted with 250 mL of acetonitrile and then quenched by slowly adding into 2 L of room temperature water. The resulting solution was placed in an ice-water bath and stirred for 1 hour, filtered, and the filter cake was collected and dissolved in 1 L of dichloromethane. 500 mL of saturated sodium bicarbonate aqueous solution was added, and stirred for 1 hour. The phases were separated. The resulting organic phase was concentrated under reduced pressure to obtain **B-2-3.** $^1$H NMR (400 MHz, $CDCl_3$): $\delta$ ppm 8.65 (s, 1H), 4.43 (q, *J*=7.0 Hz, 2H), 1.43 (t, *J*=7.0 Hz, 3H); LCMS: m/z=278.0 $[M+1]^+$.

Step 3: Synthesis of **B-2**

**B-2-3** (3.9 g, 14.03 mmol) was dissolved in tetrahydrofuran (50.0 mL), water (100.0 mL), and ethanol (150.0 mL), and then ammonium chloride (4.05 g, 75.74 mmol) was added. After purging with nitrogen, the reaction mixture was cooled to 0°C, and zinc powder (3.7 g, 56.1 mmol) was added in batches. The reaction was carried out at 0°C for 2 hours. The reaction mixture was filtered, and the filtrate was extracted with dichloromethane (50 mL * 3). The resulting organic phase was washed with saturated brine (20.0 mL), dried over anhydrous sodium sulfate, and filtered. The filtrate was concentrated under reduced pressure to obtain a crude product. The crude product was purified by column chromatography (eluent PE: EA = 1:0 to 3:1) to obtain **B-2.** $^1$H NMR (400 MHz, $CDCl_3$): $\delta$ ppm 8.65 (d, *J*=3.2 Hz, 1H), 8.50 (s, 1H), 4.36 (q, 2H), 1.36 (t, 3H); LCMS: m/z=244.0 $[M+1]^+$.

**Reference example 3: Compound B-3**

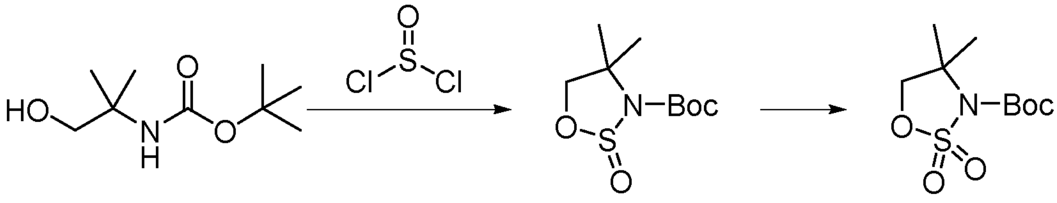

**B-3-1** **B-3-2** **B-3**

Step 1: Synthesis of**B-3-2**

Thionyl chloride (74.44 mmol, 5.4 mL) was dissolved in acetonitrile (35 mL), and **B-3-1** (5.0 g, 26.42 mmol) dissolved in acetonitrile (25 mL) was slowly added dropwise at -40°C. After stirring evenly, pyridine (142.48 mmol, 11.5 mL) was added and stirred at room temperature (about 15°C) for 2 hours. 150.0 mL of ethyl acetate was added to the reaction mixture, filtered, and the filtrate was concentrated under reduced pressure. The resulting crude product was purified by column chromatography (PE: EA = 1: 1) obtain compound **B-3-2.** $^1$H NMR (400 MHz, $CDCl_3$): $\delta$ ppm 4.77 - 4.56 (m, 1H), 4.35 - 4.33 (m, 1H), 1.52 (s, 6H), 1.46 (s, 9H); LCMS: m/z= 258.1 $[M+Na]^+$.

Step 2: Synthesis of **B-3**

**B-3-2** (1.0 g, 4.25 mmol) and ruthenium(III) chloride hydrate (4.4 mg, 19.7 μmol) were dissolved in acetonitrile (10.5 mL), and sodium periodate (5.3 g, 24.8 mmol) dissolved in 10.5 mL of water was added under a nitrogen atmosphere at 0°C, and then the reaction mixture was warmed to room temperature (about 20°C) and stirred for 16 hours. The reaction mixture was diluted with 100 mL of water and extracted with dichloromethane (100 mL * 3). The resulting organic phase was washed with 100 mL of saturated brine, dried over anhydrous sodium sulfate, filtered, and the filtrate was concentrated under reduced pressure. The resulting crude product was purified by column chromatography (PE: EA = 1: 1) to obtain **B-3.** $^1$H NMR (400 MHz, $CDCl_3$): $\delta$ ppm 4.23 (s, 2H), 1.59 (s, 9H), 1.56 (s, 6H); LCMS: m/z=252.1 $[M+1]^+$.

**Reference example 4: Compound B-4**

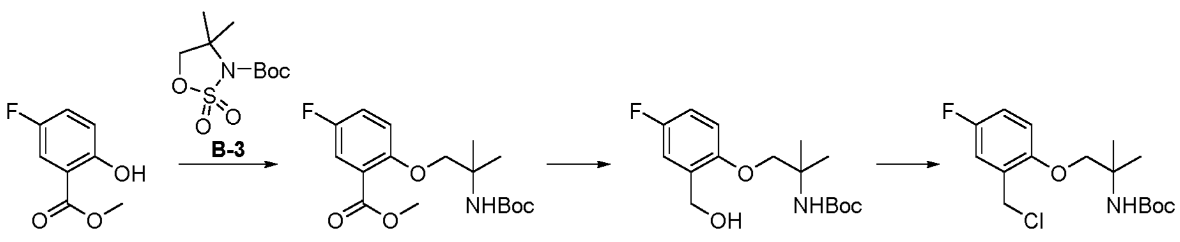


**B-4-1** **B-4-2** **B-4-3** **B-4**

Step 1: Synthesis of**B-4-2**

**B-4-1** (0.5 g, 14.03 mmol) and **B-3** (0.74 g, 2.94 mmol) were dissolved in DMF (10.0 mL), then potassium carbonate (1.02 g, 7.35 mmol) was added, and the reaction mixture was heated to 50°C and stirred for 4 hours. The reaction mixture was diluted with 5 mL of dichloromethane, filtered, and the filtrate was concentrated under reduced pressure to obtain a crude product. The crude product was purified by column chromatography (PE: EA= 10:1) to obtain **B-4-2.** $^1$H NMR (400 MHz, $CDCl_3$): $\delta$ ppm 7.49 - 7.44 (m, 1H), 7.12 -7.04 (m, 1H), 6.89 - 6.84 (m, 1H), 3.91 (s, 2H), 3.84 (s, 3H), 1.36 (s, 6H), 1.33 (s, 9H); LCMS: m/z= 364.1 $[M+Na]^+$.

Step 2: Synthesis of**B-4-3**

**B-4-2** (0.3 g, 0.88 mmol) was dissolved in tetrahydrofuran (4.0 mL), then $LiBH_4$ (38.3 mg, 1.76 mmol) was added at 0°C. After the addition was completed, the reaction mixture was stirred at room temperature for 18 hours. 5.0 mL of water was added to quench the reaction, and then 1 mL of 2 M sodium hydroxide solution was added, and the mixture was extracted with dichloromethane (10 mL * 3). The resulting organic phase was washed with 5.0 mL of saturated brine, dried over anhydrous sodium sulfate, filtered, and the filtrate was concentrated under reduced pressure to obtain a crude product. The crude product was purified by column chromatography (PE: EA = 10:1) to obtain **B-4-3.** $^1$H NMR (400 MHz, $CDCl_3$): $\delta$ ppm 7.09 - 7.05 (m, 1H), 6.97 - 6.90 (m, 1H), 6.86 - 6.79 (m, 1H), 4.73 - 4.71 (m, 1H), 4.71 - 4.67 (m, 2H), 4.03 (s, 2H), 1.43 (s, 6H), 1.41 (s, 9H); LCMS: m/z= 336.1 $[M+Na]^+$.

Step 3: Synthesis of **B-4**

**B-4-3** (0.1 g, 0.32 mmol) was dissolved in dichloromethane (3.0 mL), and *N,N*-diisopropylethylamine (0.96 mmol, 167 μL) was added. Methanesulfonyl chloride (0.41 mmol, 32.0 μL) was added at 0°C, and then the reaction mixture was warmed to room temperature (25°C) and stirred for 18 hours. The reaction was quenched by the addition of 3 mL of 2 M hydrochloric acid solution at 0°C, diluted with 15 mL of water, and extracted with dichloromethane (15 mL * 3). The organic phase was washed with saturated brine, dried over anhydrous sodium sulfate, filtered, the filtrate was concentrated under reduced pressure to obtain a crude product. The crude product was purified by column chromatography (PE: EA = 10:1) to obtain **B-4.** $^1$H NMR (400 MHz, $CDCl_3$): $\delta$ ppm 7.03 - 6.98 (m, 1H), 6.93 - 6.86 (m, 1H), 6.79 - 6.72 (m, 1H), 4.53 (s, 2H), 3.96 (s, 2H), 1.37 (s, 6H), 1.33 (s, 9H); LCMS: m/z= 354.0 $[M+Na]^+$.

**Reference example 5: Compound B-5**

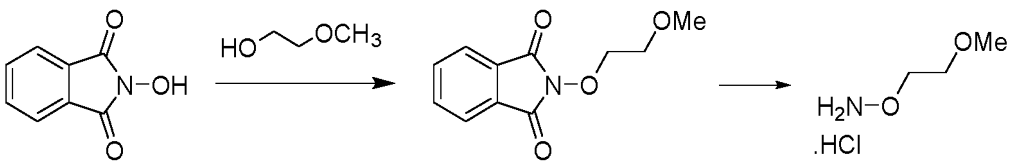

**B-5-1** **B-5-2** **B-5**

Step 1: Synthesis of **B-5-2**

**B-5-1** (7.07 g, 43.37 mmol), ethylene glycol methyl ether (3 g, 39.42 mmol), and triphenylphosphine (11.37 g, 43.37 mmol) were dissolved in tetrahydrofuran (85 mL), and a solution of diisopropyl azodicarboxylate (51.25 mmol, 9.97 mL) in tetrahydrofuran (15 mL) was slowly added dropwise into the reaction mixture under an ice bath, reacted at 0°C for 10 minutes, and then slowly warmed to 25°C and stirred for 16 hours. The reaction mixture was slowly added with water (30 mL) to quench the reaction and extracted with EA (200 mL * 2). The resulting organic phase was dried over anhydrous sodium sulfate, filtered, and the filtrate was concentrated under reduced pressure to obtain a crude product. The crude product was purified by column chromatography (PE: EA = 10:1 to 1:1) to obtain **B-5-2.** $^1$H NMR (400 MHz, $CDCl_3$) $\delta$ ppm 7.81 - 7.89 (m, 2 H), 7.72 - 7.79 (m, 2 H), 4.30 - 4.44 (m, 2 H), 3.67 - 3.82 (m, 2 H), 3.34 - 3.45 (s, 3 H); LCMS: m/z=222.0 $[M+1]^+$.

Step 2: Synthesis **of B-5**

**B-5-2** was dissolved in DCM (10 mL), then hydrazine hydrate (4.97 mmol, 302.10 μL, purity of 80%) was added,

and the reaction mixture was reacted at 25°C for 16 hours. The reaction mixture was filtered, and the resulting filtrate was concentrated under reduced pressure. The residue was added with DCM (20 mL), stirred, and filtered, and the filtrate was collected and concentrated under reduced pressure. The residue was added to DCM (5 mL), and then a solution of ethyl acetate in hydrochloric acid (4 M, 5 mL) was added, and the reaction mixture was stirred for 20 minutes. The reaction mixture was concentrated under reduced pressure. The residue was added with ethyl acetate (20 mL) and then concentrated, and the residue was added with ethyl acetate (20 mL) again and then concentrated to obtain **B-5.**

**Reference example 6: Compound B-6**

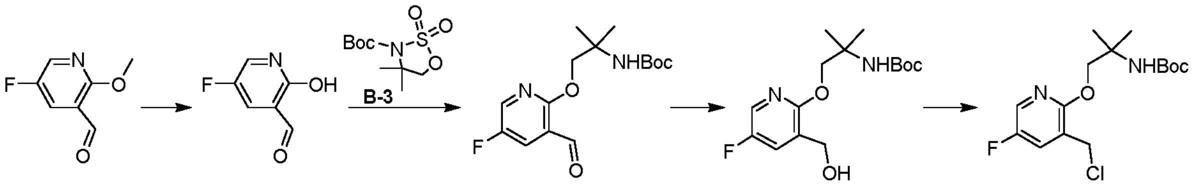


B-6-1 B-6-2 B-6-3 B-6-4 B-6

Step 1: Synthesis **of B-6-2**

**B-6-1** (5 g, 32.23 mmol) and pyridine hydrochloride (14.90 g, 128.93 mmol) were added to a round bottom flask, heated to 145°C, and reacted for 1 hour. After the reaction mixture was cooled to room temperature, the reaction mixture was added with water (200 mL) and then extracted with EA (500 mL * 2). The resulting organic phase was washed with saturated brine (100 mL), dried over anhydrous sodium sulfate, and filtered, and the resulting filtrate was concentrated under reduced pressure to obtain **B-6-2.** $^1$H NMR (400 MHz, $CDCl_3$) $\delta$ ppm 10.20 (s, 1 H), 7.93 - 8.02 (m, 1 H), 7.83 - 7.87 (m, 1 H).

Step 2: Synthesis **of B-6-3**

**B-6-2** (3.0 g, 21.26 mmol) was dissolved in DMF (30 mL), then **B-3** (4.86 g, 19.33 mmol) and potassium carbonate (6.68 g, 48.32 mmol) were sequentially added, and the reaction mixture was heated to 50°C and stirred for 14 hours. The reaction mixture was filtered, the filter cake was rinsed with EA (20 mL), and the resulting filtrate was concentrated under reduced pressure to obtain a crude product. The crude product was purified by column chromatography (PE: EA = 10: 1 to 4:1) to obtain **B-6-3.** LCMS: m/z=213.1 $[M+1-100]^+$.

Step 3: Synthesis **of B-6-4**

**B-6-3** (1.9 g, 6.08 mmol) was dissolved in EtOH (20 mL), then sodium borohydride (1.15 g, 30.42 mmol) was added under a nitrogen atmosphere at 0°C, and the reaction mixture was reacted at 25°C for 3 hours. Water (10 mL) was added to the reaction mixture at 0°C and stirred for 0.5 hours. After complete quenching, the reaction mixture was extracted with EA (200 mL * 2). The organic phase was dried over anhydrous sodium sulfate and filtered, and the resulting filtrate was concentrated under reduced pressure to obtain a crude product. The crude product was purified by column chromatography (PE: EA = 10:1 to 5:1) to obtain **B-6-4.** $^1$H NMR (400 MHz, $CDCl_3$) $\delta$ ppm 7.95 (d, *J*=3.01 Hz, 1 H), 7.57 (dd, *J*=8.78, 3.01 Hz, 1 H), 6.62 (brs, 1 H), 5.37 (t, *J*=5.52 Hz, 1 H), 4.48 (d, *J*=5.52 Hz, 2 H), 4.25 (s, 2 H), 1.33 (s, 9 H), 1.25 (s, 6 H); LCMS: m/z=315.1 $[M+1]^+$.

Step 4: Synthesis **of B-6**

**B-6-4** (1 g, 3.18 mmol) was dissolved in DCM (20 mL), then *N,N*-diisopropylethylamine (9.54 mmol, 1.66 mL) was added, and the mixture was cooled to 0°C under a nitrogen atmosphere. Then methanesulfonyl chloride (6.81 mmol, 527.03 $\mu$L) was slowly added dropwise. The reaction mixture was reacted at 0°C for 1 hour, and then warmed to 25°C and reacted for 2 hours. The reaction mixture was slowly added dropwise into an ice-water solution of saturated sodium bicarbonate (50 mL) and extracted with DCM (200 mL * 2). The resulting organic phase was dried over anhydrous sodium sulfate and filtered, and the resulting filtrate was subjected to rotary evaporation under reduced pressure to obtain a crude product. The crude product was purified by column chromatography (PE: EA = 10: 1 to 5:1) to obtain **B-6.** $^1$H NMR (400 MHz, $CDCl_3$) $\delta$ ppm 7.89-7.96 (m, 1 H), 7.45 - 7.48 (m, 1 H), 4.60 (s, 2 H), 4.35 (s, 2 H), 1.41 (s, 15H).

**Reference example 7: Compound B-7**

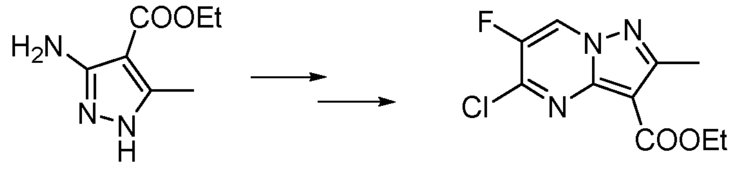

**B-7-1** **B-7**

Compound **B-7** was prepared from **B-7-1** using a synthetic route similar to that of compound **B-2.** LCMS: m/z=258.1 $[M+1]^+$.

**Reference example 8: Compound B-8**

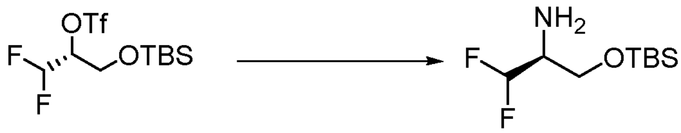

**B-1-6** **B-8**

**B-1-6** (5 g, 13.95 mmol) was dissolved in ammonia dioxane solution (0.4 M, 100 mL), and then triethylamine (2.33 mL, 16.74 mmol) was added. After purging with nitrogen, the reaction was carried out at 90°C for 12 hours. After the reaction mixture was cooled to room temperature, the reaction mixture was added with 50 mL of water and extracted with 50 mL of dichloromethane. The organic phase was collected after phase separation, and the aqueous phase was extracted with dichloromethane (50 mL * 3). The organic phases were combined, sequentially washed with saturated brine (50 mL * 3), dried over anhydrous sodium sulfate, and filtered. The filtrate was concentrated under reduced pressure to obtain a crude product. The crude product was purified by column chromatography (mobile phase PE: EA = 1:0 to 10:1) to obtain **B-8.** $^1$H NMR (400 MHz, $CDCl_3$): $\delta$ ppm 5.95 - 5.55 (m, 1H), 3.89 - 3.61 (m, 2H), 3.13 - 2.87 (m, 1H), 0.91 (s, 9H), 0.08 (s, 6H).

**Reference example 9: Compound B-9**

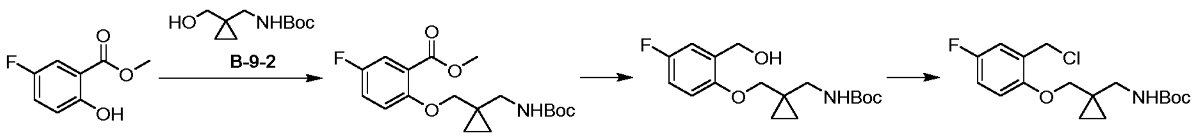


**B-4-1** **B-9-3** **B-9-4** **B-9**

Step 1: Synthesis **of B-9-3**

**B-4-1** (760 mg, 4.47 mmol) and **B-9-2** (899.03 mg, 4.47 mmol) were dissolved in 3 mL of dichloromethane, and then triphenylphosphine (1.76 g, 6.70 mmol) was added. The reaction mixture was cooled to 0°C, then added with diisopropyl azodicarboxylate (7.15 mmol, 1.39 mL), slowly warmed to 24°C, and reacted for 12 hours. The reaction mixture was quenched by adding 10 mL of water and extracted with 10 mL of dichloromethane. The organic phase was collected after phase separation, and the aqueous phase was extracted with dichloromethane (10 mL * 3). The organic phases were combined, washed with saturated brine (20 mL), dried over anhydrous sodium sulfate, filtered, and the filtrate was concentrated under reduced pressure to obtain a crude product. The crude product was purified by column chromatography (mobile phase PE: EA = 1:0 to 1:1) to obtain **B-9-3.** $^1$H NMR (400 MHz, $CDCl_3$): $\delta$ ppm 7.61 (dd, $J$ = 3.3, 8.9 Hz, 1H), 7.17 (ddd, $J$ = 3.3, 7.5, 9.1 Hz, 1H), 6.84 (dd, $J$ = 4.2, 9.2 Hz, 1H), 3.93 (s, 3H), 3.86 (s, 2H), 3.27 (d, $J$ = 5.4 Hz, 2H), 1.46 (s, 9H), 0.75 - 0.68 (m, 2H), 0.65 - 0.59 (m, 2H); LCMS: m/z= 254.2$[M-100+1]^+$.

Step 2: Synthesis **of B-9-4**

**B-9-3** (1.8 g, 3.01 mmol) and calcium chloride (333.52 mg, 3.01 mmol) were added to a reaction flask, then tetrahydrofuran (6 mL) and ethanol (12 mL) were added. The reaction mixture was cooled to 0°C under a nitrogen atmosphere, slowly added with sodium borohydride (227.38 mg, 6.01 mmol), and reacted at 24°C for 12 hours. The reaction mixture was slowly quenched with 20 mL of water, added with 2 M hydrochloric acid to adjust the pH to 6-7, and extracted with 20 mL of dichloromethane. The organic phase was collected after phase separation, and the aqueous phase was extracted with dichloromethane (2 * 20 mL), and the organic phases were combined. The organic phases were sequentially washed with saturated brine (20 mL), dried over anhydrous sodium sulfate, filtered, and the filtrate was concentrated under reduced pressure to obtain a crude product. The crude product was purified by column chromatography (mobile phase PE: EA = 1:0 to 2:1) to obtain **B-9-4.** $^1$H NMR (400 MHz, $CDCl_3$) $\delta$ ppm 7.07 (dd, *J* = 3.1, 8.6 Hz, 1H), 6.92 (dt, *J* = 3.2, 8.5 Hz, 1H), 6.71 (dd, *J* = 4.3, 8.9 Hz, 1H), 6.33 (br s, 1H), 5.23 - 5.09 (m, 1H), 4.71 (s, 2H), 3.82 (s, 2H), 3.26 (br d, *J* = 4.5 Hz, 2H), 1.39 (s, 9H), 0.67 - 0.57 (m, 4H); LCMS: m/z= 348.2 [M+Na] $^+$.

Step 3: Synthesis of **B-9**

**B-9-4** (0.65 g, 2.00 mmol) was dissolved in dichloromethane (13 mL), cooled to 0°C under a nitrogen atmosphere, then thionyl chloride (4.00 mmol, 290.19 μL) was added, and the reaction mixture was reacted at 20°C for 1 hour. The reaction mixture was added with 5 mL of water to quench the reaction, added with saturated sodium bicarbonate solution to adjust the pH to 7-8, and extracted with 10 mL of dichloromethane. The organic phase was collected after phase separation, and the aqueous phase was extracted with dichloromethane (10 mL * 3). The organic phases were combined, sequentially washed with saturated brine (20 mL), dried over anhydrous sodium sulfate, filtered, and the filtrate was concentrated under reduced pressure to obtain a crude product. The crude product was purified by column chromatography (mobile phase PE: EA = 1:0 to 1:1) to obtain **B-9.** $^1$H NMR (400 MHz, $CDCl_3$) $\delta$ ppm 7.10 (dd, *J* = 2.9, 8.5 Hz, 1H), 6.97 (dt, *J* = 3.0, 8.4 Hz, 1H), 6.76 (dd, *J* = 4.2, 8.7 Hz, 1H), 5.15 - 5.04 (m, 1H), 4.64 (s, 2H), 3.86 (s, 2H), 3.27 (br d, *J* = 5.5 Hz, 2H), 1.43 (s, 9H), 0.75 - 0.60 (m, 4H); LCMS: m/z= 366.1 [M+Na] $^+$.

**Reference example 10: Compound B-10**

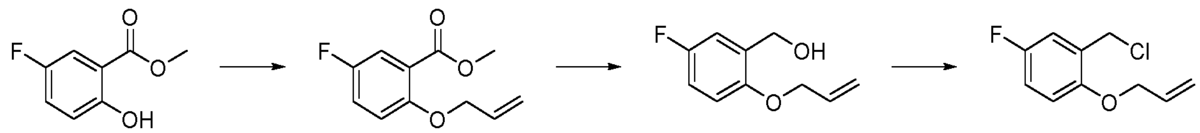

**B-4-1** **B-10-2** **B-10-3** **B-10**

Step 1: Synthesis of B-10-2

**B-4-1** (2.0 g, 11.76 mmol) was dissolved in *N,N*-dimethylformamide (6.67 mL), then 3-bromopropylene (1.56 g, 12.93 mmol), potassium carbonate (1.79 g, 12.93 mmol), and sodium iodide (1.94 g, 12.93 mmol) were sequentially added under a nitrogen atmosphere, and the reaction mixture was reacted at 20°C for 12 hours. The reaction mixture was added with 30 mL of water to quench the reaction and extracted with ethyl acetate (20 mL * 3). The organic phase was collected after phase separation, sequentially washed with saturated brine (20 mL), dried over anhydrous sodium sulfate, filtered, and the filtrate was concentrated under reduced pressure to obtain a crude product. The crude product was purified by column chromatography (mobile phase PE: EA= 1:0 to 6:1) to obtain **B-10-2.** $^1$H NMR (400 MHz, $CDCl_3$) $\delta$ ppm 7.53 (dd, *J* = 3.2, 8.8 Hz, 1H), 7.16 - 7.15 (m, 1H), 6.93 (dd, *J* = 4.0, 8.8 Hz, 1H), 6.07 - 6.03 (m, 1H), 5.50 (dd, *J* = 1.6, 17.2 Hz, 1H), 5.32 (dd, *J* = 1.6, 10.4 Hz, 1H), 4.61 (dd, *J* = 1.6, 3.2 Hz, 2H), 3.91 (s, 3H); LCMS: m/z= 211.2 [M+H] $^+$.

Step 2: Synthesis of **B-10-3**

**B-10-2** (2.0 g, 9.51 mmol) was dissolved in toluene (40.0 mL), cooled to 0°C under a nitrogen atmosphere, and a solution of diisobutylaluminum hydride in toluene (1 M, 28.54 mL) was slowly added dropwise, and the reaction mixture was stirred at 0°C for 1 hour. The reaction mixture was added with saturated sodium tartrate (100 mL) to quench the reaction and extracted with ethyl acetate (100 mL), and the aqueous phase was extracted with ethyl acetate (50 mL * 3). The organic phases were combined, sequentially washed with saturated brine (20 mL), dried over anhydrous sodium sulfate, filtered, and the filtrate was concentrated under reduced pressure to obtain a crude product. The crude product

was purified by column chromatography (mobile phase PE: EA= 1:0 to 10:1) to obtain **B-10-3.** $^1$H NMR (400 MHz, $CDCl_3$) $\delta$ ppm 7.06 (dd, *J* = 3.2, 8.8 Hz, 1H), 6.93 (td, *J* = 3.2, 4.4 Hz, 1H), 6.81 (dd, *J* = 4.4, 4.8 Hz, 1H), 6.07 - 6.02 (m, 1H), 5.44 (dd, *J* = 1.6, 15.6 Hz, 1H), 5.33 (dd, *J* = 1.6, 10.4 Hz, 1H), 4.70 (d, *J* = 2.8 Hz, 2H), 4.57 (d, *J* = 5.2 Hz, 2H), 2.25 (s, 1H).

Step 3: Synthesis **of B-10**

**B-10-3** (0.5 g, 2.74 mmol) was dissolved in dichloromethane (10.0 mL), then thionyl chloride (0.3 mL, 4.12 mmol) was added under a nitrogen atmosphere, and the reaction mixture was stirred at 20°C for 0.5 hours. The resulting reaction mixture was concentrated under reduced pressure to obtain a crude product, which was purified by column chromatography (mobile phase PE: EA= 1:0 to 20:1) to obtain **B-10.** $^1$H NMR (400 MHz, $CDCl_3$) $\delta$ ppm 7.13 (dd, *J* = 3.2, 8.8 Hz, 1H), 6.97 (td, *J* = 3.2, 4.4 Hz, 1H), 6.83 (dd, *J* = 4.4, 4.8 Hz, 1H), 6.07 - 6.02 (m, 1H), 5.45 (dd, *J* = 1.6, 17.2 Hz, 1H), 5.33 (dd, *J* = 1.6, 10.4 Hz, 1H), 4.65 (s, 2H), 4.58 (dd, *J* = 1.6, 3.2 Hz, 2H).

**Reference example 11: Compound B-11**

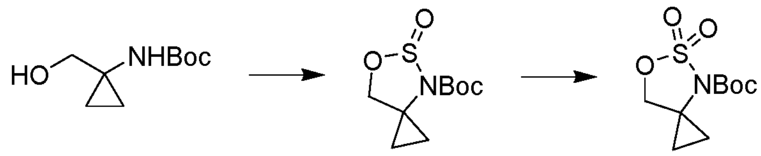

**B-11-1** **B-11-2** **B-11**

Step 1: Synthesis of **B-11-2**

Imidazole (7.27 g, 106.82 mmol), triethylamine (53.41 mmol, 7.43 mL), and dichloromethane (50 mL) were added to a reaction flask, cooled to -65°C under a nitrogen atmosphere. A solution of thionyl chloride (32.05 mmol, 2.33 mL) and **B-11-1** (5 g, 26.70 mmol) in dichloromethane (50 mL) was slowly added dropwise, and then the reaction mixture was slowly warmed to 25°C and stirred for 12 hours. The reaction mixture was added with 20 mL of water to quench the reaction and extracted with dichloromethane (20 mL * 3). The organic phase was collected after phase separation, sequentially washed with saturated brine (20 mL), dried over anhydrous sodium sulfate, filtered, and the filtrate was concentrated under reduced pressure to obtain a crude product of **B-11-2.** $^1$H NMR (400 MHz, $CDCl_3$) $\delta$ ppm 5.06 (dd, *J* = 1.2, 8.8 Hz, 1H), 4.15 (d, *J* = 8.8 Hz, 1H), 1.93 - 1.87 (m, 1H), 1.51 - 1.50 (m, 1H), 1.41 (s, 9H), 0.77 - 0.72 (m, 1H), 0.68 - 0.65 (m, 1H).

Step 2: Synthesis **of B-11**

**B-11-2** (6.2 g, 26.58 mmol), water (31 mL), and acetonitrile (62 mL) were added to a reaction flask, cooled to 0°C under a nitrogen atmosphere, and then sodium periodate (7.11 g, 33.22 mmol) and ruthenium(III) chloride (27.56 mg, 132.89 $\mu$mol) were added. The reaction mixture was slowly warmed to 20°C and then stirred for 2 hours. The reaction mixture was filtered, and the filtrate was extracted with ethyl acetate (50 mL * 3). The organic phases were combined, washed with saturated brine (50 mL), dried over anhydrous sodium sulfate, filtered, and the filtrate was concentrated under reduced pressure to obtain a crude product. The crude product was purified by column chromatography (mobile phase PE: EA = 1:0 to 3:1) to obtain **B-11.** $^1$H NMR (400 MHz, $CDCl_3$) $\delta$ ppm 4.43 (s, 2H), 1.88 (t, *J* = 6.8 Hz, 2H), 1.54 (s, 9H), 0.77 (t, *J* = 6.8 Hz, 2H).

**Reference example 12: Compound B-12**

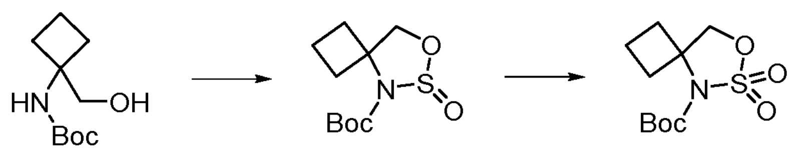

**B-12-1** **B-12-2** **B-12**

Step 1: Synthesis of **B-12-2**

Imidazole (1.01 g, 14.91 mmol) was dissolved in dichloromethane (16 mL), cooled to -5°C, then a solution of thionyl chloride (0.32 mL, 4.47 mmol) in dichloromethane (5 mL) was slowly added, and the reaction mixture was stirred at -5°C for 1 hour. After cooling to -10°C, a solution of **B-12-1** (0.5 g, 2.48 mmol) in dichloromethane (4 mL) was added, and the reaction mixture was returned to room temperature and stirred for 2 hours. The reaction mixture was added with water (10 mL) and extracted with dichloromethane (20 mL * 3). The organic phases were combined, sequentially washed with 10% citric acid solution (10 mL) and saturated brine (10 mL), dried over anhydrous sodium sulfate, filtered, and the filtrate was concentrated under reduced pressure to obtain a crude product. The crude product was purified by column chromatography (PE: EA = 1:0 to 1:1) to obtain **B-12-2.** $^1$H NMR (400 MHz, $CDCl_3$) $\delta$ ppm 4.95 (d, $J$ = 8.8 Hz, 1H), 4.80 (d, $J$ = 8.8 Hz, 1H), 2.12 - 2.08 (m, 2H), 1.93 - 1.83 (m, 2H), 1.67 - 1.64 (m, 2H), 1.58 (s, 9H).

Step 2: Synthesis **of B-12**

**B-12-2** (0.51 g, 2.06 mmol) was dissolved in acetonitrile (15 mL), cooled to 0°C under a nitrogen atmosphere, then a solution of sodium periodate (2.56 g, 11.96 mmol) and ruthenium(III) chloride trihydrate (2.7 mg, 10.31 μmol) in water (15 mL) was added, and the reaction mixture was slowly warmed to 20°C and stirred for 16 hours. The reaction mixture was filtered, and the filtrate was extracted with ethyl acetate (20 mL * 3). The organic phases were combined, washed with saturated brine (30 mL), dried over anhydrous sodium sulfate, filtered, and the filtrate was concentrated under reduced pressure to obtain a crude product of **B-12,** which was directly used for the next reaction without purification. $^1$H NMR (400 MHz, $CDCl_3$) $\delta$ ppm 4.82 (s, 2H), 2.89 - 2.86 (m, 2H), 2.22 - 2.16 (m, 2H), 1.78 - 1.73 (m, 2H), 1.51 (s, 9H).

**Reference example 13: Compound B-13**

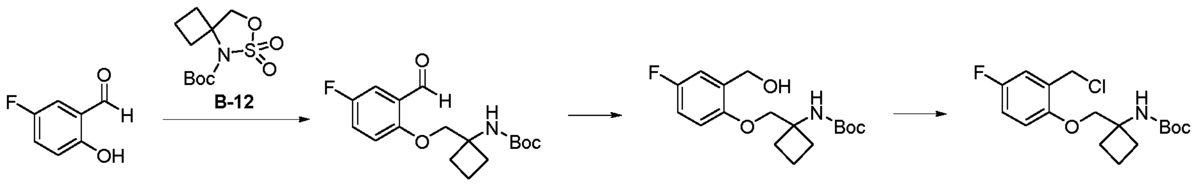


B-13-1 B-13-2 B-13-3 B-13

Step 1: Synthesis of **B-13-2**

**B-13-1** (276.0 mg, 1.97 mmol) and **B-12** (415.0 mg, 1.58 mmol) were dissolved in *N,N'*-dimethylformamide, and then cesium carbonate (1.28 g, 3.94 mmol) was added. The reaction mixture was heated to 70°C and stirred for 16 hours. After cooling to room temperature, the reaction mixture was quenched by adding water (50 mL) and extracted with ethyl acetate (20 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by column chromatography (PE: EA = 10:0 to 5:1) to obtain **B-13-2.** $^1$H NMR (400 MHz, $CDCl_3$) $\delta$ ppm 7.51 (dd, $J$ = 3.2, 8.4 Hz, 1H), 7.25 - 7.23 (m, 1H), 7.04 - 7.00 (m, 1H), 4.87 (s, 1H), 4.27 (s, 2H), 2.30 - 2.04 (m, 4H), 2.04 - 2.02 (m, 1H), 1.93 - 1.88 (m, 1H),1.42 (s, 9H).

Step 2: Synthesis of **B-13-3**

**B-13-2** (350.0 mg, 1.08 mmol) was dissolved in methanol (5 mL), then sodium borohydride (60.0 mg, 1.59 mmol) was added at 0°C, and the reaction mixture was reacted at 24°C for 1 hour. The reaction was quenched with saturated ammonium chloride (20 mL) solution and extracted with ethyl acetate (20 mL * 3). The organic phases were combined, sequentially washed with saturated brine (20 mL * 3), dried over anhydrous sodium sulfate, filtered, and the filtrate was concentrated under reduced pressure to obtain a crude product of **B-13-3,** which was directly used for the next reaction without purification. $^1$H NMR (400 MHz, $CDCl_3$) $\delta$ ppm 7.05 - 7.03 (m, 1H), 6.94 - 6.92 (m, 1H), 6.84 (dd, $J$ = 4.4, 8.8 Hz, 1H), 4.89 (s, 1H), 4.67 (s, 2H), 4.14 (s, 2H), 2.32 - 2.23 (m, 4H), 1.91 - 1.89 (m, 1H), 1.86 - 1.84 (m, 1H),1.43 (s, 9H).

Step 2: Synthesis **of B-13**

**B-13-3** (345.0 mg, 1.06 mmol) was dissolved in dichloromethane (10 mL), then*N,N'*-diisopropylethylamine was

added, cooled to 0°C. Then methanesulfonyl chloride (250.0 μL, 3.23 mmol) was added dropwise and the reaction was carried out at 24°C for 16 hours. The reaction mixture was added with water (20 mL) to quench the reaction and extracted with dichloromethane (15 mL * 3). The organic phases were combined, sequentially washed with saturated brine (10 mL), dried over anhydrous sodium sulfate, filtered, and the filtrate was concentrated under reduced pressure to obtain a crude product of **B-13.** $^1$H NMR (400 MHz, $CDCl_3$) $\delta$ ppm 7.09 (dd, *J* = 3.2, 8.4 Hz, 1H), 6.99 - 6.98 (m, 1H), 6.97 - 6.87 (m, 1H), 4.97 (s, 1H), 4.61 (s, 2H), 4.16 (s, 2H), 2.35 - 2.27 (m, 4H), 1.92 - 1.89 (m, 1H), 1.87 - 1.86 (m, 1H),1.42 (s, 9H).

**Reference example 14: Compound B-14**

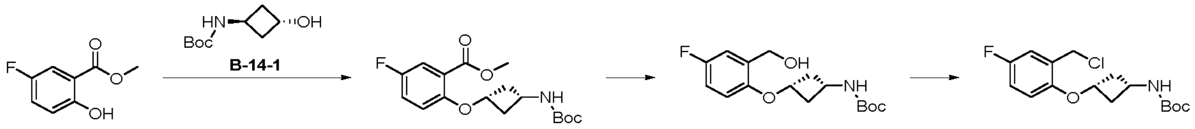


B-4-1 B-14-2 B-14-3 B-14

Step 1: Synthesis of **B-14-2**

**B-4-1** (5.45 g, 32.05 mmol), **B-14-1** (5.0 g, 26.7 mmol), and triphenylphosphine (10.51 g, 40.06 mmol) were added to dichloromethane (60 mL), cooled to 0°C and then diisopropyl azodicarboxylate (8.64 g, 42.73 mmol) was added, and the reaction mixture was stirred at 20°C for 4 hours. The reaction mixture was quenched with water (100 mL) and extracted with dichloromethane (100 mL * 2). The organic phases were combined, sequentially washed with saturated brine (100 mL), dried over anhydrous sodium sulfate, filtered, and the filtrate was concentrated under reduced pressure to obtain a crude product. The crude product was purified by column chromatography (PE: EA = 1:0 to 10:1) to obtain **B-14-2.** $^1$H NMR (400 MHz, $CDCl_3$) $\delta$ ppm 7.50 (1H, *J*=3.2 Hz, *J*=8.4 Hz, dd), 7.15 - 7.10 (m, 1H), 6.74 (1H, *J*=4.4 Hz, *J*=8.4 Hz, dd), 4.88 - 4.84 (m, 1H), 4.43 - 4.39 (m, 1H), 3.89 (s, 3H), 2.95 - 2.94 (m, 2H), 2.09 - 2.05 (m, 2H), 1.33 (s, 9H).

Step 2: Synthesis of **B-14-3**

**B-14-2** (2.0 g, 5.89 mmol) was dissolved in tetrahydrofuran (20 mL), then sodium borohydride (0.3 g, 13.77 mmol) was added at 0°C, and the reaction mixture was reacted at 24°C for 30 hours. The reaction was quenched with water (50 mL) and extracted with ethyl acetate (50 mL * 3). The organic phases were combined, sequentially washed with saturated brine (50 mL), dried over anhydrous sodium sulfate, filtered, and the filtrate was concentrated under reduced pressure to obtain a crude product. The crude product was purified by column chromatography (PE: EA= 1:0 to 4:1) to obtain **B-14-3.** LCMS: m/z =334.2 $[M+Na]^+$.

Step 3: Synthesis **of B-14**

**B-14-3** (1.47 g, 4.72 mmol) was dissolved in dichloromethane (30 mL), then *N,N'*-diisopropylethylamine (3.29 mL, 18.89 mmol) was added, cooled to 0°C. Then methanesulfonyl chloride (763.5 μL, 9.86 mmol) was added dropwise and the reaction was carried out at 20°C for 16 hours. The reaction mixture was added with water (50 mL) to quench the reaction and extracted with dichloromethane (50 mL * 3). The organic phases were combined, sequentially washed with saturated brine (50 mL), dried over anhydrous sodium sulfate, filtered, and the filtrate was concentrated under reduced pressure to obtain a crude product. The crude product was purified by column chromatography (PE: EA = 1:0 to 4:1) to obtain **B-14.** LCMS: m/z =352.1 $[M+Na]^+$.

**Reference example 14: Compound B-15**

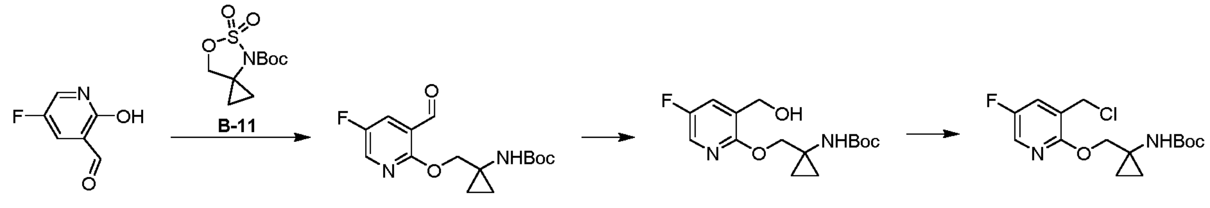


B-6-2 B-15-1 B-15-2 B-15

Step 1: Synthesis of **B-15-1**

**B-6-2** (3.1 g, 21.97 mmol) and **B-11** (6.02 g, 24.17 mmol) were dissolved in DMF (32 mL), then potassium carbonate (7.59 g, 54.93 mmol) slowly added at 0°C, and the reaction mixture was slowly warmed to 20°C and stirred for 10 hours. The reaction mixture was quenched with saturated brine (30 mL), added with 2N hydrochloric acid to adjust the pH to about 3, and extracted with ethyl acetate (30 mL). The aqueous phase was extracted with dichloromethane (20 mL * 6), and the organic phases were combined, sequentially dried over anhydrous sodium sulfate, filtered, and the filtrate was concentrated under reduced pressure to obtain a crude product. The crude product was purified by column chromatography (developing solvent PE: EA= 1:0 to 1:1) to obtain **B-15-1,** $^1$H NMR (400 MHz, $CDCl_3$) $\delta$ ppm 10.39 (d, *J*=3.1 Hz, 1 H), 8.19 (d, *J*=3.3 Hz, 1 H), 7.84 (dd, *J* = 3.2, 7.4 Hz, 1 H), 5.04 (br s, 1H), 4.48 (s, 2 H), 1.46 - 1.36 (m, 9 H), 0.95 (s, 4 H); LCMS: m/z= 333.1 $[M+Na]^+$.

Step 2: Synthesis of **B-15-2**

**B-15-1** (2.77 g, 8.93 mmol) was dissolved in tetrahydrofuran (30 mL), then sodium borohydride (0.47 g, 12.5 mmol) was added under a nitrogen atmosphere at 0°C, and the reaction mixture was reacted at 0°C for 1 hour. At 0°C, water (15 mL) was added to the reaction mixture and stirred for 0.5 hours. After complete quenching, the reaction mixture was added with 2N hydrochloric acid to adjust the pH to 6-7 and extracted with dichloromethane (50 mL * 3), and the organic phases were combined. The organic phase was sequentially washed with saturated brine (20 mL), dried over anhydrous sodium sulfate, filtered, and the filtrate was concentrated under reduced pressure to obtain a crude product of **B-15-2,** which was directly used for the next step without purification. $^1$H NMR (400 MHz, $CDCl_3$) $\delta$ ppm 7.86 (d, *J*=2.9 Hz, 1 H), 7.34 (brs, 1 H), 5.09 (brd, *J*=2.8 Hz, 1 H), 4.61 (brs, 2 H), 4.30 (s, 2 H), 1.43 (s, 9 H), 0.96-0.89 (m, 4 H); LCMS: m/z=313.2 $[M+1]^+$.

Step 4: Synthesis **of B-15**

**B-15-2** (2.5 g, 8.0 mmol) was dissolved in DCM (25 mL), then *N,N*-diisopropylethylamine (24.01 mmol, 4.18 mL) was added, and the mixture was cooled to 0°C under a nitrogen atmosphere. Then methanesulfonyl chloride (16.01 mmol, 1.24 mL) was slowly added dropwise. The reaction mixture was slowly warmed to 20°C and reacted for 12 hours. The reaction mixture was evaporated to remove the solvent under reduced pressure, and the crude product was purified by column chromatography (PE: EA = 9:1 to 1:1) to obtain **B-15.** $^1$H NMR (400 MHz, $CDCl_3$) $\delta$ ppm 7.92 (d, *J* = 2.9 Hz, 1H), 7.49 (dd, *J* = 2.9, 7.9 Hz, 1H), 5.21 - 4.82 (m, 1H), 4.60 (s, 2H), 4.39 (br s, 2H), 1.44 (br s, 9H), 0.92 (s, 4H); LCMS: m/z=331.1 $[M+1]^+$.

**Example 1**

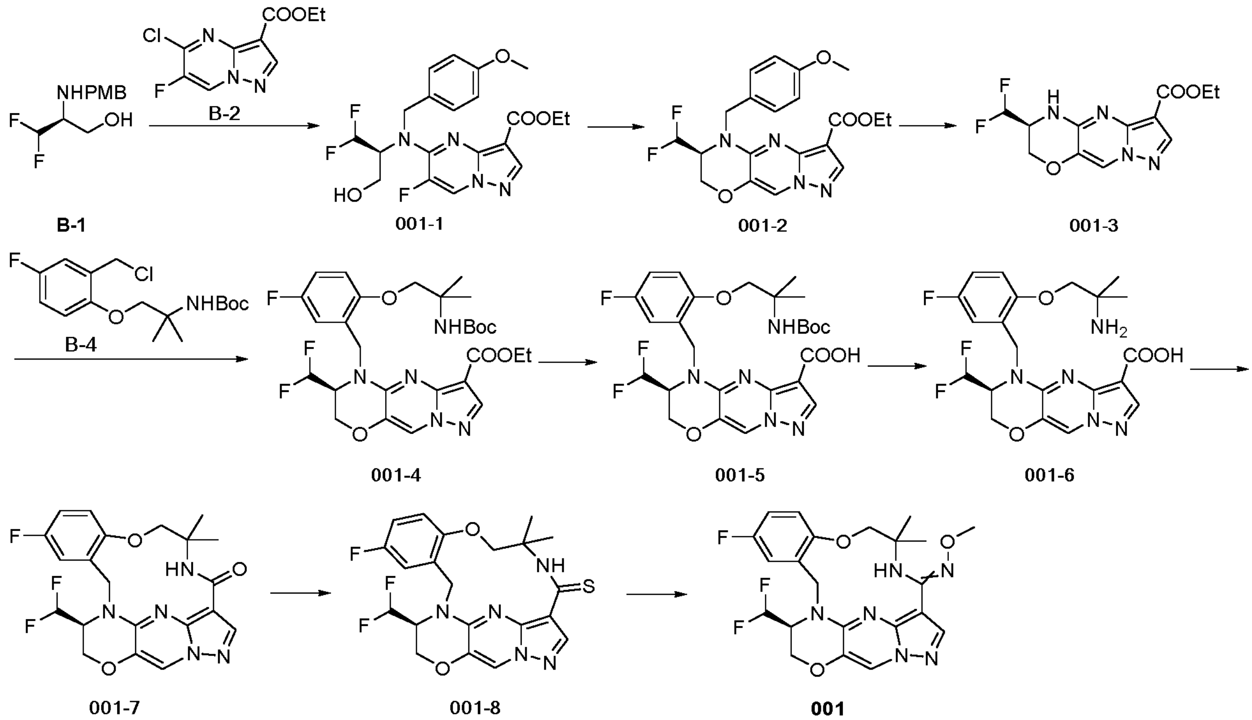

B-1 B-2 001-1 001-2 001-3

B-4 001-4 001-5 001-6

001-7 001-8 **001**

Step 1: Synthesis of Compound **001-1**

**B-1** (7.26 g, 31.4 mmol) and **B-2** (7.65 g, 31.4 mmol) were dissolved in DMSO (140.0 mL), then KF (6.38 g, 109.89 mmol) was added, and the reaction mixture was reacted at 120°C for 3 hours. The reaction mixture was washed with 300 mL of water and extracted with ethyl acetate (500 mL * 3). The resulting organic phase was washed with saturated brine (200 mL), dried over anhydrous sodium sulfate, and filtered. The filtrate was concentrated to obtain a crude product. The crude product was purified by column chromatography (PE: EA = 1:0 to 1:1) to obtain **001-1.** $^1$H NMR (400 MHz, $CDCl_3$): $\delta$ ppm 8.36 - 8.30 (m, 1H), 8.23 (s, 1H), 7.17 - 7.14 (m, 2H), 6.92 - 6.51 (m, 3H), 4.97 - 4.86 (m, 1H), 4.66 - 4.57 (m, 1H), 4.33 - 4.20 (m, 2H), 4.10 - 4.03 (m, 1H), 3.96 - 3.89 (m, 1H), 3.81 - 3.70 (m, 5H), 1.26 (t, *J*=7.2 Hz, 3H); LCMS: m/z=439.1 $[M+1]^+$.

Step 2: Synthesis of Compound **001-2**

**001-1** (0.4 g, 0.91 mmol) was dissolved in DMSO (16 mL), then cesium carbonate (1.19 g, 3.64 mmol) was added, and the reaction mixture was stirred at 26°C for 1 hour. The reaction mixture was washed with 50 mL of water and extracted with ethyl acetate (100 mL * 3). The resulting organic phase was washed with saturated brine (40 mL), dried over anhydrous sodium sulfate, and filtered. The filtrate was concentrated to obtain a crude product. The crude product was purified by column chromatography (PE: EA = 1:0 to 1:1) to obtain 001-2. $^1$H NMR (400 MHz, $CDCl_3$): $\delta$ ppm 8.31 (s, 1H), 8.14 (s, 1H), 7.36 (d, *J*=8.4 Hz, 2H), 6.88 (d, *J*=8.4 Hz, 2H), 6.16 - 6.08 (m, 1H), 6.08 - 5.76 (m, 1H), 4.55 - 4.47 (m, 1H), 4.41 - 4.32 (m, 2H), 4.25 -4.21 (m, 1H), 3.86 - 3.72 (m, 5H), 1.38 (t, *J* = 7.2 Hz, 3H); LCMS: m/z = 419.0 $[M+H]^+$.

Step 3: Synthesis of Compound **001-3**

**001-2** (0.448 g, 1.07 mmol) was dissolved in trifluoroacetic acid (8 mL) and stirred at 70°C for 7 hours. The reaction mixture was concentrated under reduced pressure to obtain a crude product, which was purified by column chromatography (PE: EA = 1:0 to 1:2) to obtain the trifluoroacetate salt of **001-3.** $^1$H NMR (400 MHz, $CDCl_3$): $\delta$ ppm 8.30 - 8.24 (m, 1H), 8.17 (s, 1H), 6.09 - 5.71 (m, 1H), 4.51 (dd, *J* = 2.0 Hz, *J* = 12.0 Hz, 1H), 4.34 (q, *J* = 7.2 Hz, 2H), 4.26 - 4.19 (m, 1H), 4.06 - 3.96 (m, 1H), 1.38 (t, *J* = 7.2 Hz, 3H); LCMS: m/z = 299.0 $[M+H]^+$.

Step 4: Synthesis of Compound **001-4**

**001-3** (0.11 g, 0.37 mmol) and **B-4** (0.15 g, 0.44 mmol) were dissolved in DMF (3 mL), then cesium carbonate (0.36 g, 1.11 mmol) was added, and the reaction mixture was stirred at 26°C for 3 hours. The reaction mixture was washed with 15 mL of water and extracted with ethyl acetate (50 mL * 3). The resulting organic phase was washed with saturated brine (30 mL), dried over anhydrous sodium sulfate, and filtered. The filtrate was concentrated to obtain a crude product.

The crude product was purified by column chromatography (PE: EA= 3:1) to obtain **001-4.** LCMS: m/z = 594.2 $[M+H]^+$.

Step 5: Synthesis of Compound **001-5**

**001-4** (0.05 g, 84.23 μmol) was dissolved in tetrahydrofuran (2 mL), ethanol (4 mL), and methanol (2 mL). Then lithium hydroxide (2 M, 4.0 mL) was added and stirred at 30°C for 20 hours. The reaction mixture was cooled to -20°C, quenched by the addition of 4.2 mL of 2 M hydrochloric acid solution, and then diluted by the addition of 20 mL of water. The reaction mixture was extracted with dichloromethane (20 mL * 3). The organic phase was washed with saturated brine, dried over anhydrous sodium sulfate, and the filtrate was concentrated under reduced pressure. The resulting crude product of **001-5** was directly used for the next step without purification. LCMS: m/z= 588.2 $[M+Na]^+$.

Step 6: Synthesis of Compound **001-6**

The crude product of **001-5** (0.045 g) was dissolved in dichloromethane (4 mL), and then hydrochloric acid/1,4-dioxane solution (4 M, 6.85 mL) was added. The reaction mixture was stirred at 20°C for 1 hour. The reaction mixture was concentrated under reduced pressure to obtain the crude hydrochloride salt of the crude product of **001-6,** which was directly used for the next step. LCMS: m/z = 466.1 $[M+H]^+$.

Step 7: Synthesis of Compound **001-7**

001-6 (38 mg, crude hydrochloride salt) was dissolved in dichloromethane (4 mL), and then *N,N*-diisopropylethylamine (731.5 μmol, 127.4 μL) and pentafluorophenyl diphenylphosphinate (42.6 mg, 111.0 μmol) were sequentially added. The reaction mixture was stirred at 20°C for 3 hours. The reaction mixture was added with 10 mL of 2 M sodium carbonate solution and extracted with dichloromethane (20 mL * 3). The organic phase was washed with saturated brine, dried over anhydrous sodium sulfate, filtered, and the filtrate was concentrated under reduced pressure to obtain a crude product. The crude product was purified by thin-layer chromatography (mobile phase DCM: MeOH = 10:1) to obtain **001-7.** $^1$H NMR (400 MHz, $CDCl_3$): $\delta$ ppm 9.29 - 9.24 (m, 1H), 8.26 - 8.24 (m, 1H), 8.14 - 8.11 (m, 1H), 7.03 - 6.95 (m, 2H ),6.90 - 6.84 (m, 1H ),6.21 -5.93 (m, 1H), 5.92 - 5.87 (m, 1H), 4.79 - 4.67 (m, 1H), 4.40 - 4.26 (m, 2H), 4.26 - 4.20 (m, 1H), 4.03 - 3.96 (m, 1H), 3.92 - 3.87 (m, 1H), 1.81 - 1.76 (m, 3H), 1.62 (s, 3H); LCMS: m/z = 448.1 $[M+H]^+$.

Step 8: Synthesis of Compound **001-8**

**001-7** (0.5 g, 1.12 mmol) was dissolved in 1,2-dimethylbenzene (20 mL), and then Lawesson's reagent (1.81 g, 4.47 mmol) was added. The reaction mixture was stirred at 140°C for 5 hours. The reaction mixture was added with 50 mL of water and extracted with ethyl acetate (60 mL × 3). The resulting organic phase was washed with saturated brine, dried over anhydrous sodium sulfate, filtered, the filtrate was concentrated under reduced pressure to obtain a crude product. The crude product was then purified by column chromatography (DCM: MeOH = 20:1) to obtain **001-8.** LCMS: m/z = 464.1 $[M+H]^+$.

Step 9: Synthesis of Compound **001**

**001-8** (0.05 g, 107.9 μmol) was dissolved in DMF (5 mL), and then methoxyamine hydrochloride (144.2 mg, 1.73 mmol), mercuric oxide (187.0 mg, 863.05 μmol), and triethylamine (1.51 mmol, 210.22 μL) were sequentially added. The reaction mixture was stirred at 60°C for 3 hours. The reaction mixture was diluted with 10 mL of dichloromethane and filtered. The resulting filtrate was washed with 10% sodium bicarbonate, and the aqueous phase was extracted with dichloromethane (10 mL * 2). The organic phases were combined, then washed with saturated brine, dried over anhydrous sodium sulfate, filtered, the filtrate was concentrated under reduced pressure to obtain a crude product. The crude product was purified by thin-layer chromatography (DCM: MeOH = 20:1) to obtain **001.** $^1$H NMR (400 MHz, $CDCl_3$): $\delta$ ppm 8.52 (s, 1H), 8.08 (s, 1H), 7.08 (s, 1H), 7.02 - 6.99 (m, 1H), 6.98 - 6.94 (m, 1H), 6.89 - 6.80 (m, 1H), 6.18 - 6.00 (m, 1H), 5.99 - 5.96 (d, J=12 Hz, 1H), 4.69 - 4.66 (d, J=12 Hz, 1H), 4.37 - 4.28 (m, 1H), 4.25 - 4.19 (m, 1H), 4.17 - 4.13 (d, J=16 Hz ,1H), 3.99-3.98 (m, 2H), 3.82 (s, 3H), 1.66 (s, 6H); LCMS: m/z = 477.1 $[M+H]^+$.

**Example 2**

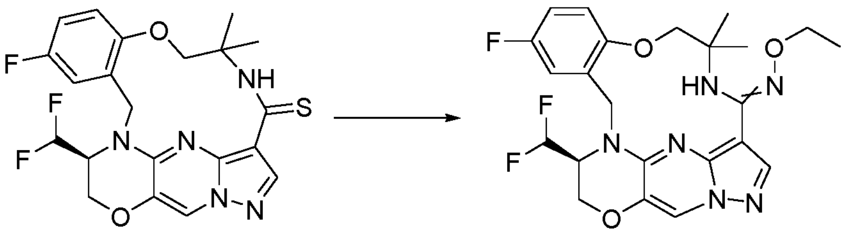

001-8 002

**001-8** (50 mg, 107.88 μmol) was dissolved in DMF (8 mL), and then ethoxyamine hydrochloride (168.37 mg, 1.73 mmol), mercuric oxide (220 mg, 1.02 mmol), and triethylamine (210.22 μL, 1.51 mmol) were sequentially added. The reaction mixture was heated to 60°C and stirred for 3 hours. The reaction mixture was diluted with 40 mL of DCM and filtered. The resulting filtrate was washed with 10% sodium bicarbonate (40 mL) and extracted with DCM (100 mL * 2). The organic phase was washed with saturated brine, dried over anhydrous sodium sulfate, filtered, the filtrate was concentrated under reduced pressure to obtain the crude product. The crude product was purified by thin-layer chromatography (DCM: MeOH = 20:1) to obtain **002.** $^{1}$H NMR (400 MHz, $CDCl_3$): $\delta$ ppm 8.57 (s, 1 H), 8.08 (s, 1 H), 7.09 (s, 1 H), 6.92 - 7.03 (m, 2 H), 6.85 (dd, *J*=8.76, 4.50 Hz, 1 H), 5.86 - 6.18 (m, 2 H), 4.67 (br d, *J*=11.63 Hz, 1 H), 3.68 - 4.44 (m, 6 H), 1.65 (s, 7 H), 1.28 - 1.35 (m, 3 H); LCMS: m/z = 491.0 $[M+H]^+$.

**Example 3**

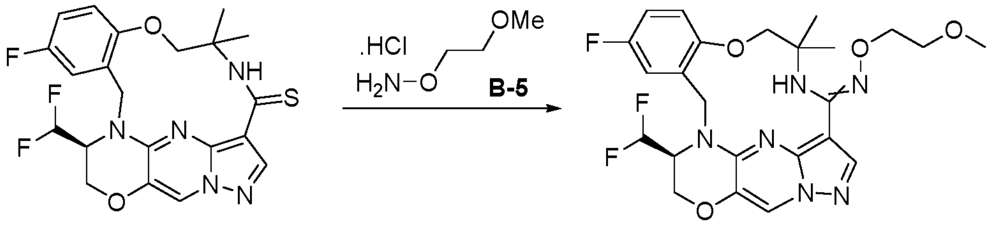


001-8 003

**001-8** (50 mg, 107.88 μmol) and **B-5** (137.62 mg, 1.08 mmol) were dissolved in DMF (5 mL), and then mercuric oxide (0.4 g, 1.85 mmol) and triethylamine (1.51 mmol, 210.22 μL) were sequentially added. The reaction mixture was heated to 60°C under a nitrogen atmosphere and stirred for 3 hours. The reaction mixture was diluted with 40 mL of dichloromethane and filtered. The resulting filtrate was washed with 10% sodium bicarbonate (40 mL) and extracted with dichloromethane (100 mL * 2). The organic phase was dried over anhydrous sodium sulfate, filtered, the filtrate was concentrated under reduced pressure to obtain a crude product. The crude product was purified by thin-layer chromatography (DCM: MeOH = 20:1) to obtain **003.** 1H NMR (400 MHz, $CDCl_3$): $\delta$ ppm 8.61 (s, 1 H), 8.08 (s, 1 H), 7.15 (s, 1 H), 6.92 - 7.05 (m, 2 H), 6.85 (dd, J=8.78, 4.52 Hz, 1 H), 5.84 - 6.19 (m, 2 H), 4.67 (d, *J*=11.80 Hz, 1 H), 4.28 - 4.39 (m, 1 H), 4.09 - 4.25 (m, 4 H), 3.86 - 4.00 (m, 2 H), 3.66 - 3.80 (m, 2 H), 3.41 (s, 3 H), 1.64 (s, 6 H); LCMS: m/z = 521.1 $[M+H]^+$.

**Example 4**

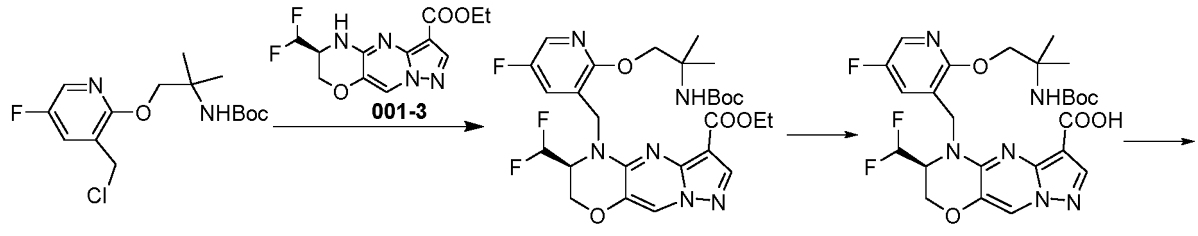


B-6 004-1 004-2

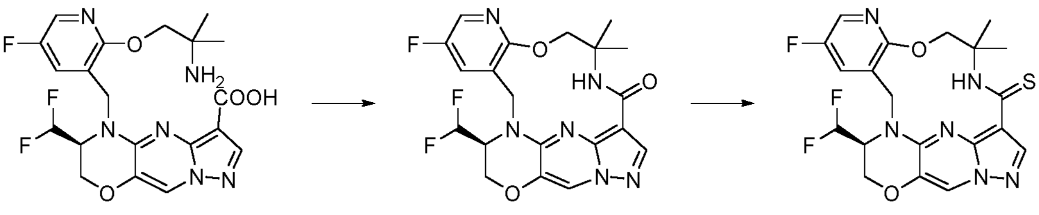

004-3 004-4 004

Step 1: Synthesis of Compound **004-1**

**B-6** (569.08 mg, 1.71 mmol) and **001-3** (510 mg, 1.71 mmol) were dissolved in DMF (25 mL), and then cesium carbonate (2.23 g, 6.84 mmol) was added. The reaction mixture was stirred at 5°C for 4 hours. The reaction mixture was added with water (20 mL) and extracted with DCM (100 mL * 2). The resulting organic phase was dried over anhydrous sodium sulfate, filtered, and the filtrate was concentrated under reduced pressure to obtain a crude product. The crude product was purified by thin-layer chromatography (PE: EA= 2:1) to obtain **004-1.** $^1$H NMR (400 MHz, $CDCl_3$): $\delta$ ppm 8.37 (br d, *J*=6.00 Hz, 1 H), 8.28 (d, *J*=2.25 Hz, 1 H), 8.09 (d, *J*=1.50 Hz, 1 H), 7.90 (dd, *J*=2.94, 1.56 Hz, 1 H), 5.78 - 6.25 (m, 1 H), 5.51 (d, *J*=14.51 Hz, 1 H), 4.47 - 4.60 (m, 4 H), 4.38 - 4.46 (m, 2 H), 4.12 (qd, *J*=7.13, 1.75 Hz, 1 H), 3.94 (br d, *J*=11.76 Hz, 1 H), 1.40 (s, 15 H), 1.26 (td, *J*=7.13, 1.63 Hz, 3 H); LCMS: m/z=495.1 $[M+H-100]^+$.

Step 2: Synthesis of Compound **004-2**

**004-1** (0.8 g, 1.345 mmol) was dissolved in tetrahydrofuran (30 mL), ethanol (60 mL), and methanol (30 mL). Then lithium hydroxide (2 M, 67.3 mL) was added and stirred at 30°C for 20 hours. The reaction mixture was cooled to -20°C, added with 70.0 mL of 2 M hydrochloric acid solution, and then diluted by the addition of 200 mL of water. The reaction mixture was extracted with dichloromethane (200 mL * 3). The organic phase was washed with saturated brine, dried over anhydrous sodium sulfate, and the filtrate was concentrated under reduced pressure. The resulting crude product of **004-2** was directly used for the next step without purification. LCMS: m/z=489.2 $[M+Na-100]^+$.

Step 3: Synthesis of Compound **004-3**

**004-2** (0.8 g, 1.41 mmol) was dissolved in DCM (10 mL), and then a solution of hydrochloric acid in 1,4-dioxane (4 M, 10 mL) was added. The reaction mixture was stirred at 25°C for 1 hour. The resulting reaction mixture was concentrated under reduced pressure, and the residue was dissolved in EA (10 mL) and concentrated, and then dissolved in EA (10 mL) and concentrated to obtain a crude product of **004-3,** which was directly used for the next reaction. LCMS: m/z = 467.1 $[M+H]^+$.

Step 4: Synthesis of Compound **004-4**

**004-3** was dissolved in DCM (50 mL), and then N,N-diisopropylethylamine (11.79 mmol, 2.05 mL) and pentafluorophenyl diphenylphosphinate (679.65 mg, 1.77 mmol) were added. The reaction mixture was stirred at 25°C for 2 hours. The reaction mixture was added with water (20 mL) and extracted with DCM (200 mL * 2). The resulting organic phase was dried over anhydrous sodium sulfate, filtered, and the filtrate was concentrated under reduced pressure to obtain a crude product. The crude product was purified by thin-layer chromatography (DCM: MeOH = 10:1) to obtain **004-4.** $^1$H NMR (400 MHz, $CDCl_3$): $\delta$ ppm 9.09 (s, 1 H), 8.22 - 8.36 (m, 1 H), 8.16 (s, 1 H), 8.00 (d, *J*=2.88 Hz, 1 H), 7.35 (dd, *J*=7.94, 2.69 Hz, 1 H), 5.83 - 6.24 (m, 1 H), 5.72 (dd, *J*=15.38, 1.50 Hz, 1 H), 4.83 (d, *J*=10.76 Hz, 1 H), 4.74 (d, *J*=12.38 Hz, 1 H), 4.21 - 4.42 (m, 3 H), 3.90 (d, *J*=10.63 Hz, 1 H), 1.77 (s, 3 H), 1.63 (s, 3 H); LCMS: m/z = 449.0 $[M+H]^+$.

Step 5: Synthesis of Compound **004**

**004-4** (0.09 g, 200.71 μmol) and Lawesson's reagent (324.73 mg, 802.86 μmol) were dissolved in 1,2-xylene (8 mL), and the reaction mixture was heated to 140°C under a nitrogen atmosphere and reacted for 5 hours. After the reaction mixture was cooled to room temperature, the reaction mixture was added with 50 mL of water and extracted with EA (60 mL * 3). The organic phases were combined, then washed with saturated brine, dried over anhydrous sodium sulfate, filtered, the filtrate was concentrated under reduced pressure to obtain a crude product. The crude product was purified by thin-layer chromatography (DCM: MeOH = 1:0 to 20:1) to obtain **004.** $^{1}$H NMR (400 MHz, $CDCl_3$): $\delta$ ppm 10.37 (brs, 1 H), 8.54 (s, 1 H), 8.13 (s, 1 H), 8.02 (d, *J*=3.01 Hz, 1 H), 7.41 (dd, *J*=7.91, 3.14 Hz, 1 H), 6.03 - 6.19 (m, 1 H), 5.69 - 5.79 (m, 1 H), 4.66 - 4.81 (m, 2 H), 4.17 - 4.35 (m, 3 H), 3.96 (d, *J*=11.04 Hz, 1 H), 2.03 (s, 3 H), 1.82 (s, 3 H); LCMS: m/z = 465.1 [M+H]$^{+}$.

**Example 5**

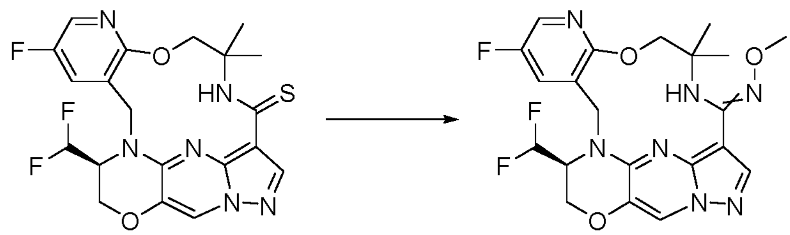

**004** **005**

**004** (90 mg, 193.77 μmol), methoxyamine hydrochloride (258.93 mg, 3.10 mmol), mercuric oxide (0.36 g, 1.66 mmol), and triethylamine (2.71 mmol, 377.59 μL) were dissolved in DMF (8 mL), and the reaction mixture was heated to 60°C and stirred for 3 hours. The reaction mixture was filtered, and the filtrate was purified by thin-layer chromatography (DCM: MeOH = 20:1) to obtain 005. Post-treatment of the filter cake: The filter cake was soaked in hydrochloric acid (1 M, 50 mL) overnight, then the pH was adjusted to 10 using sodium hydroxide (2 M). Sodium sulfide solid (1 g) was added to generate a precipitate, followed by the addition of ferrous sulfate (1 g) as a co-precipitant. After standing, the mixture was filtered, and the filter cake was discarded. The pH of the filtrate was adjusted to about 7 with hydrochloric acid (1 M) before the filtrate was discarded. $^{1}$H NMR (400 MHz, $CDCl_3$): $\delta$ ppm 8.54 (s, 1H), 8.11 (s, 1H), 7.97 (d, *J*=2.89 Hz, 1 H), 7.34 (dd, *J*=7.91, 2.89 Hz, 1 H), 5.86 - 6.19 (m, 1 H), 5.80 (dd, *J*=15.31, 1.25 Hz, 1 H), 4.81 (d, *J*=10.29 Hz, 1 H), 4.68 (m, 1 H), 4.16 - 4.32 (m, 3 H), 3.87 (m, 1 H), 3.83 (s, 3 H), 1.67 (s, 6 H); LCMS: m/z = 478.1 [M+H]$^{+}$.

**Example 6**

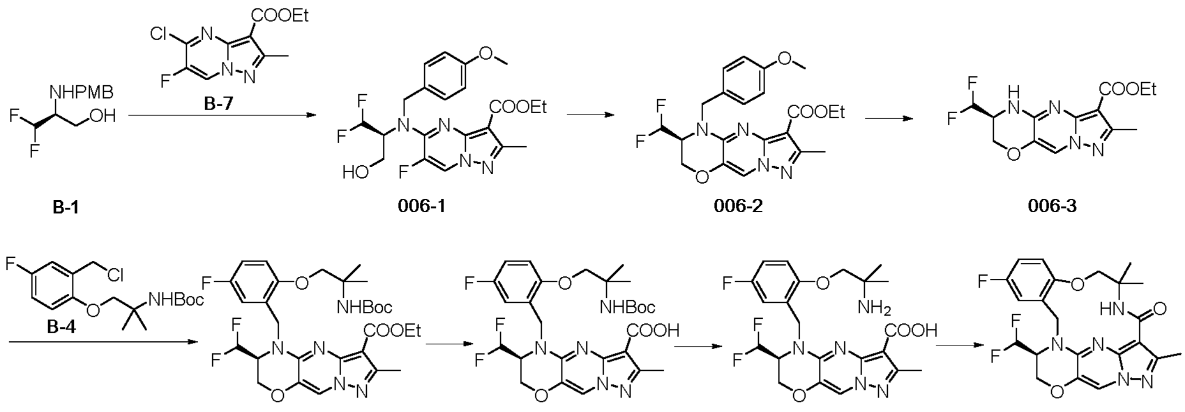

B-7 B-1 006-1 006-2 006-3 B-4 006-4 006-5 006-6 006

Step 1: Synthesis of Compound **006-1**

**B-7** (0.5 g, 1.94 mmol) and **B-1** (448.75 mg, 1.94 mmol) were dissolved in DMSO (10 mL), and then KF (281.88

mg, 4.85 mmol) was added. The reaction mixture was stirred at 120°C under a nitrogen atmosphere for 5 hours. The reaction mixture was poured into 100 mL of ice water to quench the reaction, and a solid precipitated. The solid obtained from filtration was dissolved in dichloromethane (50 mL), dried over anhydrous sodium sulfate, filtered, and the filtrate was concentrated under reduced pressure to obtain a crude product. The crude product was purified by column chromatography (PE: EA = 1:0 to 1:1) to obtain **006-1.** $^1$H NMR (400 MHz, $CDCl_3$) $\delta$ ppm 8.33 (d, *J* = 7.8 Hz, 1H), 7.25 (s, 2H), 6.95 - 6.89 (m, 2H), 6.87 - 6.67 (m, 1H), 4.96 (d, *J* = 16.5 Hz, 1H), 4.70 (dd, *J* = 1.9, 16.5 Hz, 1H), 4.38 (qtd, *J* = 7.1, 10.8, 17.8 Hz, 3H), 4.16 - 4.10 (m, 1H), 4.06 - 3.99 (m, 1H), 3.88 - 3.75 (m, 4H), 2.61 (s, 3H), 1.39 (t, *J* = 7.1 Hz, 3H); LCMS: m/z = 453.1 $[M+H]^+$.

Step 2: Synthesis of Compound **006-2**

**006-1** (700 mg, 1.55 mmol) was dissolved in DMF (49 mL), and after purging with nitrogen, cesium carbonate (1.51 g, 4.64 mmol) was added. The reaction mixture was stirred at 50°C for 12 hours. After cooling to room temperature, the reaction mixture was quenched by adding 10 mL of water and extracted with EA (20 mL * 4). The resulting organic phase was sequentially washed with saturated brine (20 mL * 3), dried over anhydrous sodium sulfate, filtered, and the filtrate was concentrated under reduced pressure to obtain a crude product. The crude product was purified by thin-layer chromatography (PE: EA = 1:1) to obtain **006-2.** $^1$H NMR (400 MHz, $CDCl_3$) $\delta$ ppm 8.04 (s, 1H), 7.36 (d, *J* = 8.6 Hz, 2H), 6.88 (d, *J* = 8.6 Hz, 2H), 6.09 (d, *J* = 14.8 Hz, 1H), 5.97 - 5.75 (m, 1H), 4.48 (m, 1H), 4.43 - 4.29 (m, 2H), 4.28 - 4.17 (m, 1H), 3.84 - 3.69 (m, 5H), 2.62 (s, 3H), 1.38 (t, *J* = 7.1 Hz, 3H); LCMS: m/z = 433.1 $[M+H]^+$.

Step 3: Synthesis of Compound **006-3**

**006-2** (440 mg, 1.02 mmol) was dissolved in trifluoroacetic acid (1 mL), and the reaction mixture was heated to 70°C under a nitrogen atmosphere and stirred for 12 hours. After cooling to room temperature, the reaction mixture was quenched by adding 50 mL of water and extracted with EA (20 mL * 4). The resulting organic phase was sequentially washed with saturated sodium bicarbonate (20 mL * 3) and saturated brine (20 mL * 3), dried over anhydrous sodium sulfate, filtered, and the filtrate was concentrated under reduced pressure to obtain a crude product of **006-3,** which was directly used for the next reaction. LCMS: m/z = 313.1 $[M+H]^+$.

Step 4: Synthesis of Compound **006-4**

**006-3** (320 mg, 1.02 mmol), **B-4** (340.02 mg, 1.02 mmol), and cesium carbonate (1.00 g, 3.07 mmol) were dissolved in DMF (1 mL), and the reaction mixture was reacted at 25°C under a nitrogen atmosphere for 3 hours. The reaction was quenched by adding 10 mL of water and extracted with EA (20 mL * 4). The EA phase was sequentially washed with saturated brine (20 mL * 3), dried over anhydrous sodium sulfate, filtered, and the filtrate was concentrated under reduced pressure to obtain a crude product. The crude product was purified by column chromatography (PE: EA = 1:0 to 1:1) to obtain **006-4.** $^1$H NMR (400 MHz, $CDCl_3$) $\delta$ ppm 8.01 (s, 1H), 7.63 (br d, *J* = 7.1 Hz, 1H), 6.99 - 6.92 (m, 1H), 6.86 (dd, *J* = 4.3, 8.9 Hz, 1H), 6.12 - 5.77 (m, 2H), 4.58 - 4.46 (m, 2H), 4.45 - 4.35 (m, 3H), 4.28 - 4.22 (m, 1H), 4.20 (d, *J* = 8.8 Hz, 1H), 3.95 (br d, *J* = 8.8 Hz, 1H), 3.84 (br d, *J* = 11.4 Hz, 1H), 2.62 (s, 3H), 1.42 - 1.35 (m, 18H); LCMS: m/z = 608.5 $[M+H]^+$.

Step 5: Synthesis of Compound **006-5**

**006-4** (170 mg, 279.78 $\mu$mol) was dissolved in THF (2 mL), EtOH (4 mL), and MeOH (2 mL), followed by the addition of lithium hydroxide monohydrate (2 M, 4 mL) and sodium hydroxide (22.38 mg, 559.56 $\mu$mol). The reaction mixture was reacted at 25°C under a nitrogen atmosphere for 144 hours. The reaction mixture was cooled to -20°C, and the pH was adjusted to about 3 with 2 M hydrochloric acid. The mixture was extracted with DCM (20 mL * 4), and the resulting organic phase was sequentially washed with saturated brine (20 mL), dried over anhydrous sodium sulfate, filtered, and the filtrate was concentrated under reduced pressure to obtain a crude product. The crude product was purified by thin-layer chromatography (PE: EA= 1:1) to obtain **006-5.** LCMS: m/z = 580.5 $[M+H]^+$.

Step 6: Synthesis of Compound **006-6**

**006-5** (100 mg, 172.54 $\mu$mol) was dissolved in trifluoroacetic acid (0.5 mL), and the reaction mixture was reacted at 25°C under a nitrogen atmosphere for 0.5 hours. The reaction mixture was concentrated under reduced pressure to obtain the crude product of **006-6.** LCMS: m/z = 480.4 $[M+H]^+$.

Step 7: Synthesis of Compound **006**

**006-6** (83 mg, 173.11 μmol) and pentafluorophenyl diphenylphosphinate (99.78 mg, 259.67 μmol) were dissolved in DCM (2 mL), and N,N-diisopropylethylamine (1.73 mmol, 301.53 μL) was added under a nitrogen atmosphere. The reaction mixture was reacted at 25°C for 12 hours. The reaction was quenched by adding 10 mL of water and extracted with DCM (20 mL * 3). The DCM phase was sequentially washed with saturated sodium carbonate (20 mL * 3) and saturated brine (20 mL * 3), dried over anhydrous sodium sulfate, filtered, and the filtrate was concentrated under reduced pressure to obtain a crude product. The crude product was purified by thin-layer chromatography (DCM: MeOH = 20:1), and then purified by prep-HPLC (chromatographic column: Waters Xbridge BEH C18 100 * 30 mm, 10 μm; mobile phase: phase A: water (10 mM ammonium bicarbonate), phase B: acetonitrile; gradient: phase B increased from 10% to 70% in the first 4.0 minutes, maintained for 1.0 minute, then decreased to 10%, maintained for 3.0 minutes) to obtain 006. 1H NMR (400 MHz, $CDCl_3$) δ ppm 9.25 (s, 1H), 8.05 (s, 1H), 7.02 - 6.95 (m, 2H), 6.89 - 6.82 (m, 1H), 6.18 - 5.87 (m, 2H), 4.70 (m, 1H), 4.39 - 4.31 (m, 1H), 4.30 - 4.25 (m, 1H), 4.21 (d, *J* = 15.3 Hz, 1H), 3.99 - 3.89 (m, 2H), 2.64 (s, 3H), 1.80 (s, 3H), 1.63 (s, 3H); LCMS: m/z = 462.2 $[M+H]^+$.

**Example 7**

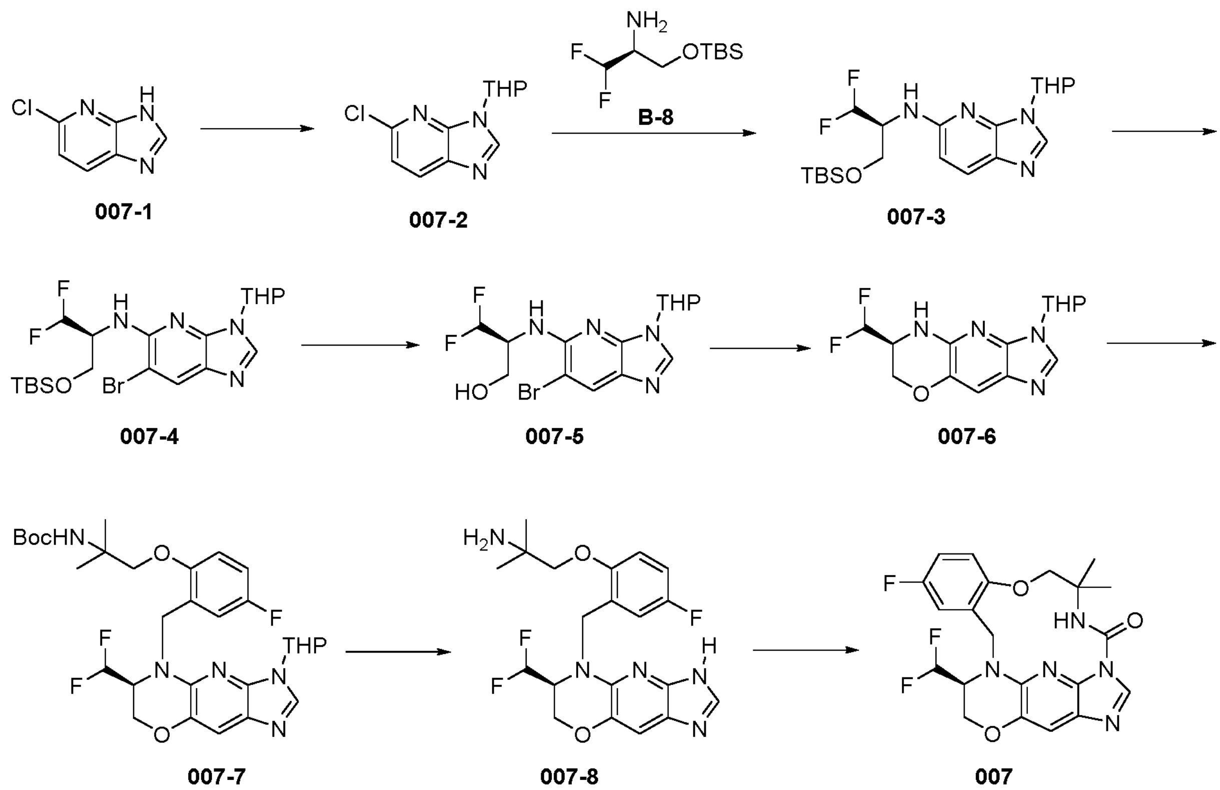

B-8

007-1 007-2 007-3

007-4 007-5 007-6

007-7 007-8 007

Step 1: Synthesis of Compound **007-2**

**007-1** (3 g, 19.54 mmol), 2-chloro-tetrahydro-2*H*-pyran (7.14 mL, 78.14 mmol), and pyridinium *p*-toluenesulfonate (294.55 mg, 1.17 mmol) were dissolved in 30 mL toluene. After purging with nitrogen, the reaction mixture was heated to 100°C and reacted for 12 hours. The reaction mixture was concentrated under reduced pressure to remove the solvent, then 10 mL of saturated sodium bicarbonate solution was added. The mixture was extracted with 15 mL of dichloromethane, and the organic phase was collected after phase separation. The aqueous phase was extracted with dichloromethane (3 * 10 mL). The organic phases were combined, sequentially washed with saturated brine (2 * 20 mL), dried over anhydrous sodium sulfate, filtered, and the filtrate was concentrated under reduced pressure to obtain a crude product. The crude product was purified by column chromatography (mobile phase PE: EA = 1:0 to 0:1) to obtain **007-2.** $^1H$ NMR (400 MHz, $CDCl_3$) δ ppm 8.28 (s, 1H), 8.02 (d, J = 8.4 Hz, 1H), 7.28 (s, 1H), 5.85 (dd, J = 2.2, 10.6 Hz, 1H), 4.18 (td, J = 2.0, 11.7 Hz, 1H), 3.90 - 3.74 (m, 1H), 2.21 - 1.96 (m, 2H), 1.91 - 1.60 (m, 4H); LCMS: m/z = 238.1 $[M+H]^+$.

Step 2: Synthesis of Compound **007-3**

**007-2** (600 mg, 2.52 mmol) and **B-8** (682.64 mg, 3.03 mmol) were dissolved in 10 mL of 1,4-dioxane. After purging with nitrogen, sodium tert-butoxide (485.18 mg, 5.05 mmol), palladium acetate (28.34 mg, 126.22 μmol), and BRETTPHOS (135.50 mg, 252.43 μmol) were sequentially added. The reaction mixture was heated to 80°C and reacted for 12 hours. The reaction mixture was cooled to room temperature, then quenched by adding 30 mL of water, and extracted with 20 mL of dichloromethane. The organic phase was collected after phase separation, and the aqueous phase was extracted with dichloromethane (20 mL * 3). The organic phases were combined, sequentially washed with saturated brine (20 mL * 3), dried over anhydrous sodium sulfate, and filtered. The filtrate was concentrated under reduced pressure to obtain a crude product. The crude product was purified by column chromatography (mobile phase PE: EA = 1:0 to 1:1) to obtain **007-3.** $^1$H NMR (400 MHz, $CDCl_3$) $\delta$ ppm 8.03 (d, J = 2.3 Hz, 1H), 7.82 (dd, J = 1.2, 8.6 Hz, 1H), 6.46 (dd, J = 2.3, 8.6 Hz, 1H), 6.27 - 5.90 (m, 1H), 5.77 - 5.62 (m, 1H), 4.89 (br d, J = 8.2 Hz, 1H), 4.66 - 4.43 (m, 1H), 4.22 - 4.14 (m, 1H), 4.00 (td, J = 1.8, 10.1 Hz, 1H), 3.86 - 3.73 (m, 2H), 2.11 - 2.07 (m, 2H), 1.86 - 1.75 (m, 4H), 0.93 (s, 9H), 0.11 (d, J = 7.2 Hz, 6H); LCMS: m/z =427.2 $[M+H]^+$.

Step 3: Synthesis of Compound **007-4**

**007-3** (490 mg, 1.15 mmol) and sodium acetate (612.50 mg, 7.47 mmol) were dissolved in acetic acid (12.5 mL), and the mixture was cooled to 0°C under a nitrogen atmosphere. Then, pyridinium tribromide (551.06 mg, 1.72 mmol) was added, and the reaction mixture was reacted at 25°C for 2 hours. The reaction mixture was quenched with 10 mL of saturated sodium thiosulfate (no blue color developed with potassium iodide test paper), stirred with 10 mL of water for 10 minutes, and extracted with 20 mL of dichloromethane. The organic phase was collected after phase separation, and the aqueous phase was extracted with dichloromethane (20 mL * 3). The organic phases were combined, sequentially washed with 20 mL saturated brine, dried over anhydrous sodium sulfate, filtered, and the filtrate was concentrated under reduced pressure to obtain a crude product of **007-4,** which was directly used for the next step without purification. LCMS: m/z =505.1 $[M+H]^+$.

Step 4: Synthesis of Compound **007-5**

**007-4** (640 mg, 1.27 mmol) and TBAF (1 M, 1.45 mL) were dissolved in 10 mL of THF and reacted at 25°C for 1 hour under a nitrogen atmosphere. The reaction mixture was concentrated under reduced pressure to obtain a crude product. The crude product was purified by column chromatography (mobile phase PE: EA = 1:0 to 0:1) to obtain **007-5.** $^1$H NMR (400 MHz, $CDCl_3$) $\delta$ ppm 8.10 (s, 1H), 8.00 (d, *J* = 4.2 Hz, 1H), 6.31 - 5.96 (m, 1H), 5.69 (br d, *J* = 7.3 Hz, 1H), 5.64 - 5.54 (m, 1H), 4.69 - 4.51 (m, 1H), 4.19 - 4.07 (m, 2H), 4.02 - 3.93 (m, 1H), 3.82 - 3.70 (m, 1H), 3.39 (t, *J* = 7.1 Hz, 1H), 2.38 (t, *J* = 8.2 Hz, 1H), 2.09 (br dd, *J* = 2.8, 6.4 Hz, 3H), 2.06 - 1.98 (m, 2H).

Step 5: Synthesis of Compound **007-6**

**007-5** (100 mg, 255.62 μmol) and cesium carbonate (108.27 mg, 332.30 μmol) were dissolved in 2.5 mL of 1,4-dioxane. Under a nitrogen atmosphere, 2-(di-*tert*-butylphosphino)-1,1'-binaphthyl (20.37 mg, 51.12 μmol) and tris(dibenzylideneacetone)dipalladium (23.41 mg, 25.56 μmol) were sequentially added. The reaction mixture was slowly heated to 88°C and reacted for 15 hours. Subsequently, additional 2-(di-*tert*-butylphosphino)-1,1'-binaphthyl (20.37 mg, 51.12 μmol) and tris(dibenzylideneacetone)dipalladium (23.41 mg, 25.56 μmol) were added, and the reaction mixture was reacted at 116°C for 16 hours. The reaction mixture was purified by preparative thin-layer chromatography (EA: PE = 2:1) to obtain **007-6.** $^1$H NMR (400 MHz, $CDCl_3$) $\delta$ ppm 7.97 (s, 1H), 7.52 - 7.29 (m, 1H), 6.04 - 5.67 (m, 1H), 5.62 (br dd, J = 2.6, 10.3 Hz, 1H), 5.20 (br s, 1H), 4.39 (br d, J = 11.1 Hz, 1H), 4.22 - 4.06 (m, 2H), 3.89 - 3.68 (m, 2H), 2.19 - 2.07 (m, 2H), 1.90 - 1.62 (m, 4H); LCMS: m/z =311.1 $[M+H]^+$.

Step 6: Synthesis of Compound **007-7**

**007-6** (40 mg, 128.91 μmol), **B-4** (85.55 mg, 257.82 μmol), cesium carbonate (126.00 mg, 386.72 μmol), and tetrabutylammonium iodide (12.38 mg, 33.52 μmol) were dissolved in 0.8 mL of DMF. The reaction mixture was heated and reacted under a nitrogen atmosphere at 40°C for 16 hours. The reaction mixture was quenched with 5 mL of water, extracted with dichloromethane (1 mL *3), and the phases were separated to obtain crude organic phase. The crude product was purified by preparative thin-layer chromatography (EA: PE = 10:1) to obtain 007-7. $^1$H NMR (400 MHz, $CDCl_3$) $\delta$ ppm 7.96 (s, 1H), 7.45 (s, 1H), 7.26 - 7.18 (m, 1H), 6.97 - 6.74 (m, 2H), 6.09 - 5.74 (m, 1H), 5.73 - 5.60 (m, 2H), 4.56 - 4.38 (m, 2H), 4.24 - 4.08 (m, 2H), 4.04 - 3.89 (m, 2H), 3.87 - 3.68 (m, 2H), 2.10 (br s, 2H), 1.82 - 1.58 (m, 4H), 1.45 - 1.39 (m, 9H), 1.36 - 1.29 (m, 6H); LCMS: m/z =606.3 $[M+H]^+$.

Step 7: Synthesis of Compound **007-8**

**007-7** (30 mg, 49.53 μmol) was dissolved in 1.5 mL of dichloromethane, and then trifluoroacetic acid (0.5 mL, 6.75 mmol) was added. The reaction was carried out at 25°C for 2 hours. The reaction mixture was concentrated under reduced pressure to remove the solvent and trifluoroacetic acid to obtain the crude trifluoroacetate salt of **007-8,** which was directly used for the next step without further purification. $^1$H NMR (400 MHz, DMSO-d6) δ ppm 7.95 (s, 1H), 7.38 (s, 1H), 7.04 - 6.92 (m, 2H), 6.89 (dd, J = 2.7, 9.3 Hz, 1H), 6.48 - 6.08 (m, 1H), 5.25 (br d, J = 16.9 Hz, 1H), 4.66 (br d, J = 16.9 Hz, 1H), 4.49 (br d, J = 11.5 Hz, 1H), 4.11 - 3.99 (m, 2H), 3.75 - 3.66 (m, 2H), 1.15 - 1.10 (m, 6H); LCMS: m/z =422.2 [M+H]$^+$.

Step 8: Synthesis of Compound **007**

**007-8** (35.36 mg, crude trifluoroacetate salt) was dissolved in 2.1 mL of dichloromethane. At 0°C, a solution of *N*, *N*'-carbonyldiimidazole (25.60 mg, 157.86 μmol) in dichloromethane (0.7 mL) was added dropwise. After thorough stirring, *N,N'*-diisopropylethylamine (0.2 mL, 1.15 mmol) was added, and the reaction mixture was slowly warmed to 25°C and reacted for 12 hours. 5 mL of 10% citric acid aqueous solution was slowly poured into the reaction mixture, and the pH was adjusted to 6. The reaction mixture was extracted with dichloromethane (1 mL * 2), and the phases were separated to obtain the organic phase, which was concentrated under reduced pressure and then purified by preparative thin-layer chromatography (PE: EA = 1:1). The crude product was purified by preparative HPLC (chromatographic column: Waters Xbridge BEH C18 100 * 30 mm * 10 μm; mobile phase: phase A water (10 mM ammonium bicarbonate), phase B acetonitrile; gradient: phase B increased from 10% to 80% in the first 4 minutes, maintained for 2.0 minutes, and then gradually decreased to 10% in the next 2 minutes) to obtain 007. $^1$H NMR (400 MHz, $CDCl_3$) δ ppm10.30 (br s, 1H), 8.38 (s, 1H), 7.48 (s, 1H), 7.07 - 6.92 (m, 2H), 6.83 (dd, J = 4.4, 9.1 Hz, 1H), 6.10 - 5.80 (m, 1H), 5.76 (br d, J = 15.5 Hz, 1H), 4.68 (br d, J = 11.6 Hz, 1H), 4.35 - 4.16 (m, 3H), 4.07 (d, J = 8.9 Hz, 1H), 3.93 (d, J = 8.9 Hz, 1H), 1.77 (s, 3H), 1.67 (s, 3H); LCMS: m/z =448.1 [M+H]$^+$.

## Example 8

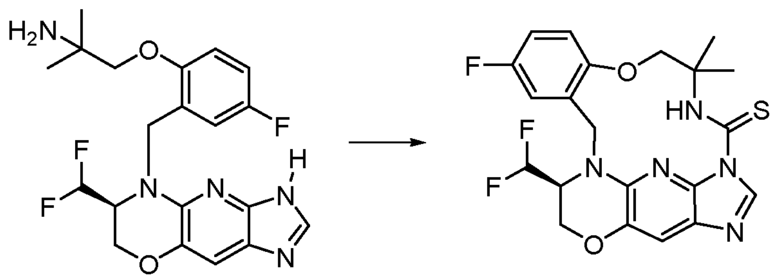

**007-8** **008**

**007-8** (150 mg, 0.33 mmol) was dissolved in dichloromethane (7.5 mL). After cooling to 0°C, *N*,*N*-thionylcarbonyldiimidazole (116.8 mg, 0.66 mmol) was added under a nitrogen atmosphere. The reaction mixture was then slowly warmed to 24°C and stirred for 12 hours. 10 mL of 10% citric acid aqueous solution was slowly poured into the reaction mixture, and the pH was adjusted to 6. The reaction mixture was extracted with dichloromethane (10 mL * 2), and the organic phase was concentrated under reduced pressure and then purified by preparative thin-layer chromatography (PE: EA = 0:1). The crude product was purified by preparative HPLC (chromatographic column: Waters Xbridge BEH C18 50 * 2.1 mm * 5 μm; mobile phase: phase A was 10 mM ammonium bicarbonate aqueous solution, phase B was acetonitrile; gradient: phase B increased from 10% to 80% in the first 4 minutes, maintained for 0.9 minutes, and then gradually decreased to 10% in the last 0.6 minutes) to obtain **008.** 1H NMR (400 MHz, $CDCl_3$) δ ppm 11.67 (s, 1H), 8.91 (s, 1H), 7.46 (s, 1H), 7.09 (dd, J = 2.8, 8.8 Hz, 1H), 6.99 (d, J = 2.8 Hz, 1H), 6.92 (dd, J = 4.4, 8.8 Hz, 1H), 5.96 - 5.79 (m, 2H), 4.67 - 4.64 (m, 1H), 4.24 - 4.20 (m, 1H), 4.18 - 4.17 (m, 1H), 4.05 - 4.02 (m, 1H), 3.95 - 3.92 (m, 2H), 1.98 (s, 3H), 1.84 (s, 3H); LCMS: m/z =464.3 [M+H]$^+$.

## Example 9

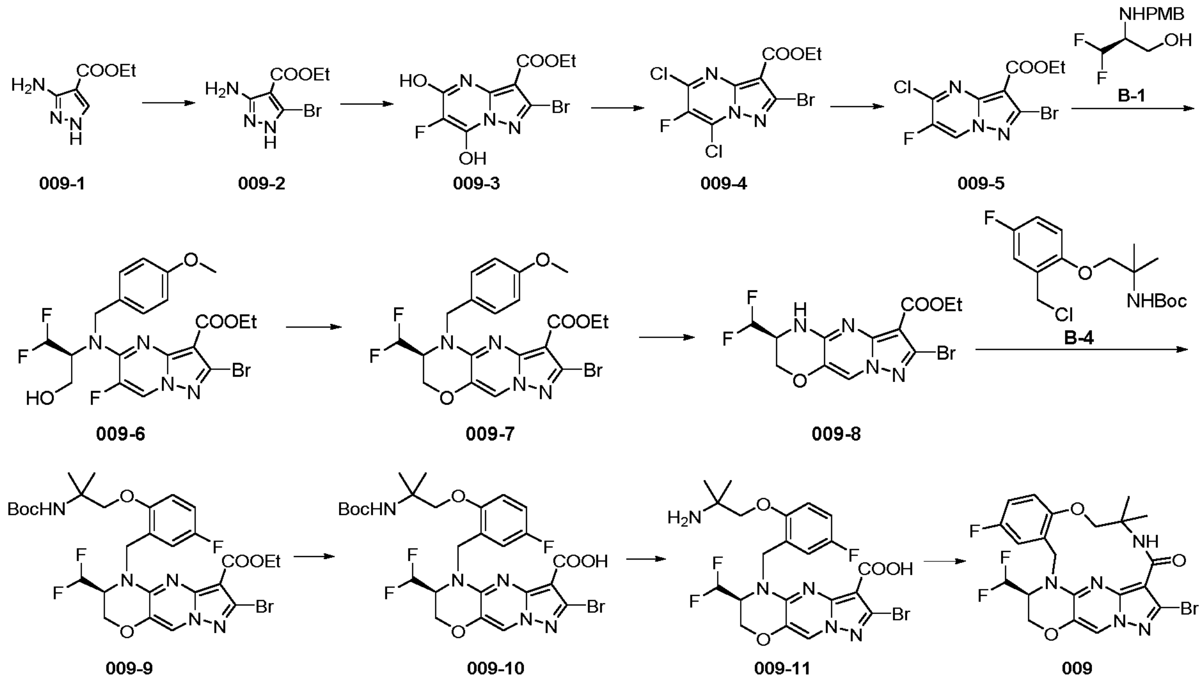

009-1 009-2 009-3 009-4 009-5 009-6 009-7 009-8 009-9 009-10 009-11 009

Step 1: Synthesis of Compound **009-2**

**009-1** (5.0 g, 32.23 mmol) was dissolved in ethyl acetate (50 mL), and then potassium carbonate (0.23 g, 1.61 mmol) was added, and the mixture was cooled to -5°C under a nitrogen atmosphere. N-bromosuccinimide (7.17 g, 40.28 mmol) was added, and then the reaction mixture was gradually warmed to 20°C and stirred for 2 hours. The reaction mixture was washed with 50 mL of saturated sodium sulfite solution and extracted with ethyl acetate (2 * 50 mL). The organic phases were combined, sequentially washed with saturated brine (2 * 50 mL), dried over anhydrous sodium sulfate, filtered, and the filtrate was concentrated under reduced pressure to obtain a crude product. The crude product was purified by column chromatography (mobile phase PE: EA = 1:0 to 1:1) to obtain **009-2.** $^1$H NMR (400 MHz, +DMSO-$d_6$) $\delta$ ppm 1.26 (t, *J*=7.03 Hz, 3 H), 4.18 (q, *J*=7.11 Hz, 2 H), 6.26 (s, 2 H), 12.15 (s, 1 H); LCMS: m/z =233.7, 235.7 $[M+H]^+$.

Step 2: Synthesis of Compound **009-3**

**009-2** (86.0 g, 367.4 mmol), diethyl fluoromalonate (98.2 g, 551.2 mmol), and sodium ethoxide (62.5 g, 918.6 mmol) were dissolved in ethanol (1000 mL), and the reaction mixture was heated to 78°C and stirred for 6 hours. After cooling to room temperature, the reaction mixture was concentrated under reduced pressure to remove ethanol, and 1 M hydrochloric acid solution was added dropwise to adjust the pH to about 3. The reaction mixture was stirred at 25°C for 1 hour, filtered, and the resulting filter cake was dried in a vacuum drying oven to abtain **009-3.** $^1$H NMR (400 MHz, DMSO-$d_6$) $\delta$ ppm 1.27 (t, *J*=7.03 Hz, 3 H), 4.25 (q, *J*=7.11 Hz, 2 H), 7.84 (s, 1 H), 9.63 (br s, 1 H); LCMS: m/z =319.7, 321.7 $[M+H]^+$.

Step 3: Synthesis of Compound **009-4**

**009-3** (43.0 g, 134.3 mmol) was dissolved in phosphorus oxychloride (454.8 mL, 4.89 mol), then *N,N*-diisopropylamine (32.2 mL, 201.5 mmol) was added dropwise at 0°C, and the reaction mixture was gradually heated to 100°C and stirred for 3 hours. After the reaction mixture was cooled to room temperature, most of phosphorus oxychloride was recovered by distillation under reduced pressure, and the resulting solution was slowly added into water (450 mL) to quench. A solid was precipitated, filtered, the filter cake was washed with water (3 * 50 mL), and the filtrate was neutralized and discarded. The resulting filter cake was redissolved in methyl *tert*-butyl ether (200 mL), stirred at 25°C for 0.5 hours, filtered, and the filter cake was collected to obtain **009-4.** LCMS: m/z =377.7, 379.7 $[M+Na]^+$.

Step 4: Synthesis of Compound **009-5**

**009-4** (1.9 g, 5.32 mmol) was dissolved in ethanol (45 mL), tetrahydrofuran (45 mL), and water (30 mL), then ammonium chloride (1.54 g, 28.77 mmol) was added, and zinc powder (1.39 g, 21.21 mmol) was added in batches at 0°C.

The reaction mixture was stirred at 0°C for 0.5 hours. The reaction mixture was filtered, and the filtrate was sequentially extracted with dichloromethane (2 * 30 mL), washed with saturated brine, and dried over anhydrous sodium sulfate. After filtration, the filtrate was concentrated under reduced pressure to obtain a crude product, which was purified by column chromatography (mobile phase PE: EA = 1:0 to 3:1) to obtain **009-5.** LCMS: m/z =321.9 $[M+H]^+$.

Step 5: Synthesis of Compound **009-6**

**009-5** (5.0 g, 15.5 mmol), **B-1** (3.58 g, 15.5 mmol), and potassium fluoride (3.15 g, 54.26 mmol) were dissolved in dimethyl sulfoxide (50 mL). The reaction mixture was heated to 120°C and stirred for 1 hour. The reaction mixture was washed with 50 mL of water and extracted with ethyl acetate (2 * 50 mL). The organic phases were combined, sequentially washed with saturated brine (2 * 50 mL), dried over anhydrous sodium sulfate, filtered, and the filtrate was concentrated under reduced pressure to obtain a crude product. The crude product was purified by column chromatography (mobile phase PE: EA= 1:0 to 3:1) to obtain **009-6.** LCMS: m/z =517.1 $[M+H]^+$.

Step 6: Synthesis of Compound **009-7**

**009-6** (1.4 g, 2.71 mmol) was dissolved in DMF (15 mL), and after purging with nitrogen, cesium carbonate (2.65 g, 8.13 mmol) was added. The reaction mixture was stirred at 24°C for 2 hours. The reaction mixture was quenched by adding 10 mL of water and extracted with EA (20 mL * 2). The resulting organic phase was sequentially washed with saturated brine (20 mL * 2), dried over anhydrous sodium sulfate, filtered, and the filtrate was concentrated under reduced pressure to obtain a crude product. The crude product was purified by thin-layer chromatography (PE: EA = 1:1) to obtain **009-7.** LCMS: m/z = 497.1 $[M+H]^+$.

Step 7: Synthesis of Compound **009-8**

**009-7** (1.1 g, 2.2 mmol) was dissolved in trifluoroacetic acid (2 mL), and the reaction mixture was heated to 70°C under a nitrogen atmosphere and stirred for 12 hours. After cooling to room temperature, the reaction mixture was quenched by adding 50 mL of water and extracted with EA (20 mL * 3). The resulting organic phase was sequentially washed with saturated sodium bicarbonate (20 mL * 3) and saturated brine (20 mL * 3), dried over anhydrous sodium sulfate, filtered, and the filtrate was concentrated under reduced pressure to obtain a crude product of **009-8.** LCMS: m/z = 377.1 $[M+H]^+$.

Step 8: Synthesis of Compound **009-9**

**009-8** (0.9 g, 2.4 mmol) and **B-4** (1.2 g, 3.7 mmol) were dissolved in *N,N*-dimethylformamide (9 mL), then cesium carbonate (2.3 g, 7.2 mmol) was added, and the reaction mixture was stirred at 24°C for 1.5 hours. The reaction mixture was quenched by adding 20 mL of water and extracted with EA (40 mL * 2). The resulting organic phase was sequentially washed with saturated brine (40 mL * 2), dried over anhydrous sodium sulfate, filtered, and the filtrate was concentrated under reduced pressure to obtain a crude product. The crude product was purified by column chromatography (PE: EA = 2:1) to obtain 009-9. LCMS: m/z=694.2, 696.2 $[M+Na]^+$.

Step 9: Synthesis of Compound **009-10**

**009-9** (0.5 g, 0.74 mmol) was dissolved in tetrahydrofuran (5 mL), methanol (5 mL), and ethanol (10 mL), then lithium hydroxide monohydrate (2.5 g, 59.48 mmol) was added, and the reaction mixture was stirred at 24°C for 72 hours. The reaction mixture was added with 2 M hydrochloric acid to adjust the pH to 7-8, extracted with dichloromethane (40 mL * 2), sequentially washed with saturated brine (20 mL * 2), dried over anhydrous sodium sulfate, filtered, and the filtrate was concentrated under reduced pressure to obtain **009-10.**

Step 10: Synthesis of Compound **009-11**

**009-10** (0.1 g, 155.2 μmol) was dissolved in ethyl acetate (1.0 mL). After purging with nitrogen, hydrochloric acid/ethyl acetate solution (4 M, 0.5 mL) was added, and the reaction was carried out at 20°C for 1 hour. The resulting reaction mixture was concentrated under reduced pressure to obtain the crude hydrochloride salt of **009-11.** LCMS: m/z = 544.0 $[M+H]^+$.

Step 11: Synthesis of Compound **009**

**009-11** (42 mg, crude hydrochloride salt) was dissolved in DCM (0.8 mL). After purging with nitrogen, pentafluorophenyl diphenylphosphinate (44.47 mg, 115.74 μmol) and *N*,*N*-diisopropylethylamine (149.58 mg, 1.16 mmol) were added. The reaction mixture was stirred at 24°C for 12 hours. The reaction mixture was added with 10 mL of water to quench the reaction and extracted with dichloromethane (20 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by thin-layer chromatography on silica gel plate (dichloromethane: methanol = 20:1), and then purified by prep-HPLC (chromatographic column: Waters Xbridge Prep OBD C18 150 * 40 mm * 10 μm, mobile phase: [phase A: water (10 mM ammonium bicarbonate); phase B: acetonitrile], gradient: phase B increased from 10% to 80% in the first 3 minutes, maintained for 0.9 minutes, and gradually decreased from 80% to 10% in the last 0.6 minutes) to obtain **009.** $^1$H NMR (400 MHz, $CDCl_3$) $\delta$ ppm 9.15 (s, 1H), 8.04 (s, 1H), 7.05 - 6.94 (m, 2H), 6.86 (dd, *J* = 4.5, 8.9 Hz, 1H), 6.20 - 5.86 (m, 2H), 4.73 (d, *J* = 12.0 Hz, 1H), 4.43 - 4.34 (m, 1H), 4.32 - 4.26 (m, 1H), 4.24 (d, *J* = 15.1 Hz, 1H), 4.00 - 3.87 (m, 2H), 1.80 (s, 3H), 1.62 (s, 3H); LCMS: m/z =526.1 $[M+H]^+$.

**Example 10**

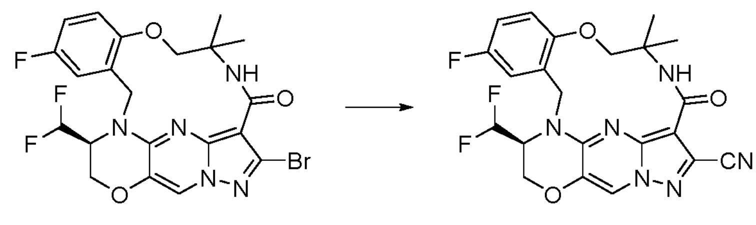

**009** **010**

**009** (40 mg, 76.0 μmol) was dissolved in *N,N*-dimethylformamide (4.0 mL), and after purging with nitrogen, cuprous cyanide (34.03 mg, 380.01 μmol) was added. The reaction mixture was stirred at 125°C for 16 hours. The reaction mixture was quenched by adding water (5 mL) and extracted with ethyl acetate (20 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by thin-layer chromatography on silica gel plate (dichloromethane: ethyl acetate: petroleum ether = 1:1:1), and then purified by prep-HPLC (chromatographic column: Waters Xbridge BEH C18 2.1 * 50 mm * 5 μm, mobile phase A: 10 mM ammonium bicarbonate aqueous solution, mobile phase B: acetonitrile, the ratio of mobile phase B increased from 10% to 80% in the first 4.5 minutes, maintained for 0.9 minutes, and decreased from 80% to 10% in the last 0.6 minutes) to obtain **010.** $^1$H NMR (400 MHz, $CDCl_3$) $\delta$ ppm 9.18 (s, 1H), 8.11 (s, 1H), 7.02 - 6.97 (m, 2H), 6.89 - 6.86 (m, 1H), 6.21 - 6.05 (m, 1H), 5.90 - 5.87 (m, 1H), 4.79 - 4.76 (m, 1H), 4.42 - 4.35 (m, 1H), 4.32 - 4.31 (m, 1H), 4.29 - 4.25 (m, 1H), 4.00 - 3.98 (m, 1H), 3.91 - 3.89 (m, 1H), 1.81 (s, 3H), 1.62 (s, 3H); LCMS: m/z =473.3 $[M+H]^+$.

**Example 11**

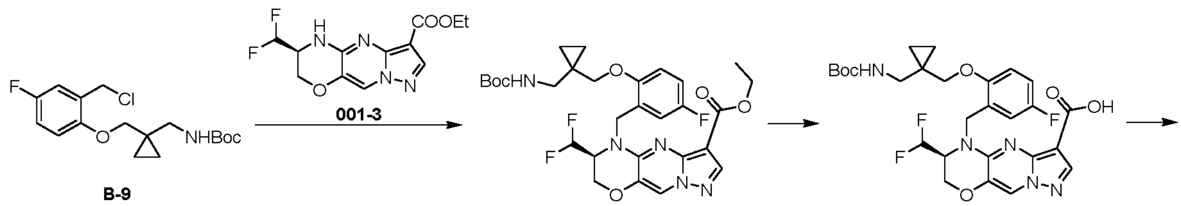


**B-9** **011-1** **011-2**

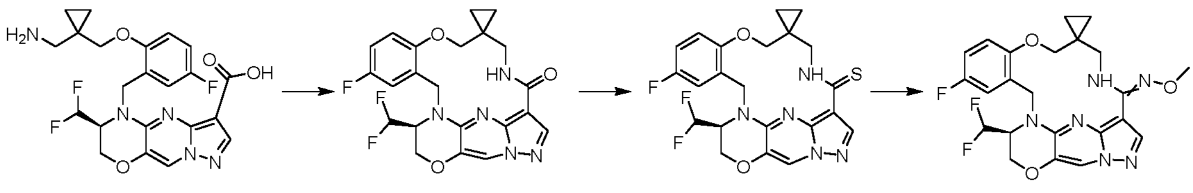

011-3 011-4 011-5 011

Step 1: Synthesis of Compound **011-1**

**001-3** (340 mg, 1.14 mmol) and **B-9** (391.96 mg, 1.14 mmol) were dissolved in *N,N*-dimethylformamide (6 mL), then cesium carbonate (1.11 g, 3.42 mmol) was added, and the reaction mixture was stirred at 24°C under a nitrogen atmosphere for 12 hours. The reaction mixture was quenched with 10 mL of water and extracted with 20 mL of ethyl acetate. The organic phase was collected after phase separation, and the aqueous phase was extracted with ethyl acetate (20 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by column chromatography (mobile phase PE: EA= 1:0 to 1:1) to obtain **011-1.** $^1$H NMR (400 MHz, $CDCl_3$) $\delta$ ppm 8.30 (s, 1H), 8.10 (s, 1H), 6.93 (dt, J = 3.3, 8.4 Hz, 1H), 6.74 (td, J = 4.2, 8.9 Hz, 2H), 6.19 - 5.85 (m, 1H), 5.73 (d, J = 14.3 Hz, 1H), 4.61 - 4.51 (m, 2H), 4.43 (q, J = 7.1 Hz, 2H), 4.39 - 4.26 (m, 2H), 3.79 - 3.74 (m, 2H), 3.33 - 3.18 (m, 2H), 1.43 (t, J = 7.1 Hz, 3H), 1.38 (s, 9H), 0.56 - 0.46 (m, 4H); LCMS: m/z =606.5 $[M+H]^+$.

Step 2: Synthesis of Compound **011-2**

**011-1** (370 mg, 610.96 μmol) was dissolved in tetrahydrofuran (8 mL), ethanol (16 mL), and methanol (8 mL), and then lithium hydroxide monohydrate solution (2 M, 16 mL) and sodium hydroxide (48.88 mg, 1.22 mmol) were added. The reaction mixture was stirred at 30°C under a nitrogen atmosphere for 12 hours. The reaction mixture was added with 2N hydrochloric acid to adjust the pH to 3-4 and extracted with 20 mL of dichloromethane. The organic phase was collected after phase separation, and the aqueous phase was extracted with dichloromethane (20 mL * 3). The organic phases were combined, sequentially washed with saturated brine (20 mL), dried over anhydrous sodium sulfate, and filtered. The filtrate was concentrated under reduced pressure to obtain a crude product. The crude product was purified by thin-layer chromatography on silica gel plate (PE: EA = 1:1) to obtain **011-2.** $^1$H NMR (400 MHz, $CDCl_3$) $\delta$ ppm 8.32 (s, 1H), 8.17 (s, 1H), 7.18 (dd, J = 3.1, 8.4 Hz, 1H), 6.96 (dt, J = 3.1, 8.4 Hz, 1H), 6.77 (dd, J = 4.3, 9.0 Hz, 1H), 6.23 - 5.90 (m, 1H), 5.70 (br d, J = 14.6 Hz, 1H), 4.70 (br s, 1H), 4.64 - 4.55 (m, 2H), 4.30 - 4.17 (m, 1H), 4.14 - 3.99 (m, 1H), 3.86 (br d, J = 9.5 Hz, 1H), 3.71 (br d, J = 9.5 Hz, 1H), 3.18 - 2.94 (m, 2H), 1.36 (s, 9H), 0.56 - 0.44 (m, 4H); LCMS: m/z =578.5 $[M+H]^+$.

Step 3: Synthesis of Compound **011-3**

**011-2** (110 mg, 190.46 μmol) was dissolved in dichloromethane (2.1 mL), then trifluoroacetic acid (9.42 mmol, 0.7 mL) was added, and the reaction mixture was reacted at 24°C for 1 hour. The resulting reaction mixture was concentrated under reduced pressure to obtain the crude trifluoroacetate salt of **011-3,** which was directly used for the next reaction. LCMS: m/z =478.4 $[M+H]^+$.

Step 4: Synthesis of Compound **011-4**

**011-3** (80 mg, crude trifluoroacetate salt) was dissolved in dichloromethane (2 mL), then benzotriazol-1-yloxytris(pyrrolidino)phosphonium hexafluorophosphate (130.80 mg, 251.34 μmol) and *N,N*-diisopropylethylamine (837.81, 145.93) were added under a nitrogen atmosphere. The reaction mixture was stirred at 24°C for 12 hours. The reaction mixture was quenched by adding 5 mL of water and extracted with 10 mL of dichloromethane. The organic phase was collected after phase separation, and the aqueous phase was extracted with dichloromethane (10 mL * 3). The organic phases were combined, sequentially washed with saturated brine (20 mL), dried over anhydrous sodium sulfate, filtered, and the filtrate was concentrated under reduced pressure to obtain a crude product. The crude product was purified by thin-layer chromatography on silica gel plate (DCM: MeOH = 20:1) to obtain **011-4.** $^1$H NMR (400 MHz, $CDCl_3$) $\delta$ ppm 8.59 (br s, 1H), 8.31 (s, 1H), 8.12 (s, 1H), 6.97 - 6.88 (m, 2H), 6.82 (dd, J = 4.4, 8.8 Hz, 1H), 6.20 - 5.83 (m, 2H), 4.73 (br d, J = 10.0 Hz, 2H), 4.33 - 4.30 (m, 1H), 4.18 - 4.13 (m, 1H), 4.01 (br dd, J = 1.7, 14.1 Hz, 1H), 3.71 (d, J = 4.3 Hz, 1H), 3.38 (br d, J = 9.6 Hz, 1H), 2.95 (br dd, J = 2.6, 13.8 Hz, 1H), 0.88 - 0.65 (m, 4H); LCMS: m/z =460.4 $[M+H]^+$.

Step 5: Synthesis of Compound **011-5**

**011-4** (76 mg, 165.43 μmol) and Lawesson's reagent (267.64 mg, 661.70 μmol) were added to 1,2-xylene (2 mL), and the reaction mixture was stirred at 140°C under a nitrogen atmosphere for 7 hours. The reaction mixture was quenched by adding 10 mL of water and extracted with 20 mL of dichloromethane. The organic phase was collected after phase separation, and the aqueous phase was extracted with dichloromethane (20 mL * 3). The organic phases were combined, sequentially washed with saturated sodium carbonate solution (20 mL * 3) and saturated brine (20 mL * 3), dried over anhydrous sodium sulfate, filtered, and the filtrate was concentrated under reduced pressure to obtain a crude product. The crude product was purified by thin-layer chromatography on silica gel plate (PE: EA = 1:2) to obtain **011-5.** $^1$H NMR (400 MHz, $CDCl_3$) $\delta$ ppm 10.17 (br s, 1H), 8.65 (s, 1H), 8.11 (s, 1H), 6.99 - 6.88 (m, 2H), 6.82 (dd, *J* = 4.5, 9.0 Hz, 1H), 6.21 - 5.85 (m, 2H), 4.80 (d, *J* = 9.4 Hz, 1H), 4.71 (d, *J* = 11.4 Hz, 1H), 4.45 - 4.22 (m, 3H), 4.16 (d, *J* = 15.3 Hz, 1H), 3.37 - 3.27 (m, 2H), 1.00 - 0.81 (m, 3H), 0.74 (td, *J* = 4.8, 9.5 Hz, 1H); LCMS: m/z =476.4 $[M+H]^+$.

Step 6: Synthesis of Compound **011**

**011-5** (35 mg, 73.61 μmol), methoxyamine hydrochloride (1.18 mmol, 89.42 μL), triethylamine (1.03 mmol, 143.44 μL), and mercuric oxide (136.79 mg, 631.56 μmol) were dissolved in *N*,*N*-dimethylformamide (3.5 mL). The reaction mixture was stirred at 60°C under a nitrogen atmosphere for 3 hours. The reaction mixture was filtered using diatomite as a filtering aid. The filtrate was washed with dichloromethane solution (20 mL * 3), and the filtrate was concentrated under reduced pressure to obtain a crude product. The crude product was purified by prep-HPLC (chromatographic column: Waters Xbridge BEH C18 50 * 2.1 mm * 5 μm, mobile phase A: 10 mM ammonium bicarbonate aqueous solution, mobile phase B: acetonitrile, the ratio of mobile phase B increased from 10% to 80% in the first 4.5 minutes, maintained for 0.9 minutes, and gradually decreased to 10% in the last 0.6 minutes) to obtain compound **011.** After the reaction mixture was filtered, the filter cake was soaked in hydrochloric acid (2 M, 30 mL) overnight, then the pH was adjusted to 10 using sodium hydroxide (2 M). Sodium sulfide solid (1 g) was added to generate a precipitate, followed by the addition of ferrous sulfate (1 g) as a co-precipitant. After standing, the mixture was filtered, and the filter cake was discarded. The pH of the filtrate was adjusted to 7-8 with hydrochloric acid (1 M) before the filtrate was discarded. $^1$H NMR (400 MHz, $CDCl_3$) $\delta$ ppm 8.85 (br s, 1H), 8.11 (s, 1H), 6.97 - 6.85 (m, 2H), 6.80 (br d, *J* = 3.5 Hz, 1H), 6.18 - 5.82 (m, 2H), 4.84 - 4.61 (m, 2H), 4.29 (br s, 2H), 4.15 - 4.04 (m, 1H), 3.99 - 3.90 (m, 1H), 3.86 (s, 3H), 3.29 (br d, *J* = 9.3 Hz, 1H), 2.85 - 2.66 (m, 1H), 0.93 - 0.63 (m, 4H); LCMS: m/z = 489.4 $[M+H]^+$.

**Example 12**

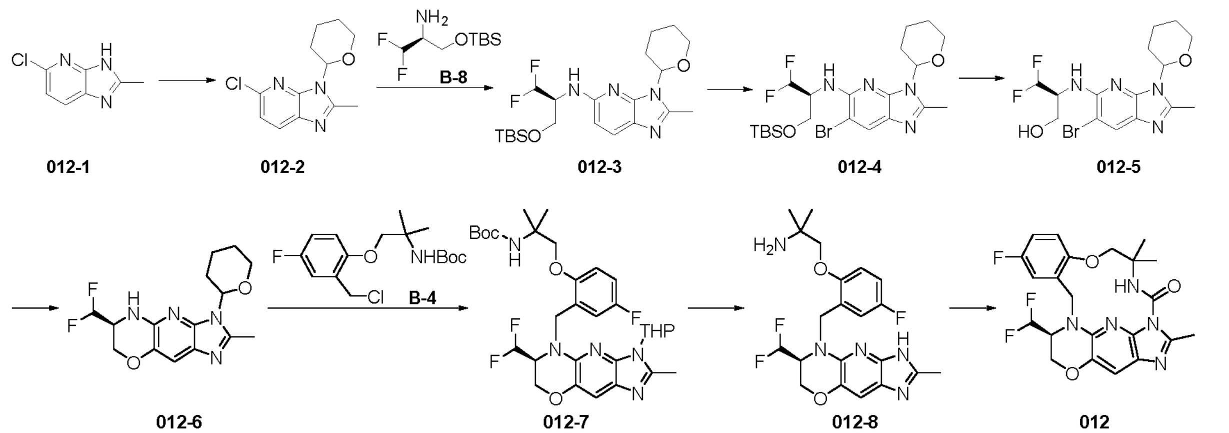


012-1 012-2 012-3 012-4 012-5

012-6 012-7 012-8 012

Step 1: Synthesis of Compound **012-2**

**012-1** (2.5 g, 14.92 mmol), 3,4-dihydro-2*H*-pyran (5.46 mL, 59.67 mmol), and pyridinium *p*-toluenesulfonate (224.9 mg, 0.89 mmol) were added to toluene (25 mL), and the reaction mixture was stirred at 100°C under a nitrogen atmosphere for 12 hours. Then, additional 3,4-dihydro-2*H*-pyran (5.46 mL, 59.67 mmol) and pyridinium *p*-toluenesulfonate (112.5 mg, 0.45 mmol) were added, and the reaction was carried out for another 6 hours. After cooling to room temperature, the reaction mixture was concentrated under reduced pressure, and the resulting crude product was purified by column chromatography (mobile phase PE: EA= 1:0 to 1:1) to obtain **012-2.** $^1$H NMR (400 MHz, $CDCl_3$) $\delta$ ppm 7.85 (d, *J*

= 8.4 Hz, 1H), 7.19 (d, *J* = 8.4 Hz, 1H), 5.89 (dd, *J* = 2.8, 11.2 Hz, 1H), 4.21 - 4.17 (m, 1H), 3.80 - 3.74 (m, 1H), 2.78 (s, 3H), 2.38 - 2.34 (m, 1H), 2.08 - 2.07 (m, 1H), 1.94 - 1.91 (m, 1H), 1.82 - 1.77 (m, 2H), 1.76 - 1.67 (m, 1H); LCMS: m/z = 252.1 $[M+H]^+$.

Step 2: Synthesis of Compound **012-3**

**012-2** (1.0 g, 3.97 mmol), **B-8** (1.07 g, 4.77 mmol), and sodium *tert*-butoxide (0.76 g, 7.95 mmol) were added to 1,4-dioxane (15 mL). Under a nitrogen atmosphere, 2-(dicyclohexylphosphino)-3,6-dimethoxy-2',4',6'-triisopropyl-1,1'-biphenyl (0.21 g, 0.40 mmol) and palladium acetate (44.6 mg, 0.20 mmol) were added. The reaction mixture was stirred at 80°C for 12 hours. After cooling to room temperature, the reaction mixture was added with 20 mL of water to quench the reaction and extracted with dichloromethane (20 mL * 3). The organic phase was collected after phase separation, washed with saturated brine (20 mL), dried over anhydrous sodium sulfate, filtered, and the filtrate was concentrated under reduced pressure to obtain a crude product. The crude product was purified by column chromatography (mobile phase PE: EA = 1:0 to 0:1) to obtain **012-3.** $^1H$ NMR (400 MHz, $CDCl_3$) $\delta$ ppm 7.66 (d, *J* = 8.4 Hz, 1H), 6.37 (d, *J* = 8.4 Hz, 1H), 6.23 - 5.92 (m, 1H), 5.69-5.65 (m, 1H), 4.75 (dd, *J* = 5.6, 8.4 Hz, 1H), 4.55 - 4.40 (m, 1H), 4.20 - 4.17 (m, 1H), 3.97 - 3.79 (m, 1H), 3.78 - 3.72 (m, 2H), 2.67 (s, 3H), 2.10 - 2.05 (m, 1H), 1.90 - 1.87 (m, 1H), 1.78 - 1.63 (m, 3H), 0.92 (s, 9H), 0.11 (s, 3H), 0.09 (s, 3H); LCMS: m/z = 441.3 $[M+H]^+$.

Step 3: Synthesis of Compound **012-4**

**012-3** (1.05 g, 2.38 mmol) and sodium acetate (1.27 g, 15.49 mmol) were added to acetic acid (25 mL), and the mixture was cooled to 0°C under a nitrogen atmosphere. Pyridinium tribromide (1.14 g, 3.57 mmol) was added, and the reaction mixture was slowly warmed to 25°C and stirred for 2 hours. 200 mL of saturated sodium thiosulfate was added to the reaction mixture quench the reaction, and then 50 mL of water was added and stirred for half an hour. The reaction mixture was extracted with dichloromethane (50 mL), then the organic phase was collected after phase separation, and the aqueous phase was extracted with dichloromethane (50 mL * 3). The organic phases were combined, sequentially washed with saturated brine (100 mL), dried over anhydrous sodium sulfate, filtered, and the filtrate was concentrated under reduced pressure to obtain a crude product of **012-4.** LCMS: m/z = 519.2, 521.2 $[M+H]^+$.

Step 4: Synthesis of Compound **012-5**

**012-4** (1.2 g, 2.31 mmol) and a solution of tetrabutylammonium fluoride in tetrahydrofuran (1 M, 3.46 mL) were added to tetrahydrofuran (18.75 mL). Under a nitrogen atmosphere, the reaction mixture was stirred at 20°C for 12 hours. The resulting reaction mixture was concentrated under reduced pressure to obtain a crude product, which was purified by column chromatography (mobile phase PE: EA = 1:0 to 1:1) to obtain **012-5.** $^1H$ NMR (400 MHz, $CDCl_3$) $\delta$ ppm 7.93 (d, *J* = 2.4 Hz, 1H), 6.33 - 6.05 (m, 1H), 5.60 - 5.53 (m, 2H), 4.55 - 4.45 (m, 1H), 4.20 - 4.14 (m, 2H), 4.12 - 4.00 (m, 1H), 3.74 - 3.68 (m, 1H), 2.65 - 2.52 (m, 5H), 2.11 - 2.05 (m, 1H), 1.88 - 1.85 (m, 1H), 1.77 - 1.63 (m, 3H); LCMS: m/z = 405.1, 407.0 $[M+H]^+$.

Step 5: Synthesis of Compound **012-6**

**012-5** (0.25 g, 0.62 mmol) and cesium carbonate (0.26 g, 0.80 mmol) were added to 1,4-dioxane (6.25 mL). Under a nitrogen atmosphere, 2-(di-tert-butylphosphino)-1,1'-binaphthyl (0.1 g, 0.25 mmol) and tris(dibenzylideneacetone)dipalladium (0.11 g, 0.12 mmol) were added. The reaction mixture was slowly heated to 88°C and stirred for 1 hour, then heated to 100°C and stirred for 1 hour, and further heated to 115°C and stirred for 12 hours. After slowly cooling to room temperature, the resulting reaction mixture was concentrated under reduced pressure, and the crude product was purified by column chromatography (mobile phase PE: EA= 1:0 to 0:1) to obtain **012-6.** LCMS: m/z = 325.1 $[M+H]^+$.

Step 6: Synthesis of Compound **012-7**

**012-6** (0.10 g, 0.31 mmol), **B-4** (0.15 g, 0.46 mmol), and tetrabutylammonium iodide (29.6 mg, 0.08 mmol) were added to N,N-dimethylformamide (2.0 mL). Under a nitrogen atmosphere, cesium carbonate (0.30 g, 0.93 mmol) was added, and the reaction mixture was heated to 40°C and stirred for 16 hours. After cooling to room temperature, the reaction mixture was added with 10 mL of water to quench the reaction and extracted with ethyl acetate (10 mL * 3). The organic phase was collected after phase separation, washed with saturated brine (10 mL), dried over anhydrous sodium sulfate, filtered, and the filtrate was concentrated under reduced pressure to obtain a crude product. The resulting crude product was purified by column chromatography (mobile phase PE: EA= 1:0 to 0:1) to obtain **012-7.** LCMS: m/z = 620.3 $[M+H]^+$.

Step 7: Synthesis of Compound **012-8**

**012-7** (0.15 g, 0.24 mmol) was added to a solution of ethyl acetate in hydrochloric acid(4.0 M, 3.03 mL), and the reaction mixture was stirred at 20°C for 0.5 hours. The resulting reaction mixture was concentrated under reduced pressure to obtain the crude hydrochloride salt of **012-8,** which was directly used for the next step. LCMS: m/z = 436.1 $[M+H]^+$.

Step 8: Synthesis of Compound **012**

**012-8** (0.07 g, crude hydrochloride salt) was added to dichloromethane (3.5 mL), cooled to 0°C under a nitrogen atmosphere. Then, a solution of *N,N'*-carbonyldiimidazole (48.1 mg, 0.30 mmol) in dichloromethane (0.7 mL) and 4-dimethylaminopyridine (5.4 mg, 0.45 mmol) were slowly added dropwise. The reaction mixture was slowly warmed to 25°C and stirred for 12.0 hours. 10% citric acid aqueous solution (10 mL) was added to the reaction mixture, and the pH was adjusted to about 6. The reaction mixture was extracted with dichloromethane (10 mL * 2), and the organic phase was collected after phase separation and concentrated under reduced pressure to obtain a crude product. The crude product was purified by thin-layer chromatography on silica gel plate (PE: EA = 0:1), and then purified by prep-HPLC (column model: Waters Xbridge Prep OBD C18 column 150 * 40 mm * 10 μm, mobile phase: phase A(10 mM ammonium bicarbonate aqueous solution), phase B: acetonitrile, gradient: the gradient of phase B increased from 25% to 65% in 8 minutes for gradient elution) to obtain 012. LCMS: m/z = 462.3 $[M+H]^+$; $^1H$ NMR (400 MHz, $CDCl_3$) $\delta$ ppm 10.32 (s, 1H), 7.36 (s, 1H), 7.02 - 7.00 (m, 1H), 6.95 - 6.94 (m, 1H), 6.83 - 6.81 (m, 1H), 6.05 - 5.77 (m, 2H), 4.67 - 4.64 (m, 1H), 4.25 - 4.20 (m, 2H), 4.16 - 4.06 (m, 1H), 4.04 - 3.91 (m, 2H), 2.81 (s, 3H), 1.75 (s, 3H), 1.68 (s, 3H).

**Example 13**

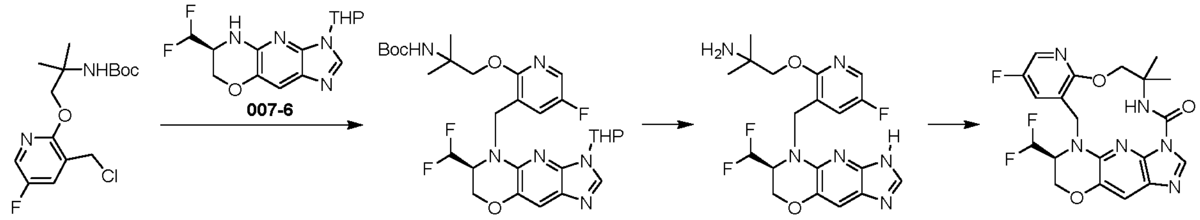

B-6 007-6 013-1 013-2 013

Step 1: Synthesis of Compound **013-1**

**B-6** (0.26 g, 0.77 mmol) and **007-6** (0.12 g, 0.39 mmol) were dissolved in *N,N*-dimethylformamide (8 mL). Then, cesium carbonate (0.19 g, 0.58 mmol) was added. The reaction mixture was stirred at 5°C for 4 hours, followed by warming to 20°C and reacting for 12 hours. The reaction mixture was added with 20 mL of water to quench the reaction and extracted with dichloromethane (100 mL * 2). The organic phase was collected after phase separation, dried over anhydrous sodium sulfate, filtered, and the filtrate was concentrated under reduced pressure to obtain a crude product. The crude product was purified by thin-layer chromatography (PE: EA = 0:1) to obtain **013-1.** $^1H$ NMR (400 MHz, $CDCl_3$) $\delta$ ppm 8.01 (br d, *J* = 8.1 Hz, 1H), 7.90 (s, 1H), 7.65 (br d, *J* = 8.1 Hz, 1H), 7.48 (s, 1H), 6.10 - 5.75 (m, 1H), 5.68 - 5.55 (m, 1H), 5.31 (s, 1H), 4.58 - 4.42 (m, 3H), 4.35 (s, 1H), 4.25 - 4.18 (m, 1H), 3.90 (br s, 1H), 3.77 (br s, 1H), 2.06 (s, 3H), 1.77 - 1.73 (m, 1H), 1.71 (br d, J = 2.8 Hz, 1H), 1.67 (br s, 1H), 1.62 - 1.55 (m, 2H), 1.43 (s, 9H), 1.39 (d, *J* = 9.7 Hz, 6H); LCMS: m/z = 607.4 $[M+H]^+$.

Step 2: Synthesis of Compound **013-2**

**013-1** (0.1 g, 0.16 mmol) and a methanol solution of hydrochloric acid (4 M, 31.25 mL) were added to a reaction flask, and the reaction mixture was stirred at 25°C for 12 hours. The reaction mixture was concentrated under reduced pressure to obtain the crude hydrochloride salt of **013-2,** which was directly used for the next step without purification. $^1H$ NMR (400 MHz, $CD_3OD$) $\delta$ ppm 9.04 (s, 1H), 7.93 (d, *J* = 2.8 Hz, 1H), 7.57 (s, 1H), 7.44 (dd, *J* = 2.3, 8.9 Hz, 1H), 6.21 (dt, *J* = 4.5, 55.2 Hz, 1H), 5.24 (br d, *J* = 17.4 Hz, 1H), 4.94 (br s, 1H), 4.70 (br d, *J* = 11.8 Hz, 1H), 4.54 - 4.47 (m, 1H), 4.44 - 4.38 (m, 1H), 4.28 - 4.14 (m, 2H), 1.50 (d, *J* = 2.6 Hz, 6H); LCMS: m/z = 423.2 $[M+H]^+$.

Step 3: Synthesis of Compound **013**

**013-2** (40 mg, crude hydrochloride salt) was dissolved in dichloromethane (0.4 mL) and cooled to 0°C. Then, a solution of *N,N'*-carbonyldiimidazole (18.43 mg, 113.64 μmol) in dichloromethane (0.2 mL) and 4-dimethylaminopyridine (2.31 mg, 18.94 μmol) were slowly added dropwise. The reaction mixture was stirred at 25°C for 3 hours. The reaction mixture was added with water (10 mL) to quench the reaction and extracted with dichloromethane (10 mL * 3). The organic phases were combined after phase separation, sequentially washed with saturated brine (10 mL * 2), dried over anhydrous sodium sulfate, filtered, and the filtrate was concentrated under reduced pressure to obtain a crude product. The crude product was purified by thin-layer chromatography (PE: EA = 0:1), and then purified by prep-HPLC [chromatographic column: Phenomenex Luna C18 column, 100 * 30 mm * 3 μm; mobile phase: phase A (0.2% trifluoroacetic acid aqueous solution), phase B: acetonitrile, gradient: the gradient of phase B increased from 40% to 70% in 8 minutes for gradient elution) to obtain 013. $^1$H NMR (400 MHz, $CDCl_3$) $\delta$ ppm 9.95 (br s, 1H), 8.40 (s, 1H), 7.95 (d, *J* = 2.9 Hz, 1H), 7.50 (s, 1H), 7.37 (dd, *J* = 2.6, 7.9 Hz, 1H), 5.95 (dt, *J* = 6.8, 55.5 Hz, 1H), 5.58 (br d, *J* = 15.8 Hz, 1H), 4.86 (d, *J* = 10.6 Hz, 1H), 4.68 (br d, *J* = 11.6 Hz, 1H), 4.30 - 4.17 (m, 3H), 3.97 (d, *J* = 10.8 Hz, 1H), 1.77 (s, 3H), 1.68 (s, 3H); LCMS: m/z = 449.1 $[M+H]^+$.

**Example 14**

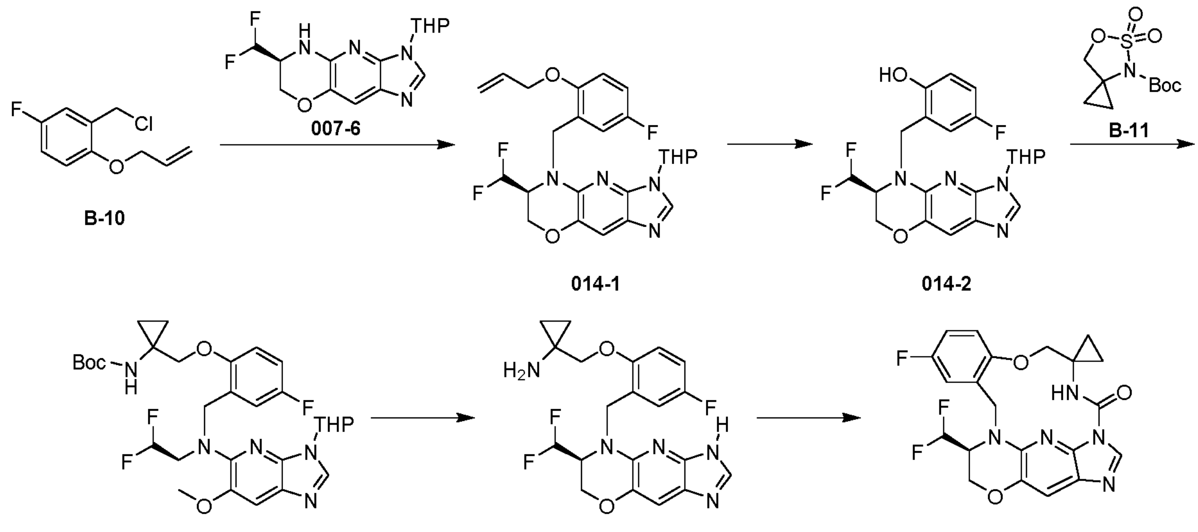


B-10

014-1

014-2

014-3

014-4

014

Step 1: Synthesis of Compound **014-1**

**007-6** (0.14 g, 0.45 mmol), **B-10** (0.14 g, 0.68 mmol), and cesium carbonate (0.29 g, 0.90 mmol) were dissolved in *N,N*-dimethylformamide (1.0 mL), and the reaction mixture was stirred at 20°C for 12 hours. The resulting reaction mixture was concentrated under reduced pressure to obtain a crude product, which was purified by thin-layer chromatography (PE: EA = 0:1) to obtain **014-1.** LCMS: m/z = 475.2 $[M+H]^+$.

Step 2: Synthesis of Compound **014-2**

**014-1** (0.16 g, 0.34 mmol) and 1,3-dimethylbarbituric acid (0.11 g, 0.67 mmol) were added to ethanol (3.0 mL). Under a nitrogen atmosphere, tetrakis(triphenylphosphine)palladium was added, and the reaction mixture was stirred at 40°C for 12 hours. The resulting reaction mixture was concentrated under reduced pressure to obtain a crude product, which was purified by thin-layer chromatography (PE: EA = 0:1) to obtain **014-2.** LCMS: m/z = 435.2 $[M+H]^+$.

Step 3: Synthesis of Compound **014-3**

**B-11** (80.3 mg, 0.32 mmol), **014-2** (0.14 g, 0.32 mmol), and cesium carbonate (0.26 g, 0.81 mmol) were added to *N,N*-dimethylformamide (3.0 mL), and the reaction mixture was stirred at 70°C for 12 hours. The reaction mixture was added with water (10 mL) to quench the reaction and extracted with ethyl acetate (5 mL * 3). The organic phases were combined after phase separation, sequentially washed with saturated brine (10 mL), dried over anhydrous sodium sulfate,

filtered, and the filtrate was concentrated to obtain a crude product. The crude product was purified by thin-layer chromatography (ethyl acetate: methanol = 10:1) to obtain **014-3.**

Step 4: Synthesis of Compound **014-4**

**014-3** (80.0 mg, 0.13 mmol) and a solution of ethyl acetate in hydrochloric acid (4 M, 0.83 mL) were added to ethyl acetate (0.2 mL). The reaction mixture was stirred at 20°C for 1 hour. The resulting reaction mixture was concentrated under reduced pressure to obtain the crude hydrochloride salt of **014-4,** which was directly used for the next step. LCMS: m/z = 420.1 $[M+H]^+$.

Step 5: Synthesis of Compound **014**

**014-4** (45.0 mg, crude hydrochloride salt) and dichloromethane (0.5 mL) were added to a reaction flask, cooled to 0°C under a nitrogen atmosphere. Then at 0°C, a solution of N,N'-carbonyldiimidazole (48.0 mg, 0.3 mmol) in dichloromethane (0.1 mL) and 4-dimethylaminopyridine (36.2 mg, 0.3 mmol) were slowly added dropwise. The reaction mixture was then slowly warmed to 25°C and stirred for 1 hour. The reaction mixture was added with water (2 mL) to quench the reaction and extracted with dichloromethane (2 mL * 3). The organic phases were combined after phase separation, sequentially washed with saturated brine (2 mL), dried over anhydrous sodium sulfate, filtered, and the filtrate was concentrated to obtain a crude product. The crude product was purified by thin-layer chromatography (dichloromethane: ethyl acetate = 1:1), and then purified by prep-HPLC (chromatographic column: Waters Xbridge BEH C18 column, 100 * 30 mm * 10 μm, mobile phase: phase A (10 mM ammonium bicarbonate aqueous solution), phase B: acetonitrile, gradient: the gradient of phase B increased from 25% to 55% in 8 minutes for gradient elution) to obtain **014.** $^1$H NMR (400 MHz, $CDCl_3$) $\delta$ ppm 10.36 (s, 1H), 8.31 (s, 1H), 7.48 (s, 1H), 6.94 - 6.90 (m, 2H), 6.76 - 6.74 (m, 1H), 5.96 - 5.80 (m, 2H), 4.68 (d, *J* = 10.8 Hz, 1H), 4.45 (d, *J* = 8.4 Hz, 1H), 4.26 - 4.25 (m, 2H), 4.23 (d, *J* = 8.4 Hz, 1H), 3.79 (d, *J* = 9.2 Hz, 1H), 2.27 - 2.23 (m, 1H), 1.15 - 1.05 (m, 2H), 0.99 - 0.96 (m, 1H); LCMS: m/z = 446.2 $[M+H]^+$.

**Example 15**

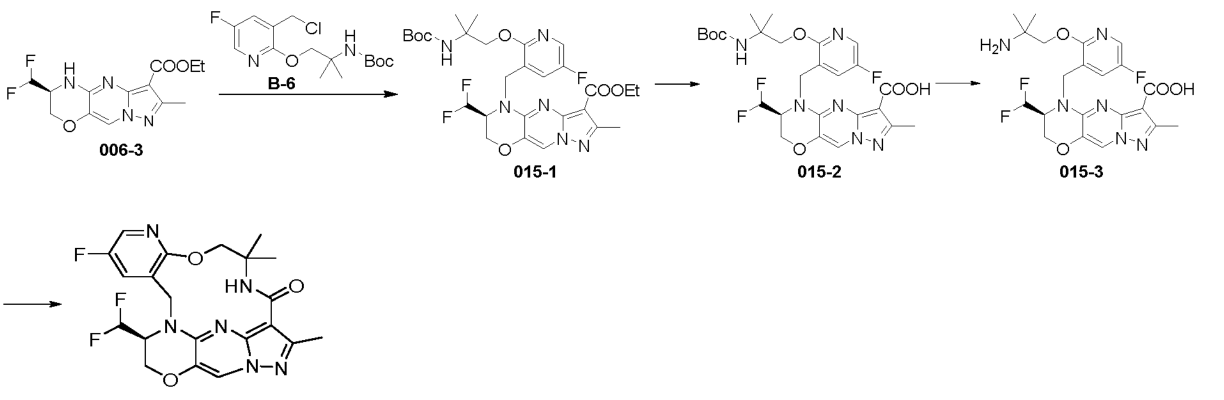
006-3
B-6
015-1
015-2
015-3
015

Step 1: Synthesis of Compound **015-1**

**006-3** (0.27 g, 0.86 mmol) and **B-6** (0.43 g, 1.30 mmol) were dissolved in *N,N*-dimethylformamide (3.0 mL), then cesium carbonate (0.85 g, 2.59 mmol) was added, and the reaction mixture was stirred at 25°C for 3 hours. The reaction mixture was added with water (10 mL) to quench the reaction and extracted with ethyl acetate (30 mL * 3). The organic phases were combined after phase separation, sequentially washed with saturated brine (30 mL * 2), dried over anhydrous sodium sulfate, filtered, and the filtrate was concentrated under reduced pressure to obtain a crude product. The crude product was purified by column chromatography (petroleum ether: ethyl acetate = 10:1 to 1:1) to obtain **015-1.** $^1$H NMR (400 MHz, $CDCl_3$) $\delta$ ppm 8.39 - 8.32 (m, 1H), 8.01 (s, 1H), 7.91 (d, *J* = 3.1 Hz, 1H), 6.17 - 5.79 (m, 1H), 5.53 (d, *J* = 14.5 Hz, 1H), 4.67 - 4.60 (m, 1H), 4.58 - 4.51 (m, 2H), 4.50 - 4.39 (m, 3H), 4.37 - 4.25 (m, 1H), 3.96 - 3.87 (m, 1H), 2.64 - 2.58 (m, 3H), 1.45 - 1.36 (m, 19H); LCMS: m/z = 631.2 $[M+Na]^+$.

Step 2: Synthesis of Compound **015-2**

**015-1** (0.36 g, 0.59 mmol), an aqueous solution of lithium hydroxide monohydrate (2 M, 20 mL), and sodium hydroxide (47.3 mg, 1.18 mmol) were added to tetrahydrofuran (7.2 mL), methanol (7.2 mL), and ethanol (14.4 mL), and the reaction mixture was stirred at 30°C for 40 hours. The reaction mixture was added with water (2 mL) and washed with ethyl acetate (10 mL), and the phases were separated. The resulting aqueous phase was added with 2 M hydrochloric acid to adjust the pH to about 3 and extracted with dichloromethane (10 mL * 3). The organic phases were combined after phase separation, sequentially washed with saturated brine (30 mL* 2), dried over anhydrous sodium sulfate, filtered, and the filtrate was concentrated under reduced pressure to obtain a crude product. The crude product was purified by thin-layer chromatography (petroleum ether: ethyl acetate = 1:1) to obtain **015-2.** LCMS: m/z = 581.3 $[M+H]^+$.

Step 3: Synthesis of Compound **015-3**

**015-2** (80 mg, 137.80 μmol) was added to trifluoroacetic acid (4 mL), and the reaction mixture was stirred at 25°C for 0.5 hours, and concentrated under reduced pressure to obtain a crude product of **015-3,** which was directly used for the next step. LCMS: m/z = 481.3 $[M+H]^+$.

Step 4: Synthesis of Compound **015**

**015-3** (80.0 mg, crude product) was dissolved in dichloromethane (2 mL), and then N,N-diisopropylethylamine (1.67 mmol, 290.03 μL) and pentafluorophenyl diphenylphosphinate (96.0 mg, 0.25 mmol) were sequentially added. The reaction mixture was stirred at 25°C for 12 hours. The reaction was quenched with 10 mL of water and extracted with dichloromethane (20 mL). The organic phase was collected after phase separation, and the aqueous phase was extracted with dichloromethane (20 mL * 3). The organic phases were combined, sequentially washed with saturated sodium carbonate solution (20 mL * 3) and saturated brine (20 mL * 3), dried over anhydrous sodium sulfate, filtered, and the filtrate was concentrated under reduced pressure to obtain a crude product. The crude product was purified by thin-layer chromatography (PE: EA = 1: 1), and then purified by prep-HPLC (chromatographic column: Waters Xbridge Prep OBD C18 column 150 * 40 mm * 10 μm, mobile phase: phase A:10 mM ammonium bicarbonate aqueous solution, phase B: acetonitrile; gradient: the gradient of phase B increased from 25% to 55% in 8 minutes) to obtain **015.** H NMR (400 MHz, $CDCl_3$) $\delta$ 8.95 (br s, 1H), 8.02 (br d, *J* = 27.9 Hz, 2H), 7.39 - 7.29 (m, 1H), 6.22 - 5.85 (m, 1H), 5.72 (br d, *J* = 15.4 Hz, 1H), 4.88 - 4.66 (m, 2H), 4.34 - 4.20 (m, 3H), 3.88 (br *d, J* = 10.1 Hz, 1H), 1.78 (br s, 3H), 1.62 (br *d, J* = 11.4 Hz, 6H); LCMS: m/z = 463.2 $[M+H]^+$.

**Example 16**

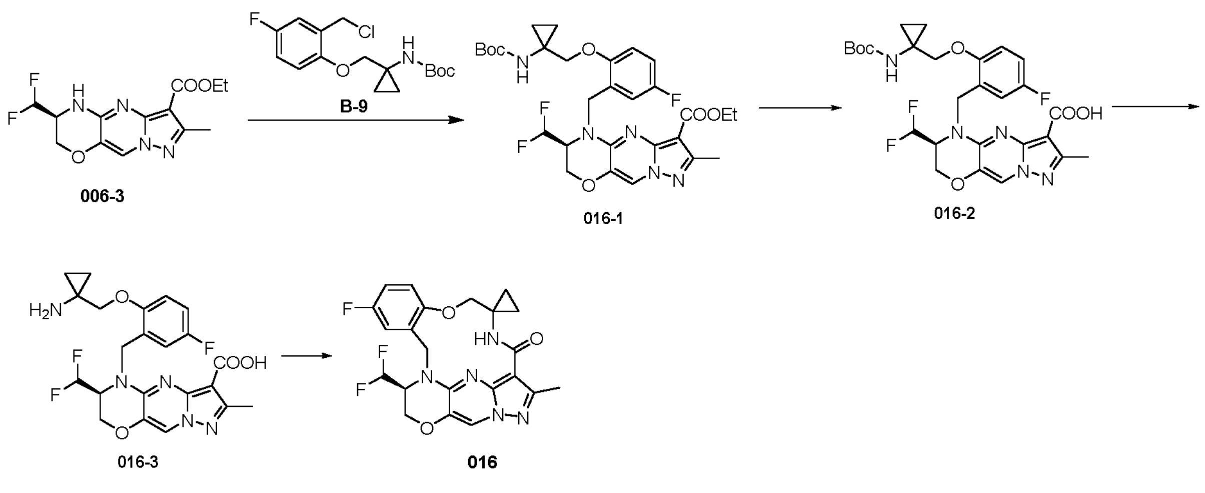


006-3 016-1 016-2 016-3 016

Step 1: Synthesis of Compound **016-1**

**006-3** (0.27 g, 0.86 mmol) and **B-9** (0.31 g, 0.95 mmol) were dissolved in *N,N*-dimethylformamide (5.4 mL), then cesium carbonate (0.85 g, 2.59 mmol) was added, and the reaction mixture was stirred at 25°C for 12 hours. The reaction mixture was added with water (10 mL) to quench the reaction and extracted with ethyl acetate (20 mL), and the aqueous

phase was then extracted with ethyl acetate (20 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by column chromatography (petroleum ether: ethyl acetate = 1:1 to 1:1) to obtain **016-1.** $^1$H NMR (400 MHz, $CDCl_3$) $\delta$ 8.01 (s, 1H), 7.62 (br d, *J* = 6.3 Hz, 1H), 6.95 (dt, *J* = 3.1, 8.4 Hz, 1H), 6.76 (dd, *J* = 4.3, 9.0 Hz, 1H), 6.16 - 5.91 (m, 1H), 5.88 - 5.82 (m, 1H), 5.06 (br s, 1H), 4.56 - 4.47 (m, 2H), 4.43 (q, *J* = 7.1 Hz, 2H), 4.33 - 4.24 (m, 1H), 4.05 (d, *J* = 9.8 Hz, 1H), 3.90 - 3.78 (m, 2H), 2.62 (s, 3H), 1.45 - 1.41 (m, 12H), 0.87 (br dd, *J* = 5.1, 9.8 Hz, 1H), 0.80 - 0.68 (m, 3H); LCMS: m/z = 606.4 $[M+H]^+$.

Step 2: Synthesis of Compound **016-2**

**016-1** (0.37 g, 0.61 mmol), an aqueous solution of lithium hydroxide monohydrate (2 M, 24.7 mL), and sodium hydroxide (48.9 mg, 1.22 mmol) were dissolved in tetrahydrofuran (12.3 mL), methanol (12.3 mL), and ethanol (24.7 mL), and the reaction mixture was stirred at 35°C for 55 hours. The reaction mixture was added with water (2 mL) and washed with dichloromethane (20 mL), and the phases were separated. The resulting aqueous phase was added with 2 M hydrochloric acid to adjust the pH to about 3 and extracted with dichloromethane (20 mL * 3). The organic phases were combined after phase separation, sequentially washed with saturated brine (20 mL), dried over anhydrous sodium sulfate, filtered, and the filtrate was concentrated under reduced pressure to obtain a crude product. The crude product was purified by column chromatography (mobile phase petroleum ether: ethyl acetate = 1:0 to 1:1) to obtain **016-2.** $^1$H NMR (400 MHz, $CDCl_3$) $\delta$ 10.27 - 9.95 (m, 1H), 8.11 (s, 1H), 7.10 (dd, *J* = 2.9, 8.4 Hz, 1H), 6.97 (dt, *J* = 3.0, 8.4 Hz, 1H), 6.80 (dd, *J* = 4.3, 9.0 Hz, 1H), 6.15 - 5.86 (m, 1H), 5.66 (br d, *J* = 15.1 Hz, 1H), 4.95 - 4.78 (m, 2H), 4.63 - 4.51 (m, 2H), 4.15 - 3.90 (m, 6H), 1.41 (br s, 9H), 0.97 (br d, *J* = 6.5 Hz, 2H), 0.83 - 0.80 (m, 2H); LCMS: m/z = 578.3 $[M+H]^+$.

Step 3: Synthesis of Compound **016-3**

**016-2** (0.17 g, 0.29 mmol) and TFA (13.5 mmol, 1 mL) were added to dichloromethane (2.0 mL), and the reaction mixture was stirred at 25°C for 0.5 hours, and concentrated under reduced pressure to obtain a crude product of **016-3,** which was directly used for the next step. LCMS: m/z = 478.3 $[M+H]^+$.

Step 4: Synthesis of Compound **016**

**016-3** (95.0 mg, crude product) was dissolved in dichloromethane (2 mL), and then N,N-diisopropylethylamine (1.99 mmol, 346.6 µL) and pentafluorophenyl diphenylphosphinate (114.7 mg, 0.30 mmol) were sequentially added. The reaction mixture was stirred at 25°C for 2 hours. The reaction mixture was added with 5 mL of water and extracted with dichloromethane (10 mL). The organic phase was collected after phase separation, and the aqueous phase was then extracted with dichloromethane (10 mL * 3). The organic phases were combined, sequentially washed with saturated sodium carbonate solution (20 mL * 3) and saturated brine (20 mL * 3), dried over anhydrous sodium sulfate, filtered, and the filtrate was concentrated under reduced pressure to obtain a crude product. The crude product was purified by thin-layer chromatography (PE: EA = 0:1), and then purified by prep-HPLC (chromatographic column: Waters Xbridge Prep OBD C18 column 150 * 40 mm * 10 µm, mobile phase: phase A: 10 mM ammonium bicarbonate aqueous solution, phase B: acetonitrile; gradient: the gradient of phase B increased from 30% to 65% in 8 minutes) to obtain 016. $^1$H NMR (400 MHz, $CDCl_3$) $\delta$ 9.04 (s, 1H), 8.01 (s, 1H), 6.98 - 6.85 (m, 2H), 6.73 (dd, *J* = 4.5, 9.0 Hz, 1H), 6.18 - 5.87 (m, 2H), 4.70 (br d, *J* = 11.4 Hz, 1H), 4.43 (d, *J*= 9.3 Hz, 1H), 4.33 - 4.22 (m, 2H), 4.14 (d, *J* = 15.3 Hz, 1H), 3.67 (d, *J* = 9.3 Hz, 1H), 2.58 (s, 3H), 2.28 - 2.16 (m, 1H), 1.08 - 0.97 (m, 2H), 0.84 - 0.74 (m, 1H)LCMS: m/z = 460.2 $[M+H]^+$.

**Example 17**

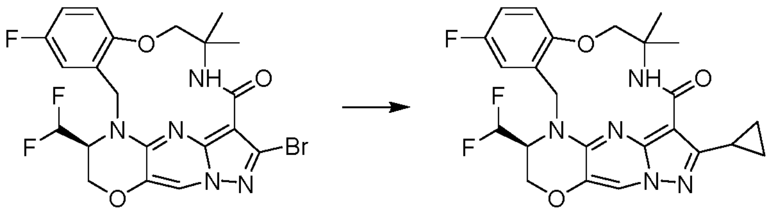

009 017

**009** (60.0 mg, 114.0 µmol), cyclopropylboronic acid (9.8 mg, 114.0 µmol), cesium carbonate (74.3 mg, 228.0

μmol), 1,4-dioxane (2.5 mL), and water (0.5 mL) were added to a reaction flask. After purging three times with nitrogen, tetrakis(triphenylphosphine)palladium (26.4 mg, 22.8 μmol) was added. The mixture was reacted at 100°C for 5 hours. After cooling to room temperature, the reaction mixture was diluted with water (2 mL) and extracted with dichloromethane (5 mL * 3). The organic phases were combined, washed with saturated brine (10 mL), dried over anhydrous sodium sulfate, filtered, and the filtrate was concentrated under reduced pressure to obtain a crude product. The crude product was purified by thin-layer chromatography on silica gel plate (PE: EA = 1:1), and then purified by prep-HPLC (chromatographic column: Waters Xbridge Prep OBD C18 column 150 * 40 mm * 10 μm, mobile phase: phase A:10 mM ammonium bicarbonate aqueous solution, phase B: acetonitrile; gradient: the gradient of phase B increased from 40% to 70% in 8 minutes) to obtain **017.** $^1H$ NMR (400 MHz, $CDCl_3$) $\delta$ 9.26 (s, 1H), 7.99 (s, 1H), 7.02 - 6.95 (m, 2H), 6.88 - 6.83 (m, 1H), 6.18 - 5.83 (m, 2H), 4.68 (brd, *J*= 12.3 Hz, 1H), 4.37 - 4.23 (m, 2H), 4.20 (d, *J*= 15.4 Hz, 1H), 3.99 - 3.90 (m, 2H), 3.04 - 2.92 (m, 1H), 1.81 (s, 3H), 1.64 (s, 3H), 1.27 (brs, 2H), 1.00 (d, *J* = 5.4 Hz, 2H); LCMS: m/z = 488.4 $[M+H]^+$.

**Example 18**

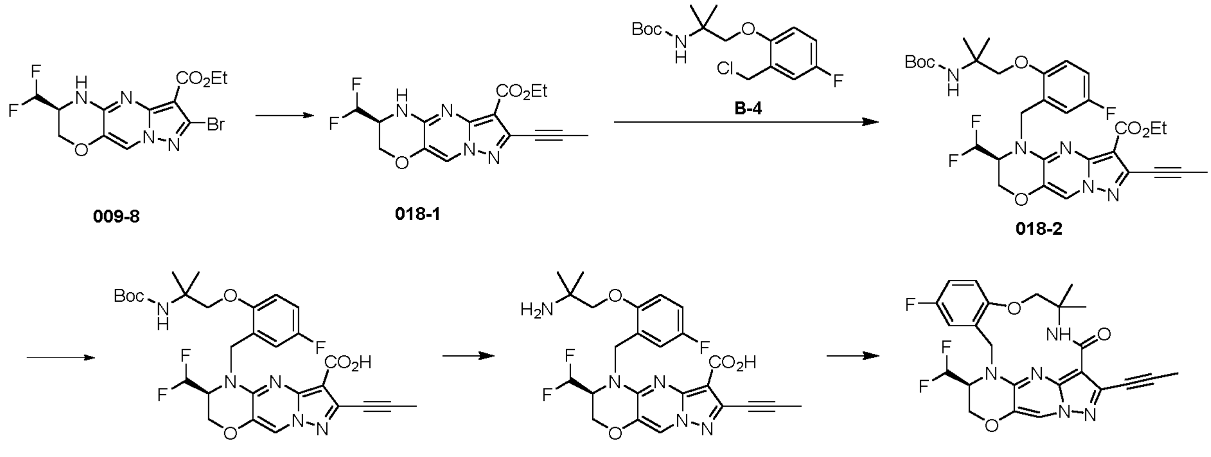


009-8 018-1 018-2

018-3 018-4 018

Step 1: Synthesis of Compound **018-1**

**009-8** (0.3 g, 0.8 mmol) was added to acetonitrile (0.2 mL). After purging three times with nitrogen, [(2-dicyclohexylphosphino-2',4',6'-triisopropyl-1,1'-biphenyl)[2-(2'-amino-1,1'-biphenyl)]methanesulfonato] palladium (67.3 mg, 0.08 mmol) and cesium carbonate (0.52 g, 1.6 mmol) were added. The mixture was slowly heated to 80°C, and then a solution of propargyl (1 M, 2.39 mL) in acetonitrile (0.2 mL) was added. The reaction was carried out for 12 hours. After cooling to room temperature, the reaction mixture was added with water (5.0 mL) to quench the reaction and extracted with dichloromethane (5.0 mL * 3). The organic phases were combined, sequentially washed with saturated brine (10.0 mL * 3), dried over anhydrous sodium sulfate, filtered, and the filtrate was concentrated under reduced pressure to obtain a crude product. The crude product was purified by column chromatography (petroleum ether: ethyl acetate = 1:0 to 1:1) to obtain **018-1.** LCMS: m/z = 337.2 $[M+H]^+$.

Step 2: Synthesis of Compound **018-2**

**018-1** (0.13 g, 0.39 mmol) and **B-4** (0.19 g, 0.58 mmol) were dissolved in *N,N*-dimethylformamide (2.0 mL), then cesium carbonate (0.38 g, 1.16 mmol) was added, and the reaction mixture was stirred at 25°C under a nitrogen atmosphere for 2 hours. The reaction mixture was added with water (2.0 mL) to quench the reaction and extracted with ethyl acetate (10.0 mL * 3). The organic phases were combined, sequentially washed with saturated brine (10.0 mL * 3), dried over anhydrous sodium sulfate, filtered, and the filtrate was concentrated under reduced pressure to obtain a crude product. The crude product was purified by column chromatography (petroleum ether: ethyl acetate = 1:0 to 1:1) to obtain **018-2.** $^1H$ NMR (400 MHz, $CDCl_3$) $\delta$ 8.00 (s, 1H), 7.68 - 7.53 (m, 1H), 7.02 - 6.91 (m, 1H), 6.90 - 6.79 (m, 1H), 6.13 - 5.77 (m, 2H), 4.55 - 4.36 (m, 5H), 4.28 - 4.15 (m, 2H), 4.04 - 3.93 (m, 1H), 3.91 - 3.79 (m, 1H), 2.18 (s, 3H), 1.62 - 1.56 (m, 3H), 1.41 (brs, 9H), 1.36 (brs, 3H), 1.32 (brs, 3H); LCMS: m/z = 632.3 $[M+H]^+$.

Step 3: Synthesis of Compound **018-3**

**018-2** (0.1 g, 0.16 mmol) was added to a mixed solution of ethanol (2 mL), tetrahydrofuran (1 mL), and methanol (1 mL), followed by the addition of lithium hydroxide (95.8 mg, 4.0 mmol). The reaction was carried out at 30°C for 24 hours. The reaction mixture was quenched with water (5 mL), added with 2 M hydrochloric acid to adjust the pH to about 5, and extracted with ethyl acetate (5 mL * 3). The organic phases were combined, sequentially washed with saturated brine (5 mL * 3), dried over anhydrous sodium sulfate, and filtered. The filtrate was concentrated under reduced pressure to obtain a crude product. The crude product was purified by thin-layer chromatography (PE: EA = 1:1) to obtain **018-3.** LCMS: m/z = 604.3 $[M+H]^+$.

Step 4: Synthesis of Compound **018-4**

**018-3** (55 mg, 91.12 μmol) was dissolved in dichloromethane (1 mL), then trifluoroacetic acid (0.3 mL) was added, and the reaction mixture was reacted at 20°C for 2 hours. The reaction mixture was evaporated under reduced pressure to remove trifluoroacetic acid to obtain a crude product of **018-4,** which was directly used in the next reaction without purification. LCMS: m/z = 504.3 $[M+H]^+$.

Step 5: Synthesis of Compound **018**

**018-4** (50.0 mg, 99.31 μmol) was dissolved in dichloromethane (0.1 mL), and then *N*,*N*-diisopropylethylamine (0.5 mmol, 86.5 μL) and pentafluorophenyl diphenylphosphinate (57.2 mg, 0.15 mmol) were sequentially added. The reaction mixture was stirred at 20°C for 3 hours. The reaction mixture was added with 5 mL of water and extracted with dichloromethane (10 mL * 3). The organic phases were combined, sequentially washed with saturated brine (20 mL * 3), dried over anhydrous sodium sulfate, filtered, and the filtrate was concentrated under reduced pressure to obtain a crude product. The crude product was purified by thin-layer chromatography (PE: EA = 0:1), and then purified by prep-HPLC (chromatographic column: Waters Xbridge Prep OBD C18 column 150 * 40 mm * 10 μm, mobile phase: phase A:10 mM ammonium bicarbonate aqueous solution, phase B: acetonitrile; gradient: the gradient of phase B increased from 35% to 65% in 8 minutes) to obtain 018. $^1H$ NMR (400 MHz, $CDCl_3$) $\delta$ 9.20 (br s, 1H), 8.03 (s, 1H), 7.03 - 6.92 (m, 2H), 6.89 - 6.81 (m, 1H), 6.19 - 5.85 (m, 2H), 4.72 (br d, *J*= 11.9 Hz, 1H), 4.39 - 4.18 (m, 3H), 3.98 - 3.86 (m, 2H), 2.17 (s, 3H), 1.81 (s, 3H), 1.61 (s, 3H); LCMS: m/z = 486.3 $[M+H]^+$.

**Example 19**

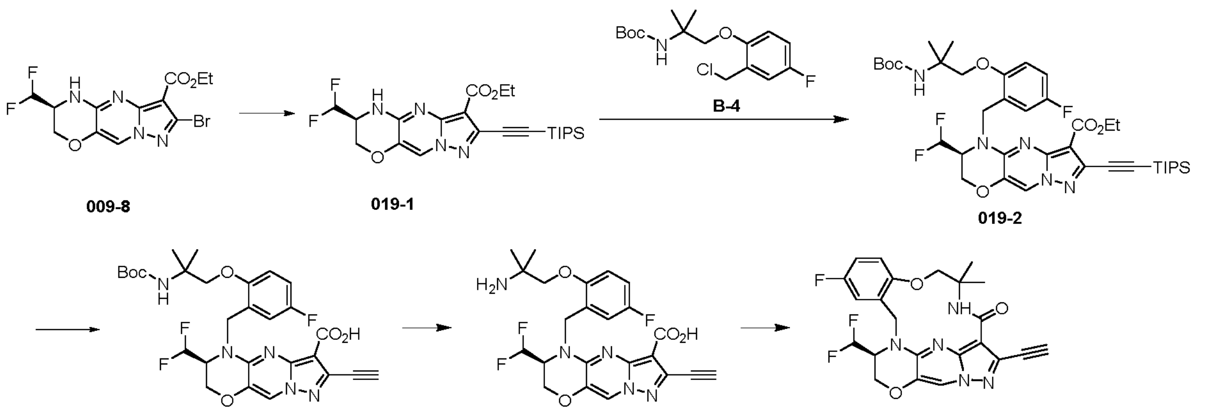


009-8 019-1 019-2 019-3 019-4 019

Step 1: Synthesis of Compound **019-1**

**009-8** (0.3 g, 0.8 mmol) was added to acetonitrile (6.0 mL). After purging three times with nitrogen, [(2-dicyclohexylphosphino-2',4',6'-triisopropyl-1,1'-biphenyl)[2-(2'-amino-1,1'-biphenyl)]methanesulfonato] palladium (67.3 mg, 0.08 mmol) and cesium carbonate (0.52 g, 1.6 mmol) were added. The mixture was slowly heated to 80°C, and then a solution of (triisopropylsilyl)acetylene (535.4 μL, 2.39 mmol) in acetonitrile (6.0 mL) was added. The reaction was carried out for 12 hours. The resulting reaction mixture was evaporated under reduced pressure to remove acetonitrile and purified by column chromatography (petroleum ether: ethyl acetate = 1:0 to 1:1) to obtain **019-1.** LCMS: m/z = 479.3

[M+H]+.

Step 2: Synthesis of Compound **019-2**

**019-1** (0.26 g, 0.54 mmol) and **B-4** (0.22 g, 0.65 mmol) were dissolved in *N,N*-dimethylformamide (2.6 mL), then cesium carbonate (0.53 g, 1.63 mmol) was added, and the reaction mixture was stirred at 20°C for 2 hours. The reaction mixture was added with water (10 mL) to quench the reaction and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (petroleum ether: ethyl acetate = 1:0 to 1:1) to obtain **019-2.** $^1$H NMR (400 MHz, $CDCl_3$) $\delta$ ppm 8.04 (s, 1H), 7.70 - 7.56 (m, 1H), 7.00 - 6.91 (m, 1H), 6.89 - 6.80 (m, 1H), 6.13 - 5.78 (m, 2H), 4.53 - 4.40 (m, 5H), 4.31 - 4.22 (m, 1H), 4.18 (br d, *J*= 9.0 Hz, 1H), 4.00 - 3.91 (m, 1H), 3.86 (br d, *J* = 11.7 Hz, 1H), 1.42 - 1.38 (m, 12H), 1.37 - 1.31 (m, 9H), 1.19 (s, 18H), LCMS: m/z = 774.4 [M+H]+.

Step 3: Synthesis of Compound **019-3**

**019-2** (0.13 g, 0.17 mmol) was added to a solution of ethanol (2.6 mL), tetrahydrofuran (1.3 mL), and methanol (1.3 mL), followed by the addition of a mixed solvent solution of sodium hydroxide (2 M, 2.6 mL). The reaction mixture was stirred at 30°C for 24 hours. After cooling to 0°C, the reaction mixture was quenched with water (5 mL), added with 2 M hydrochloric acid to adjust the pH to about 7, and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by thin-layer chromatography (petroleum ether: ethyl acetate = 1:1) to obtain **019-3.** LCMS: m/z = 590.3 [M+H]+.

Step 4: Synthesis of Compound **019-4**

**019-3** (35.0 mg, 59.4 μmol) was dissolved in dichloromethane (1 mL), then trifluoroacetic acid (0.3 mL) was added, and the reaction mixture was stirred at 20°C for 1 hour. The reaction mixture was evaporated under reduced pressure to remove trifluoroacetic acid to obtain a crude product of **019-4,** which was directly used in the next reaction without purification. LCMS: m/z = 490.3 [M+H]+.

Step 5: Synthesis of Compound **019**

**019-4** (25.0 mg, 51.1 μmol) was dissolved in dichloromethane (0.3 mL), and then pentafluorophenyl diphenylphosphinate (29.4 mg, 76.6 μmol) and *N,N*-diisopropylethylamine (33.0 mg, 255.4 μmol) were sequentially added. The reaction mixture was stirred at 20°C for 3 hours. The reaction mixture was added with water (5 mL) to quench the reaction and extracted with dichloromethane (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by thin-layer chromatography (petroleum ether: ethyl acetate = 1:1), and then purified by prep-HPLC (chromatographic column: Waters Xbridge BEH C18 column 100 * 30 mm * 10 μm, mobile phase: phase A:10 mM ammonium bicarbonate aqueous solution, phase B: acetonitrile; gradient: the gradient of phase B increased from 35% to 65% in 8 minutes) to obtain **019.** $^1$H NMR (400 MHz, $CDCl_3$) $\delta$ 9.19 (br s, 1H), 8.06 (br s, 1H), 7.08 - 6.72 (m, 3H), 6.23 - 5.79 (m, 2H), 4.73 (br d, *J*= 11.3 Hz, 1H), 4.44 - 4.17 (m, 3H), 4.05 - 3.82 (m, 2H), 3.46 (br s, 1H), 1.81 (br s, 3H), 1.62 (br s, 3H); LCMS: m/z = 472.3 [M+H]+.

**Example 20**

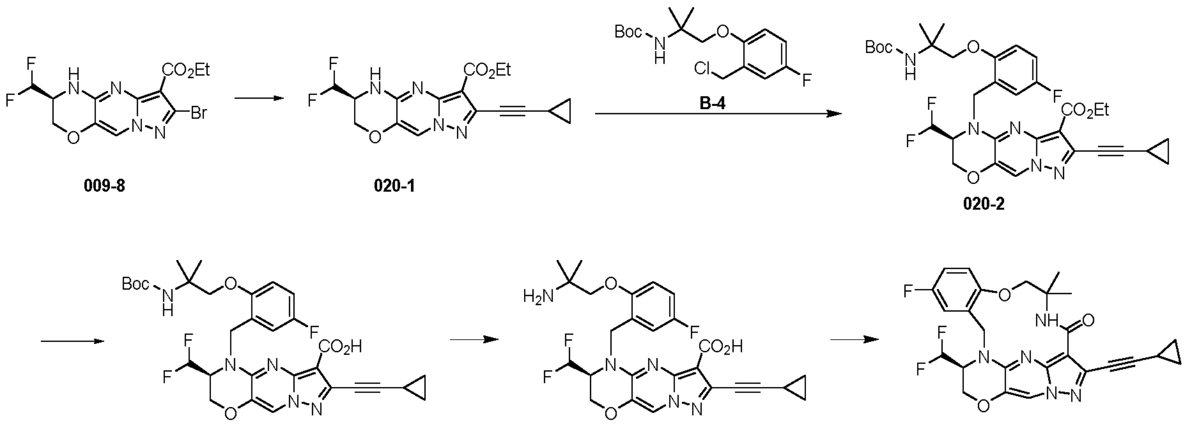

009-8 020-1 B-4 020-2

020-3 020-4 020

Step 1: Synthesis of Compound **020-1**

**009-8** (0.4 g, 1.06 mmol) was added to acetonitrile (4.0 mL). After purging three times with nitrogen, [(2-dicyclohexylphosphino-2',4',6'-triisopropyl-1,1'-biphenyl)[2-(2'-amino-1,1'-biphenyl)]methanesulfonato] palladium (89.8 mg, 0.106 mmol) and cesium carbonate (0.69 g, 2.12 mmol) were added. The mixture was slowly heated to 80°C, and then a solution of cyclopropylacetylene (263.9 μL, 3.18 mmol) in acetonitrile (2.0 mL) was added. The reaction was carried out for 12 hours. The resulting reaction mixture was evaporated under reduced pressure to remove acetonitrile and purified by column chromatography (petroleum ether: ethyl acetate = 1:0 to 1:1) to obtain **020-1.** LCMS: m/z = 363.2 $[M+H]^+$.

Step 2: Synthesis of Compound **020-2**

**020-1** (0.24 g, 0.66 mmol) and **B-4** (0.26 g, 0.79 mmol) were dissolved in *N,N*-dimethylformamide (4.0 mL), then cesium carbonate (0.65 g, 1.99 mmol) was added, and the reaction mixture was stirred at 20°C for 2 hours. The reaction mixture was added with water (10 mL) to quench the reaction and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by thin-layer chromatography (petroleum ether: ethyl acetate = 1:1) to obtain **020-2.** $^1H$ NMR (400 MHz, $CDCl_3$) $\delta$ 7.99 (s, 1H), 7.72 - 7.47 (m, 1H), 7.00 - 6.83 (m, 2H), 6.13 - 5.68 (m, 2H), 4.67 - 4.33 (m, 5H), 4.32 - 4.08 (m, 3H), 4.02 - 3.93 (m, 1H), 3.89 - 3.78 (m, 1H), 1.44 - 1.31 (m, 18H), 1.01 - 0.86 (m, 4H); LCMS: m/z = 658.4 $[M+H]^+$.

Step 3: Synthesis of Compound **020-3**

**020-2** (0.17 g, 0.26 mmol) was added to a solution of ethanol (3.4 mL), tetrahydrofuran (1.7 mL), and methanol (1.7 mL), followed by the addition of a mixed solvent solution of potassium hydroxide (2 M, 3.4 mL) and sodium fluoride (21.7 mg, 0.52 mmol). The reaction mixture was stirred at 30°C for 60 hours. The reaction mixture was quenched with water (10 mL), added with 2 M hydrochloric acid to adjust the pH to 3-4, and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (petroleum ether: ethyl acetate = 1:0 to 1:1) to obtain **020-3.** LCMS: m/z = 630.3 $[M+H]^+$.

Step 4: Synthesis of Compound **020-4**

**020-3** (80.0 mg, 127.1 μmol) was dissolved in dichloromethane (1.6 mL), then trifluoroacetic acid (0.5 mL) was added, and the reaction mixture was stirred at 20°C for 0.5 hours. The reaction mixture was evaporated under reduced pressure to remove trifluoroacetic acid to obtain the crude trifluoroacetate salt of **020-4,** which was directly used in the next reaction without purification. LCMS: m/z = 530.3 $[M+H]^+$.

Step 5: Synthesis of Compound **020**

**020-4** (67.0 mg, crude trifluoroacetate salt) was dissolved in dichloromethane (1.4 mL), and then benzotriazol-1-yloxytris(pyrrolidino)phosphonium hexafluorophosphate (98.8 mg, 189.8 μmol) and *N,N*-diisopropylethylamine (110.2 μL, 632.7 μmol) were sequentially added. The reaction mixture was stirred at 20°C for 2 hours. The reaction mixture was

added with water (5 mL) to quench the reaction and extracted with dichloromethane (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by thin-layer chromatography (petroleum ether: ethyl acetate = 1:1), and then purified by prep-HPLC (chromatographic column: Phenomenex Luna C18 column 75 * 30 mm * 3 μm; mobile phase: phase A: 0.2% formic acid aqueous solution, phase B: acetonitrile, gradient: the gradient of phase B increased from 25% to 60% in 8 minutes) to obtain **020.** $^1$H NMR (400 MHz, $CDCl_3$) $\delta$ 9.18 (s, 1H), 8.01 (s, 1H), 7.04 - 6.93 (m, 2H), 6.85 (br dd, *J* = 4.4, 9.5 Hz, 1H), 6.20 - 5.83 (m, 2H), 4.71 (br d, *J* = 12.0 Hz, 1H), 4.40 - 4.32 (m, 1H), 4.28 (td, *J* = 1.8, 12.0 Hz, 1H), 4.22 (d, *J* = 15.1 Hz, 1H), 3.99 - 3.92 (m, 1H), 3.91 - 3.83 (m, 1H), 1.81 (s, 3H), 1.61 (s, 3H), 1.33 - 1.22 (m, 1H), 0.98 - 0.92 (m, 2H), 0.91 - 0.85 (m, 2H); LCMS: m/z = 512.3 $[M+H]^+$.

**Example 21**

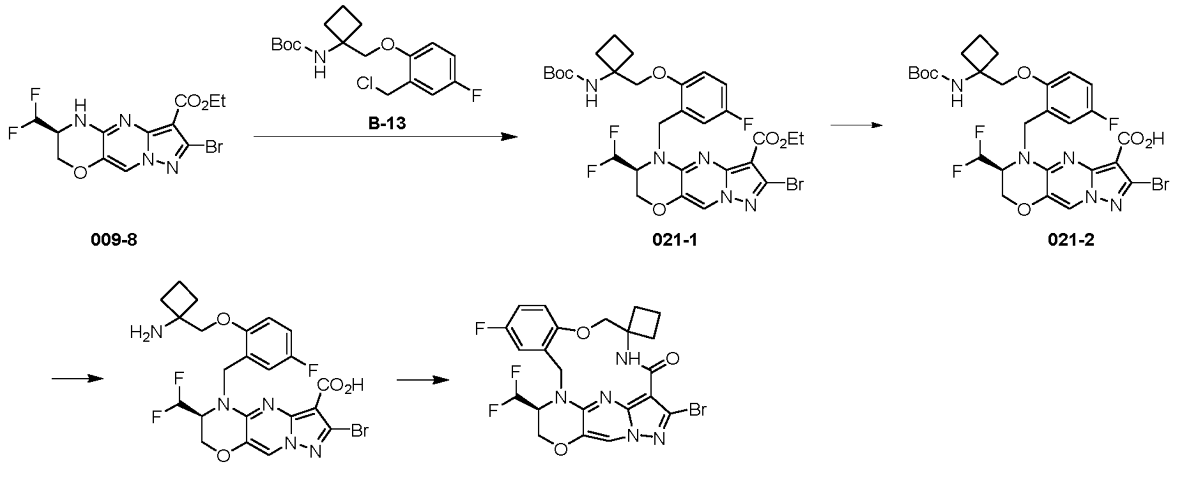

B-13

009-8 021-1 021-2

021-3 021

Step 1: Synthesis of Compound **021-1**

**009-8** (329.0 mg, 0.87 mmol) and **B-13** (299.9 mg, 0.87 mmol) were dissolved in *N,N*-dimethylformamide (10 mL), then cesium carbonate (0.85 g, 2.62 mmol) was added, and the reaction mixture was stirred at 20°C for 4 hours. The reaction mixture was quenched with water (30 mL) and extracted with ethyl acetate (20 mL * 4). The organic phases were combined, sequentially washed with saturated brine (20 mL * 3), dried over anhydrous sodium sulfate, filtered, and the filtrate was concentrated under reduced pressure to obtain a crude product. The crude product was purified by column chromatography (PE: EA = 1:0 to 3:1) to obtain **021-1.** LCMS: m/z = 584.1 $[M+H-Boc]^+$.

Step 2: Synthesis of Compound **021-2**

**021-1** (0.54 g, 0.78 mmol) was added to a mixed solution of methanol (5 mL), tetrahydrofuran (5 mL), and ethanol (10 mL), followed by the addition of a mixed solvent solution of sodium hydroxide (2 M, 10.55 mL). The reaction mixture was stirred at 30°C for 24 hours. The reaction mixture was added with 1 M hydrochloric acid to adjust the pH to 3-4 and extracted with dichloromethane (30 mL * 3). The organic phases were combined, sequentially washed with saturated brine (30 mL), dried over anhydrous sodium sulfate, and filtered. The filtrate was concentrated under reduced pressure to obtain a crude product. The crude product was purified by column chromatography (PE: EA = 10:1 to 1:1) to obtain **021-2.** LCMS: m/z = 556.3 $[M+H-Boc]^+$.

Step 3: Synthesis of Compound **021-3**

**021-2** (320 mg, 487.5 μmol) was dissolved in ethyl acetate (3.5 mL), and then a solution of hydrochloric acid in ethyl acetate (4 M, 0.32 mL) was added, and the reaction mixture was reacted at 20°C for 16 hours. The reaction mixture was evaporated under reduced pressure to remove hydrochloric acid gas to obtain a crude product of **021-3,** which was directly used in the next reaction without purification. LCMS: m/z = 556.0 $[M+H]^+$.

Step 4: Synthesis of Compound **021**

**021-3** (168.0 mg, 283.3 μmol) was dissolved in dichloromethane (8.0 mL), and then *N*,*N*-diisopropylethylamine (1.42 mmol, 246.8 μL) and pentafluorophenyl diphenylphosphinate (163.3 mg, 0.42 mmol) were sequentially added. The reaction mixture was stirred at 20°C for 3 hours. The reaction mixture was added with 20 mL of water and extracted with dichloromethane (10 mL * 3). The organic phases were combined, sequentially washed with saturated brine (20 mL), dried over anhydrous sodium sulfate, filtered, and the filtrate was concentrated under reduced pressure to obtain a crude product. The crude product was purified by column chromatography (PE: EA = 10:1 to 2:1), and then purified by preparative chiral chromatography (chromatographic column: DAICEL CHIRALCEL OJ column 250 * 30 mm * 10 μm; mobile phase: phase A: supercritical carbon dioxide, phase B: 0.1% ammonia water/ethanol solution, gradient: B was 40% isobaric elution) to obtain **021.** $^1$H NMR (400 MHz, $CDCl_3$) $\delta$ 8.97 (s, 1H), 8.03 (s, 1H), 7.02-6.95 (m, 2H), 6.91-6.88 (m, 1H), 6.19-5.92 (m, 1H), 5.85 (dd, *J*= 1.2 Hz, 15.2 Hz, 1H), 4.73 (d, *J*= 11.6 Hz, 1H), 4.49 (d, *J* = 9.2 Hz, 1H), 4.37- 4.35 (m, 1H), 4.30 (dm, *J*= 11.6 Hz, 1H), 4.19 (d, *J*= 15.2 Hz, 1H), 3.98 (d, *J*= 9.2 Hz, 1H), 3.95 - 3.90 (m, 1H), 2.66 - 2.61 (m, 1H), 2.44 - 2.39 (m, 1H), 2.13 - 201 (m, 2H), 1.91 - 1.84 (m, 1H); LCMS: m/z = 538.0 [M+H]$^+$, 540.0 [M+2+H]$^+$.

**Example 22**

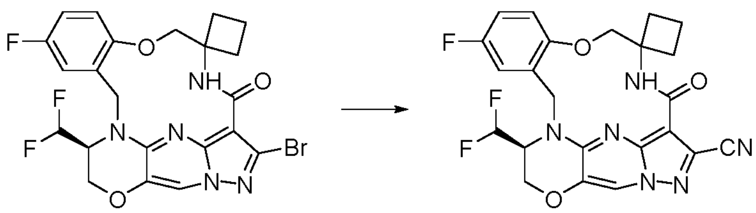

**021** **022**

**021** (50 mg, 92.9 μmol) was dissolved in N-methylpyrrolidone (2.0 mL), and after purging with nitrogen, cuprous cyanide (16.64 mg, 185.8 μmol) and tetrakis(triphenyl)palladium (21.5 mg, 18.6 μmol) were added. The reaction mixture was stirred at 120°C for 16 hours. After cooling to room temperature, the reaction mixture was filtered through diatomite and the filter cake was washed with ethyl acetate (5 mL). The resulting filtrate was added with water (20.0 mL) and extracted with ethyl acetate (5 mL * 3). The organic phases were combined, sequentially washed with saturated brine (10 mL), dried over anhydrous sodium sulfate, filtered, and the filtrate was concentrated under reduced pressure to obtain a crude product. The crude product was purified by thin-layer chromatography (petroleum ether: ethyl acetate = 6:4), and then purified by prep-HPLC (chromatographic column: Xtimate C18 column 150 * 40 mm * 5 μm; mobile phase: phase A: 0.1% hydrochloric acid aqueous solution, phase B: acetonitrile, gradient: the gradient of phase B increased from 43% to 73% in 15 minutes) to obtain **022.** $^1$H NMR (400 MHz, $CDCl_3$) $\delta$ ppm 9.09 (s, 1H), 8.08 (s, 1H), 7.02 - 6.95 (m, 2H), 6.91 - 6.89 (m, 1H), 6.23 - 5.95 (m, 1H), 5.86 - 5.82 (m, 1H), 4.79 - 4.76 (m, 1H), 4.51 - 4.49 (m, 1H), 4.42 - 4.25 (m, 2H), 4.25 - 4.22 (m, 1H), 3.99 - 3.95 (m, 2H), 2.67 - 2.64 (m, 1H), 2.42 - 2.41 (m, 1H), 2.11 - 1.99 (m, 2H), 1.90 - 1.85 (m, 1H); LCMS: m/z =485.3 [M+H]$^+$.

**Example 23**

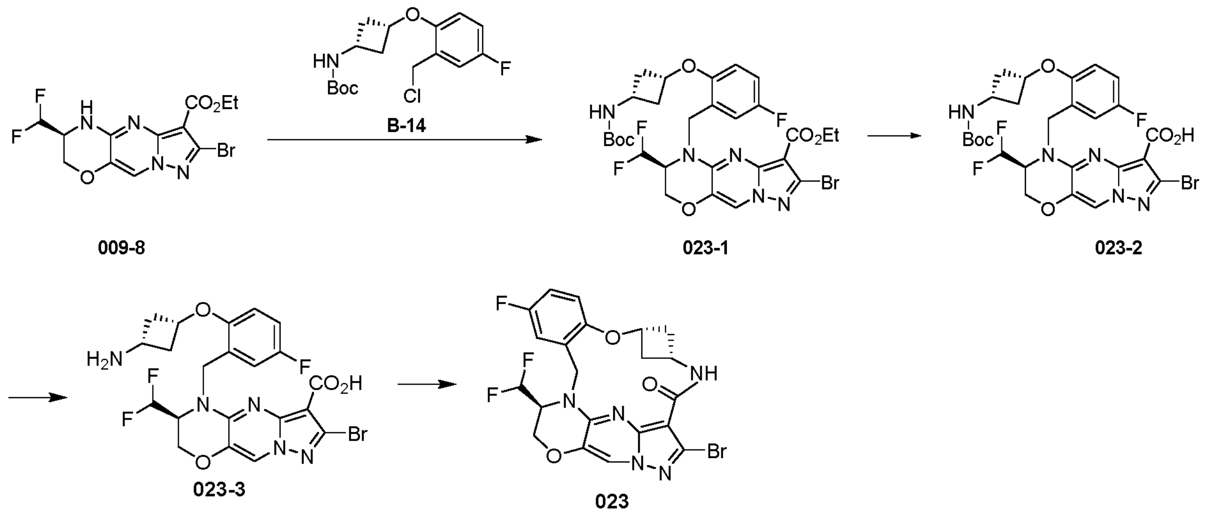

B-14

009-8 023-1 023-2

023-3 023

Step 1: Synthesis of Compound **023-1**

**009-8** (0.3 g, 0.80 mmol) and **B-14** (262.3 mg, 0.80 mmol) were dissolved in *N,N*-dimethylformamide (3.0 mL), then a solution of cesium carbonate (0.78 g, 2.39 mmol) in *N,N'*-dimethylformamide (3.0 mL) was added, and the reaction mixture was stirred at 20°C for 3 hours. The reaction mixture was quenched with water (10 mL) and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (PE: EA = 4:1 to 2:1) to obtain **023-1.** LCMS: m/z = 670.2 $[M+H]^+$, 672.2 $[M+2+H]^+$.

Step 2: Synthesis of Compound **023-2**

**023-1** (0.45 g, 0.67 mmol) was added to a mixed solution of methanol (5 mL), tetrahydrofuran (5 mL), and ethanol (10 mL), followed by the addition of a mixed solvent solution of sodium hydroxide (2 M, 9.06 mL). The reaction mixture was stirred at 20°C for 30 hours. The reaction mixture was added with 2 M hydrochloric acid to adjust the pH to 7-8 and extracted with dichloromethane (20 mL * 3). The organic phases were combined, sequentially washed with saturated brine (10 mL), dried over anhydrous sodium sulfate, and filtered. The filtrate was concentrated under reduced pressure to obtain a crude product. The crude product was purified by column chromatography (PE: EA = 1:0 to 3:1) to obtain **023-2.** LCMS: m/z = 542.0 $[M+H\text{-}Boc]^+$, m/z = 544.0 $[M+H+2\text{-}Boc]^+$.

Step 3: Synthesis of Compound **023-3**

**023-2** (0.23 g, 358.0 μmol) was dissolved in dichloromethane (10.0 mL), then a solution of hydrochloric acid in 1,4-dioxane (4 M, 5.75 mL) was added, and the reaction mixture was reacted at 20°C for 3 hours. The reaction mixture was evaporated under reduced pressure to remove hydrochloric acid gas to obtain a crude product of **023-3,** which was directly used in the next reaction without purification. LCMS: m/z = 542.0 $[M+H]^+$, m/z = 544.0 $[M+2+H]^+$.

Step 4: Synthesis of Compound **023**

**023-3** (209.6 mg, 362.8 μmol) was dissolved in dichloromethane (16.0 mL), and then *N,N*-diisopropylethylamine (1.81 mmol, 316.0 μL) and pentafluorophenyl diphenylphosphinate (209.1 mg, 0.54 mmol) were sequentially added. The reaction mixture was stirred at 20°C for 3 hours. The reaction mixture was added with 20 mL of water and extracted with dichloromethane (20 mL * 2). The organic phases were combined, sequentially washed with saturated brine (20 mL), dried over anhydrous sodium sulfate, filtered, and the filtrate was concentrated under reduced pressure to obtain a crude product. The crude product was purified by column chromatography (PE: EA = 1:0 to 2:1), and then purified by preparative chiral chromatography (chromatographic column: DAICEL CHIRALCEL OD column 250 * 30 mm * 10 μm; mobile phase: phase A: supercritical carbon dioxide, phase B: 0.1% ammonia water-ethanol solution, gradient: phase B was 40%, isobaric elution) to obtain **023.** $^1H$ NMR (400 MHz, $CDCl_3$) $\delta$ 9.03 (d, *J*= 10.4 Hz, 1H), 8.07 (s, 1H), 6.95-6.87 (m, 2H), 6.48 (dd, *J*= 4.8 Hz, *J*= 8.8 Hz, 1H), 6.21-5.92 (m, 2H), 5.00-4.97 (m, 1H), 4.90-4.87 (m, 1H), 4.78-4.75 (m, 1H), 4.41-4.33 (m, 2H), 4.26-4.22 (m, 1H), 3.13-3.07 (m, 1H), 2.85-2.79 (m, 1H), 2.31-2.25 (m, 1H), 1.95-1.90 (m, 1H); LCMS: m/z = 524.0,

526.0 [M+H]+.

**Example 24**

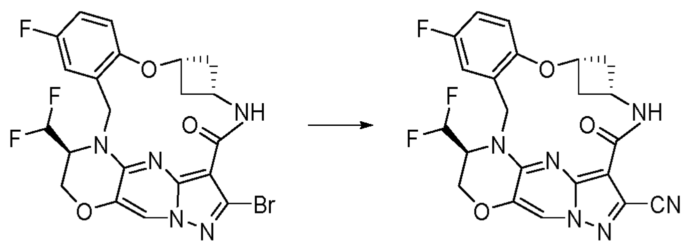

023 024

**023** (85 mg, 162.1 μmol) was dissolved in *N*-methylpyrrolidone (2.0 mL), and after purging with nitrogen, cuprous cyanide (29.04 mg, 324.3 μmol) and tetrakis(triphenyl)palladium (37.5 mg, 32.4 μmol) were added. The reaction mixture was stirred at 125°C for 16 hours. After cooling to room temperature, the reaction mixture was quenched by adding water (10.0 mL) and extracted with ethyl acetate (5 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (petroleum ether: ethyl acetate = 1:0 to 1:1), and then purified by chiral chromatography (chromatographic column: ChiralPak IH column 250 * 30 mm * 10 μm; mobile phase: phase A: supercritical carbon dioxide, phase B: 0.1% ammonia water-ethanol solution, gradient: phase B was 40%, isobaric elution) to obtain **024.** $^1$H NMR (400 MHz, $CDCl_3$) $\delta$ ppm 9.02 (d, *J* = 10.0 Hz, 1H), 8.14 (s, 1H), 6.98 - 6.94 (m, 1H), 6.90 - 6.87 (m, 1H), 6.51 (dd, *J* = 4.0 Hz, *J* = 8.8 Hz, 1H), 6.23 - 5.94 (m, 2H), 4.98 - 4.95 (m, 1H), 4.91 - 4.89 (m, 1H), 4.82 - 4.79 (m, 1H), 4.46 - 4.37 (m, 2H), 4.29 - 4.25 (m, 1H), 3.15 - 3.08 (m, 1H), 2.88 - 2.82 (m, 1H), 2.31 - 2.26 (m, 1H), 1.97 - 1.91 (m, 1H); LCMS: m/z =471.1 [M+H]+.

**Example 25**

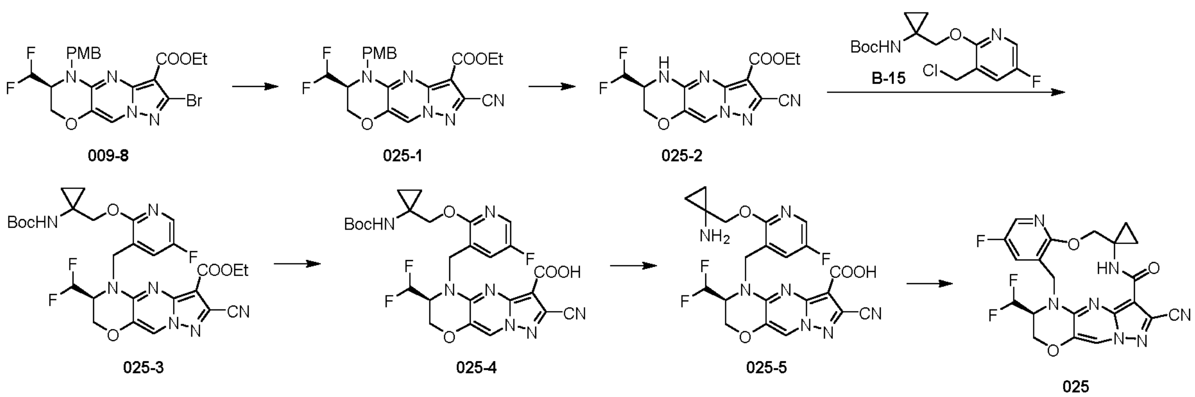


Step 1: Synthesis of Compound **025-1**

**009-8** (1.5 g, 3.02 mmol), cuprous cyanide (540.29 mg, 6.03 mmol), and *N*-methylpyrrolidone (30 mL) were added to a reaction flask, then cuprous iodide (574.46 mg, 3.02 mmol) was added. Under a nitrogen atmosphere, the reaction mixture was heated to 125°C and reacted for 12 hours. The reaction mixture was added with ammonia water (15.0 mL) and stirred for 10 minutes to quench the reaction. Water (15.0 mL) was added, and a solid precipitated. The filter cake obtained from filtration was dissolved in dichloromethane (50 mL), dried over anhydrous sodium sulfate, filtered, and the filtrate was concentrated under reduced pressure to obtain **025-1.** $^1$H NMR (400 MHz, $CDCl_3$) $\delta$ ppm 8.11 (br s, 1H), 7.48 - 7.30 (m, 2H), 6.89 (br d, *J* = 7.5 Hz, 2H), 6.21 - 5.70 (m, 2H), 4.71 - 4.12 (m, 4H), 3.81 (br s, 5H), 1.52 - 1.33 (m, 3H), LCMS: m/z =444.3 [M+H]+.

Step 2: Synthesis of Compound **025-2**

**025-1** (1.2 g, 2.71 mmol) and trifluoroacetic acid (12 mL) were added to a reaction flask, and the reaction mixture was heated to 90°C under a nitrogen atmosphere and reacted for 2 hours. The reaction mixture was concentrated under reduced pressure to remove trifluoroacetic acid, then added with saturated sodium bicarbonate solution to adjust the pH to 7-8, and extracted with dichloromethane (20.0 mL * 3) The organic phases were combined, sequentially washed with saturated brine (20 mL * 3), dried over anhydrous sodium sulfate, filtered, and the filtrate was concentrated under reduced pressure to obtain a crude product. The crude product was purified by column chromatography (PE: EA = 1:0 to 0:1) to obtain **025-2.** $^1$H NMR (400 MHz, $CDCl_3$) $\delta$ ppm 8.16 (s, 1H), 7.16 (br s, 1H), 6.03 - 5.72 (m, 1H), 4.53 (br d,*J*= 12.6 Hz, 1H), 4.42 (q,*J*= 6.9 Hz, 2H), 4.27 (br d,*J*= 12.1 Hz, 1H), 4.10 - 3.96 (m, 1H), 1.44 (t,*J*= 6.8 Hz, 3H), LCMS: m/z =324.2 $[M+H]^+$.

Step 3: Synthesis of Compound **025-3**

**025-2** (0.3 g, 928.06 μmol) was dissolved in *N*,*N*-dimethylformamide (3 mL), cooled to 0°C. Potassium carbonate (0.38 g, 2.78 mmol) was then added, followed by the addition of a solution of **B-15** (0.46 g, 1.39 mmol) in *N*,*N*-dimethylformamide (1 mL). Under a nitrogen atmosphere, the reaction mixture was stirred at 0°C for 1 hour, then slowly warmed to 20°C and stirred for 2 hours. The reaction mixture was quenched with water (6 mL) and extracted with ethyl acetate (6.0 mL * 3). The organic phases were combined, sequentially washed with saturated brine (10 mL * 2), dried over anhydrous sodium sulfate, filtered, and the filtrate was concentrated under reduced pressure to obtain a crude product. The crude product was purified by column chromatography (PE: EA = 1:0 to 1:1) to obtain **025-3.** $^1$H NMR (400 MHz, $CDCl_3$) $\delta$ ppm 8.38 (dd, *J* = 2.9, 8.1 Hz, 1H), 8.05 (s, 1H), 7.87 (d, *J* = 2.7 Hz, 1H), 6.25 - 5.93 (m, 1H), 5.46 - 5.28 (m, 1H), 5.14 - 4.79 (m, 2H), 4.66 (br d,*J* = 11.5 Hz, 1H), 4.58 (d, *J*= 14.2 Hz, 1H), 4.53 - 4.46 (m, 2H), 4.41 - 4.34 (m, 1H), 4.33 - 4.24 (m, 1H), 4.21 - 4.08 (m, 1H), 1.48 (t, *J*= 7.1 Hz, 3H), 1.45 - 1.29 (m, 9H), 1.12 - 1.00 (m, 1H), 0.95 - 0.78 (m, 3H), LCMS: m/z =618.3 $[M+H]^+$.

Step 4: Synthesis of Compound **025-4**

**025-3** (0.5 g, 0.81 mmol) was dissolved in ethanol (10 mL), tetrahydrofuran (5 mL), and methanol (5 mL). After cooling to 0°C, lithium hydroxide monohydrate (2 M, 10 mL) was added. Under a nitrogen atmosphere, the reaction mixture was stirred at 0°C for 1 hour, then warmed to 25°C and reacted for 5 hours. The reaction mixture was quenched with water (10 mL), then added with 2 N hydrochloric acid to adjust the pH to about 4, and extracted with ethyl acetate (10 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (PE: EA = 1:0 to 1:1) to obtain **025-4.** $^1$H NMR (400 MHz, $CDCl_3$) $\delta$ ppm 13.32 - 12.57 (m, 1H), 8.77 (d, *J*= 0.9 Hz, 1H), 8.27 (dd, *J* = 2.7, 8.4 Hz, 1H), 7.96 (d, *J* = 2.2 Hz, 1H), 7.30 (br s, 1H), 6.66 - 6.32 (m, 1H), 5.07 (br d, *J*= 14.6 Hz, 1H), 4.90 - 4.73 (m, 1H), 4.68 (br d, *J*= 12.1 Hz, 1H), 4.51 (br d, *J*= 14.4 Hz, 1H), 4.40 - 4.21 (m, 3H), 1.32 (br s, 9H), 0.84 - 0.67 (m, 4H), LCMS: m/z =590.3 $[M+H]^+$.

Step 5: Synthesis of Compound **025-5**

**025-4** (0.32 g, 0.54 mmol) was dissolved in dichloromethane (3.0 mL), cooled to 0°C, then trifluoroacetic acid (1 mL) was added. The reaction mixture was slowly warmed to 20°C and stirred for 1 hour. The reaction mixture was evaporated under reduced pressure to remove trifluoroacetic acid to obtain a crude product of **025-5,** which was directly used in the next reaction without purification. LCMS: m/z =490.2 $[M+H]^+$.

Step 6: Synthesis of Compound **025**

**025-5** (265 mg, 0.54 mmol) was dissolved in dichloromethane (2.6 mL) and cooled to 0°C under a nitrogen atmosphere. Then benzotriazol-1-yloxytris(pyrrolidino)phosphonium hexafluorophosphate (0.42 g, 0.81 mmol) and N,N-diisopropylethylamine (0.47 mL, 2.71 mmol) were added, and the reaction mixture was slowly warmed to 20°C and stirred for 1.0 hour. The reaction mixture was quenched with water (10.0 mL) and extracted with dichloromethane (20.0 mL * 3). The organic phases were combined, sequentially washed with saturated brine (20 mL), dried over anhydrous sodium sulfate, filtered, and the filtrate was concentrated under reduced pressure to obtain a crude product. The crude product was purified by column chromatography (PE: EA = 1:0 to 1:1) and then further purified by preparative chromatography (column model: Waters Xbridge Prep OBD C18 150 * 40 mm * 10 μm; mobile phase A: 10 mM ammonium bicarbonate aqueous solution, mobile phase B: acetonitrile; gradient: phase B from 25% to 55% in 8.0 minutes). The resulting target eluent was concentrated under reduced pressure and then freeze-dried to obtain **025.** SFC chiral analysis and detection(chromatographic column: Chiralcel OD-3, 50 × 4.6 mm I.D., 3 μm; mobile phase A: supercritical carbon

dioxide, mobile phase B: isopropanol [0.2% ammonia methanol solution (7M)], gradient: phase B increased from 5% to 50% in the first 1.2 minutes, maintained for 1 minute, and then phase B decreased from 50% to 5% in the next 0.8 minutes), showed ee = 100% (Rt = 1.609 minutes). $^1H$ NMR (400 MHz, $CDCl_3$) $\delta$ ppm 8.94 (s, 1H), 8.79 (s, 1H), 8.06 (br s, 1H), 7.99 (br d, *J*= 8.3 Hz, 1H), 6.68 - 6.33 (m, 1H), 5.47 (br d, *J*= 14.8 Hz, 1H), 5.05 (br t, *J*= 10.1 Hz, 1H), 4.91 (br d, *J*= 10.9 Hz, 1H), 4.72 (br d, *J*= 12.4 Hz, 1H), 4.52 - 4.38 (m, 2H), 3.74 (br d, *J*= 10.9 Hz, 1H), 1.96 - 1.80 (m, 1H), 1.06 - 0.96 (m, 2H), 0.89 - 0.77 (m, 1H), LCMS: m/z =472.2 $[M+H]^+$.

**Example 26**

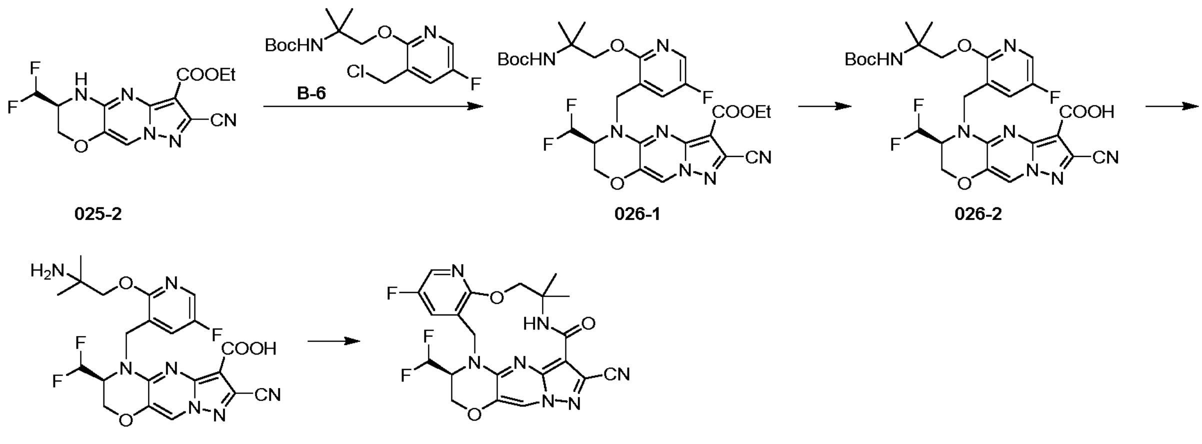


025-2 026-1 026-2

026-3 026

Step 1: Synthesis of Compound **026-1**

**025-2** (0.3 g, 0.93 mmol) was dissolved in *N*,*N*-dimethylformamide (3 mL), cooled to 0°C. Potassium carbonate (0.38 g, 2.78 mmol) was then added, followed by the addition of a solution of **B-6** (463.28 mg, 1.39 mmol) in *N*,*N*-dimethylformamide (1 mL). Under a nitrogen atmosphere, the reaction mixture was stirred at 0°C for 1 hour, then slowly warmed to 20°C and stirred for another 2 hours. The reaction mixture was quenched with water (6 mL) and extracted with ethyl acetate (6 mL * 3). The organic phases were combined, sequentially washed with saturated brine (10 mL * 2), dried over anhydrous sodium sulfate, filtered, and the filtrate was concentrated under reduced pressure to obtain a crude product. The crude product was purified by column chromatography (mobile phase PE: EA = 1:0 to 1:1) to obtain **026-1.** $^1H$ NMR (400 MHz, $CDCl_3$) $\delta$ ppm 8.37 (br d, *J*= 5.4 Hz, 1H), 8.07 (s, 1H), 7.92 (d, *J*= 3.0 Hz, 1H), 6.18 - 5.85 (m, 1H), 5.47 (d, *J*= 14.4 Hz, 1H), 4.68 - 4.45 (m, 7H), 4.34 - 4.28 (m, 1H), 4.00 (br d, *J*= 11.4 Hz, 1H), 1.50 - 1.45 (m, 3H), 1.43 (s, 9H), 1.40 (br s, 3H), 1.39 (s, 3H), LCMS: m/z =620.3 $[M+H]^+$.

Step 2: Synthesis of Compound **026-2**

**026-1** (0.44 g, 0.71 mmol) was dissolved in ethanol (8 mL), tetrahydrofuran (4 mL), and methanol (4 mL). After cooling to 0°C, lithium hydroxide monohydrate (2 M, 8.80 mL) was added. Under a nitrogen atmosphere, the reaction mixture was reacted at 0°C for 1 hour, then slowly warmed to 25°C and reacted for 5 hours. The reaction mixture was quenched with water (10 mL), then added with 2 N hydrochloric acid to adjust the pH to about 4, and extracted with ethyl acetate (5 mL * 3). The organic phases were combined, sequentially washed with saturated brine (10 mL), dried over anhydrous sodium sulfate, filtered, and the filtrate was concentrated under reduced pressure to obtain a crude product. The crude product was purified by column chromatography (mobile phase PE: EA = 1:0 to 1:1) to obtain **026-2.** $^1H$ NMR (400 MHz, $CDCl_3$) $\delta$ ppm 13.14 - 12.54 (m, 1H), 8.81 (s, 1H), 8.23 (br d, *J*= 8.4 Hz, 1H), 8.03 (br s, 1H), 6.68 (br dd, *J*= 1.1, 9.8 Hz, 1H), 6.63 - 6.34 (m, 1H), 5.23 (br d, *J*= 15.0 Hz, 1H), 4.61 (br d, *J*= 12.0 Hz, 1H), 4.52 (br d,*J*= 14.8 Hz, 2H), 4.40 - 4.33 (m, 1H), 4.26 (br d, *J*= 10.6 Hz, 2H), 1.36 (s, 9H), 1.31 (s, 3H), 1.25 (s, 3H), LCMS: m/z =592.3 $[M+H]^+$.

Step 3: Synthesis of Compound **026-3**

**026-2** (0.27 g, 0.46 mmol) was dissolved in dichloromethane (3 mL), then cooled to 0°C under a nitrogen atmosphere, and trifluoroacetic acid (1 mL) was added. The reaction mixture was stirred at 0°C for 0.2 hours, warmed to 20°C, and then reacted for 0.8 hours. The reaction mixture was evaporated under reduced pressure to remove

dichloromethane and trifluoroacetic acid to obtain a crude product of **026-3.** LCMS: m/z =492.3 $[M+H]^+$.

Step 4: Synthesis of Compound **026**

**026-3** (224 mg, 0.46 mmol) was dissolved in dichloromethane (2.3 mL) and cooled to 0°C under a nitrogen atmosphere. Then benzotriazol-1-yloxytris(pyrrolidino)phosphonium hexafluorophosphate (0.36 g, 0.68 mmol) and *N*,*N*-diisopropylethylamine (0.40 mL, 2.28 mmol) were added, and the reaction mixture was slowly warmed to 20°C and stirred for 1.0 hour. The reaction mixture was quenched with water (10.0 mL) and extracted with dichloromethane (20.0 mL * 3). The organic phases were combined, sequentially washed with saturated brine (20 mL), dried over anhydrous sodium sulfate, filtered, and the filtrate was concentrated under reduced pressure to obtain a crude product. The crude product was purified by column chromatography (PE: EA = 1:0 to 1:1) and then further purified by preparative chromatography (column model: Waters Xbridge Prep OBD C18 150 * 40 mm * 10 μm; mobile phase A: 10 mM ammonium bicarbonate aqueous solution, mobile phase B: acetonitrile; gradient: phase B from 30% to 60% in 8.0 minutes). The resulting target eluent was concentrated under reduced pressure and then freeze-dried to obtain **026.** Chiral analysis detection (chromatographic column: (S,S)-WHELK-O1, 50 × 4.6 mm I.D., 3.5 μm; mobile phase A: supercritical carbon dioxide, mobile phase B: ethanol [0.2% ammonia methanol solution (7M)], gradient: phase B increased from 5% to 50% in the first 1.2 minutes, maintained for 1 minute, and then phase B decreased from 50% to 5% in the next 0.8 minutes) showed that compound **26** had an ee of 93.86%, Rt = 2.146 minutes, while its enantiomer had a Rt of 1.971 minutes. $^1H$ NMR (400 MHz, $CDCl_3$) $\delta$ ppm 8.91 (s, 1H), 8.80 (s, 1H), 8.11 (brs, 1H), 8.07 (brd, *J* = 8.8 Hz, 1H), 6.35-6.67 (m, 1H), 5.39 (br d, *J*= 14.8 Hz, 1H), 5.00-5.15 (m, 1H), 4.66-4.80 (m, 2H), 4.43-4.55 (m, 2H), 3.94 (br d, *J*= 10.8 Hz, 1H), 1.64 (s, 3H), 1.52 (s, 3H); LCMS: m/z =474.2 $[M+H]^+$.

**Example 27**

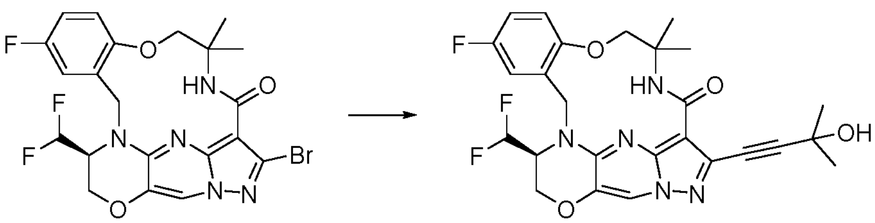

**009** **027**

**009** (20 mg, 38.0 μmol) and 2-methyl-3-butyn-2-ol (37.1 μL, 380.1 μmol) were dissolved in 1,4-dioxane (0.1 mL). Under a nitrogen atmosphere, a pre-prepared solution of bis(acetonitrile)dichloropalladium(II) (0.3 mg, 1.14 μmol), cuprous iodide (0.15 mg, 0.76 μmol), tri-*tert*-butylphosphine (4.61 mg, 2.28 μmol), and I-diisopropylethylamine (6.4 μL, 45.6 μmol) in 1,4-dioxane (0.2 mL) was added. The reaction mixture was heated to 96°C and reacted for 12 hours. After cooling to room temperature, additional 2-methyl-3-butyn-2-ol (37.1 μL, 380.1 μmol) and the pre-prepared solution of bis(acetonitrile)dichloropalladium(II) (0.3 mg, 1.14 μmol), cuprous iodide (0.15 mg, 0.76 μmol), tri-*tert*-butylphosphine (4.61 mg, 2.28 μmol), and I-diisopropylethylamine (6.4 μL, 45.6 μmol) in 1,4-dioxane (0.2 mL) were added. The reaction mixture was heated to 96°C and reacted for another 12 hours. After cooling to room temperature, the reaction mixture was quenched by adding 10 mL of water and extracted with ethyl acetate (20 mL * 3). The organic phases were combined, sequentially washed with saturated brine (20 mL), dried over anhydrous sodium sulfate, filtered, and the filtrate was concentrated under reduced pressure to obtain a crude product. The crude product was purified by preparative thin-layer chromatography (developing solvent: petroleum ether: ethyl acetate = 1:1), and then purified by preparative chromatography (chromatographic column: Waters Xbridge BEH C18 100 * 30 mm * 10 μm; mobile phase: phase A: 10 mM ammonium bicarbonate aqueous solution, phase B: acetonitrile; the gradient of phase B increased from 35% to 65% in 8 minutes). The resulting target eluent was concentrated under reduced pressure and then further purified by thin-layer chromatography (developing solvent: petroleum ether: ethyl acetate = 1:1) to obtain **027.** Chiral analysis detection (chromatographic column: Chiralcel OD-3, 150 × 4.6 mm I.D., 3 μm; mobile phase A: supercritical carbon dioxide, mobile phase B: methanol [0.2% $NH_3$/MeOH (7 M) ], gradient: phase B increased from 0% to 50% in the first 3.5 minutes, maintained for 1 minute, and then decreased to 10% in the next 0.5 minutes) showed ee = 98.9%, Rt = 3.491 minutes, while its enantiomer had a Rt of 3.079 minutes. $^1H$ NMR (400 MHz, $CDCl_3$) $\delta$ ppm 9.22 (s, 1H), 8.04 (s, 1H), 7.03 - 6.96 (m, 2H), 6.85 (br dd, *J* = 4.5, 9.6 Hz, 1H), 6.21 - 5.86 (m, 2H), 4.72 (br d, *J* = 12.0 Hz, 1H), 4.42 - 4.33 (m, 1H), 4.32 - 4.26 (m, 1H), 4.23 (d, *J* = 15.1 Hz, 1H), 3.99 - 3.93 (m, 1H), 3.89 - 3.84 (m, 1H), 1.78 (s, 3H), 1.66 (br s, 3H), 1.65 (br s, 6H), LCMS: m/z =530.3

$[M+H]^+$.

**Bioassay data**

**Experimental example 1: *In vitro* kinase inhibitory activity assay**

The kinase inhibitory activity assays of the compounds against ALK, $ALK^{L1196M}$, $ALK^{G1202R}$, $ALK^{G1202R/L1196M}$, ROS1, and TRKB were conducted at Wuhan Heyan Biotechnology Co., Ltd. or Hefei PreceDo Biotechnology Co., Ltd.

**Experimental method 1:**

Experimental materials: ALK Active, ALK(L1196M) Active, ALK(G1202R) Active, and ALK(G1202R/L1196M) Active were purchased from Carna; SRC Substrate was purchased from SignalChem; IGF1R tide was purchased from SignalChem; ADP-Glo Kinase Assay was purchased from Promega; Kinase assay buffer III was purchased from SignalChem; and Nivo multimode plate reader (PerkinElmer).

ALK kinase inhibition assay method: The test compound was diluted to 1 mM with 100% DMSO as the first concentration, and then 5-fold diluted to the 8th concentration with a pipette, that is, diluted from 1 mM to 0.0128 μM. Each concentration of the compounds was further diluted 20-fold using 1X kinase buffer to prepare compound working solutions containing 5% DMSO. 1 μL of each concentration gradient working solution was added to a microplate, with duplicate wells set for each concentration. 2 μL of ALK enzyme (15 ng per well) and 2 μL of a mixture of substrate and ATP (25 μM ATP, 0.5 μg/μL SRC Substrate) were added to the microplate. At this time, the final concentration gradient of the compound was diluted from 10 μM to 0.128 nM, and the final concentrations of ATP and substrate were 10 μM and 0.2 μg/μL. The reaction system was placed at 25°C for 1 hour. After the reaction, 5 μL of ADP-Glo reagent was added to each well and reacted at 25°C for another 40 minutes. After the reaction, 10 μL of kinase detection reagent was added to each well and reacted at 25°C for 30 minutes. Chemiluminescence was then measured using the PerkinElmer Nivo multimode plate reader with an integration time of 0.5 seconds.

ALK (L1196M) kinase inhibition assay method: The preparation method for the working solution of the test compound was the same as for the ALK kinase. 2 μL of ALK (L1196M) enzyme (4 ng per well) and 2 μL of a mixture of substrate and ATP (125 μM ATP, 0.5 μg/μL IGF1R tide) were added to the microplate. At this time, the final concentration gradient of the compound was diluted from 10 μM to 0.128 nM, and the final concentrations of ATP and substrate were 50 μM and 0.2 μg/μL. The subsequent steps were the same as for ALK kinase.

ALK (G1202R) kinase inhibition assay method: The preparation method for the working solution of the test compound was the same as for the ALK kinase. 2 μL of ALK (G1202R) enzyme (15 ng per well) and 2 μL of a mixture of substrate and ATP (125 μM ATP, 0.5 μg/μL SRC Substrate) were added to the microplate. At this time, the final concentration gradient of the compound was diluted from 10 μM to 0.128 nM, and the final concentrations of ATP and substrate were 50 μM and 0.2 μg/μL. The subsequent steps were the same as for ALK kinase.

ALK (G1202R/L1196M) kinase inhibition assay method: The preparation method for the working solution of the test compound was the same as for the ALK kinase. 2 μL of ALK (G1202R/L1196M) enzyme (5 ng per well) and 2 μL of a mixture of substrate and ATP (125 μM ATP, 0.5 μg/μL SRC Substrate) were added to the microplate. At this time, the final concentration gradient of the compound was diluted from 10 μM to 0.128 nM, and the final concentrations of ATP and substrate were 50 μM and 0.2 μg/μL. The subsequent steps were the same as for ALK kinase.

ROS1 kinase inhibition assay method: The preparation method for the working solution of the test compound was the same as for the ALK kinase. 2 μL of ROS1 (3.0 ng per well) and 2 μL of a mixture of substrate and ATP (50 μM ATP, 0.2 μg/μL IGF1R tide substrate) were added to the microplate. At this time, the final concentration gradient of the compound was diluted from 10 μM to 0.128 nM, and the final concentrations of ATP and substrate were 0.25 μM and 0.2 μg/μL. The subsequent steps were the same as for ALK kinase.

TRKB kinase inhibition assay method: The preparation method for the working solution of the test compound was the same as for the ALK kinase. 2 μL of TRKB (0.5 ng per well) and 4 μL of a mixture of substrate and ATP (5 μM ATP, 0.5 μM SRC Substrate) were added to the microplate. At this time, the final concentration gradient of the compound was diluted from 10 μM to 0.128 nM, and the final concentrations of ATP and substrate were 0.25 μM and 0.2 μg/μL. The subsequent steps were the same as for ALK kinase.

Raw data was converted into inhibition rate using the equation: (Sample - Min) / (Max - Min) * 100%. The $IC_{50}$ value could then be derived by performing a curve fit using a four-parameter logistic model (obtained using the "log(inhibitor) vs. response -- Variable slope" mode in GraphPad Prism). The assay results for compounds **001 to 005** are shown in Table 1-1:

Table 1-1: Kinase half maximal inhibitory concentration $IC_{50}$ (nM)

| Compound | $ALK^{G1202R/L1196M}$ | TRKB | ALK | $ALK^{L1196M}$ | $ALK^{G1202R}$ | ROS1 |
|---|---|---|---|---|---|---|
| **001** | 12.4 | 2.2 | 22.2 | 12.8 | 16.9 | 17.3 |
| **002** | 14.6 | / | / | / | 27.8 | / |
| **003** | 11.3 | / | / | / | 12.2 | / |
| **004** | 1.3 | 2.4 | / | / | / | / |
| **005** | 5.2 | 1.2 | / | / | 3.8 | 0.6 |

**Experimental method 2:**

Experimental materials: ALK (G1202R/L1196M) Active and TRKB were purchased from Carna; IGF1 was purchased from GenScript; PolyE4Y1 was purchased from SIGMA; ADP-Glo™ Reagent was purchased from Promega; ATP was purchased from Promega; Kinase Detection Reagent was purchased from Promega; microplate reader ( PerkinElmer).

Reaction system: ALK(G1202R/L1196M) reaction concentration 8.63 nM, ATP concentration 5.0 μM, substrate IGF1 concentration 0.2 μg/μL. ALK(G1202R/L1196M) kinase buffer was 40 mM Tris-HCl (pH 7.5) + 20 mM magnesium chloride + 0.1 mg/mL BSA + 50 μM DTT.

TRKB reaction concentration 4.47 nM, ATP concentration 5.0 μM, substrate PolyE4Y1 concentration 0.2 μg/μL. TRKB kinase buffer was 40 mM Tris-HCl (pH 7.5) + 20 mM magnesium chloride + 2.5 mM manganese chloride + 0.1mg/mL BSA+ 50 μM DTT.

ALK(G1202R/L1196M) and TRKB detection method: 2 μL of kinase and 1 μL of compound were added to a 384-well plate; 2 μL of substrate/ATP mixture was added and incubated at room temperature in the dark for 60 minutes; 5 μL of ADP-Glo™ reagent was added and incubated at room temperature for 40 minutes; 10 μL of kinase detection reagent was added and incubated at room temperature for 30 minutes; the plate was read on a microplate reader, and luminescence was recorded (integration time set to 0.5 seconds).

Raw data was converted into inhibition rate using the equation: (Sample - Min) / (Max - Min) * 100%. The $IC_{50}$ value could then be derived by performing a curve fit using a four-parameter logistic model (obtained using the "log(inhibitor) vs. response -- Variable slope" mode in GraphPad Prism). The assay results for compounds **006 to 024** are shown in Table 1-2:

Table 1-2: Kinase half maximal inhibitory concentration $IC_{50}$ (nM)

| Compound | $ALK^{G1202R/L1196M}$ | TRKB | Compound | $ALK^{G1202R/L1196M}$ | TRKB |
|---|---|---|---|---|---|
| **006** | 0.6 | 1.2 | **016** | 0.9 | 0.3 |
| **007** | 1.0 | 2.6 | **017** | 15.3 | 31.3 |
| **008** | 1.7 | 7.8 | **018** | 0.7 | 4.9 |
| **009** | 0.8 | 2.0 | **019** | 0.9 | 2.5 |
| **010** | 1.5 | 4.4 | **020** | 1.1 | 10.0 |
| **011** | 1.7 | 3.4 | **021** | 4.0 | 7.0 |
| **012** | 1.5 | 2.9 | **022** | 7.8 | 24.7 |
| **014** | 0.4 | 0.7 | **023** | 1.2 | 1.5 |
| **015** | 0.6 | 1.1 | **024** | 2.3 | 5.3 |

Conclusion: The compounds of the present disclosure show high kinase inhibitory activity against ALK kinase and its mutants, ROS1, and TRKB kinases. Among them, the compounds exhibit more efficient inhibition of ALK kinase and its mutants, while the inhibition of TRKB is relatively weaker, showing better selectivity.

**Experimental example 2: Cell permeability test**

Experimental purpose:

The permeability and efflux ratio of the test compounds were evaluated using the MDCKII-MDR1 monolayer cell test system to determine the potential of the compounds to cross the blood-brain barrier and to be effluxed by the P-gp transporter.

Experimental methods:

MDCKII-MDR1 cells (from the Netherlands Cancer Institute) were seeded into a 96-well cell plate (from Corning) at a cell density of $2.5 \times 10^5$ cells/mL and cultured for 7 days to form a confluent monolayer. Hank's balanced salt buffer (pH $7.40 \pm 0.05$) containing 10 mM 4-(2-hydroxyethyl)-1-piperazineethanesulfonic acid was used as a transport buffer (for compound **005,** the transport buffer was further supplemented with 1% BSA (bovine serum albumin). The bidirectional transport of the test compounds at a concentration of 2 $\mu$M was tested, with the concentration of DMSO in the incubation system controlled below 1%. After adding the samples, the cell plates were incubated at $37 \pm 1$°C, 5% $CO_2$, and saturated humidity for 150 minutes. All samples were quantitatively analyzed using the LC-MS/MS method. The apparent permeability coefficient ($P_{app}$, cm/s), efflux ratio (ER), and recovery rate were calculated using the following formulas.

The apparent permeability coefficient ($P_{app}$, cm/s) was calculated using the following formula: $P_{app} = (dC_r / d_t) \times V_r / (A \times C_0)$. Herein, $dC_r/d_t$ is the cumulative concentration of the compound at the receiving end per unit time ($\mu$M/s); $V_r$ is the volume of the solution at the receiving end (the volumes of the solution at the apical and basal sides are 0.075 mL and 0.250 mL, respectively); A is the relative surface area of the cell monolayer (0.0804 $cm^2$); $C_0$ is the initial concentration (nM) of the test compound at the dosing end or the peak area ratio of the control compound. The efflux ratio was calculated using the following formula: $ER = P_{app}(BA) / P_{app}(AB)$. The recovery rate was calculated using the following formula: $\% \text{ Recovery} = 100 \times [(V_r \times C_r)+(V_d \times C_d)] / (V_d \times C_0)$. Herein, $C_0$ is the initial concentration of the test compound at the dosing end (nM) or the peak area ratio of the control compound; $V_d$ is the volume at the dosing end (0.075 mL for the apical side and 0.250 mL for the basal side); $C_d$ and $C_r$ are the final concentrations (nM) of the test compound at the dosing end and the receiving end or the peak area ratios of the control compound, respectively.

The test results are shown in Table 2:

Table 2: MDCKII-MDR1 test results

| Compound | $10^{-6}$ cm/s A to B | $10^{-6}$ cm/s B to A | Efflux Ratio |
|---|---|---|---|
| **001** | 5.07 | 2.83 | 0.56 |
| **004** | 4.04 | 6.19 | 1.53 |
| **005** | 8.29 | 7.43 | 0.90 |
| **006** | 17.26 | 14.97 | 0.87 |
| **007** | 6.01 | 4.87 | 0.81 |
| **010** | 4.37 | 5.18 | 1.19 |
| **013** | 11.8 | 9.02 | 0.77 |
| **015** | 10.2 | 27.1 | 2.67 |
| **020** | 3.83 | 3.23 | 0.84 |

Conclusion: The compounds of the present disclosure are hypertonic and low efflux compounds.

**Experimental example 3: Anti-proliferative activity test on three Ba/F3 cell lines Ba/F3-EML4-ALK-G1202R, Ba/F3-EML4-ALK-G1202R-L1198F, and Ba/F3-EML4-ALK-G1202R-L1196M**

Experimental materials

(1) Cell Lines (constructed by Hefei PreceDo Biopharmaceutical Technology Co., Ltd.)
$ALK^{G1202R/L1196M}$ cell line: Ba/F3-EML4-ALK-G1202R/L1196M; TRKB cell line: Ba/F3-TEL-TRKB.
(2) Reagents

RPMI 1640: Brand VivaCell, Catalog No. C3010-0500;
Fetal Bovine Serum: Brand VivaCell, Catalog No. C04001-500;
Penicillin-Streptomycin Solution: Brand Gibco, Catalog No. 15140-122;
CellTiterGlo: Brand Promega, Catalog No. G7573;
Trypan Blue: Brand Solarbio, Catalog No. C0040;
96-well White Plate: Brand Biosharp, Catalog No. BS-MP-96W;
96-well Drug Plate: Brand Beaver, Catalog No. 40196.

(3) Instruments
Cell counter, Manufacturer: Count star, Model: IC1000; Microplate reader, Manufacturer: Molecular Devices, Model: SpectraMax Paradigm; $CO_2$ Incubator, Manufacturer: Thermo, Model: HERA cell vios 160i.
(4) Compound Information: Compounds were prepared using DMSO to a working concentration of 1 mM.

Experimental method

(1) Cell culture: The cell lines were cultured in an incubator at 37°C with 5% $CO_2$. Regular passaging was performed, and cells in the logarithmic growth phase were selected for plating;
(2) Preparation of compound storage plate: The test compounds were prepared into a 10 mM solution with DMSO (Stock Con.); The compounds were then prepared into a 1 mM solution with DMSO (Work Con.); 1 μL of the stock solution was mixed with 9 μL of DMSO to prepare a 10 μL work solution.
(3) Preparation of 1000× compound storage plate (tube): The compounds were 3-fold serially diluted from 1 μM to the lowest concentration, for a total of 8 concentrations;
(4) Preparation of 20× compound working solution: 98 μL of cell culture medium was added to a flat-bottomed 96-well transparent drug plate. From the 1000× compound storage plate, 2 μL of the compound was pipetted into the cell culture medium in the 96-well transparent drug plate. In the solvent control, 2 μL of DMSO was added. After the compound or DMSO was added, the mixture was homogenized using a pipette.
(5) Cells were stained with trypan blue and viable cells were counted to ensure a cell viability of 90% or more. The cell concentration was adjusted to an appropriate concentration;
(6) 95 μL of cell suspension (2000 cells/well) was added to each well of the compound assay cell plate, and cell-free culture medium containing 0.1% DMSO was added to the Min control wells;
(7) Administration in the compound assay cell plate: 5 μL of 20× compound working solution was added to the cell culture plate, 5 μL of DMSO-cell culture medium mixture was added to the Max control wells, and the final concentration of DMSO was 0.1%;
(8) The culture plate was then incubated in an incubator at 37°C with 5% $CO_2$ for 72 hours;
(9) The following operations were conducted in accordance with the instructions of the Promega CellTiter-Glo Luminescent Cell Viability Assay Kit (Promega-G7573): CellTiter-Glo buffer was thawed and brought to room temperature; CellTiter-Glo substrate was brought to room temperature; CellTiter-Glo buffer was added to a bottle of CellTiter-Glo substrate to dissolve the substrate, thereby preparing CellTiter-Glo working solution; The mixture was slowly vortexed and shaken to be fully dissolved; The cell culture plate was removed and allowed to equilibrate to room temperature for 10 minutes; 50 μL (equal to half the volume of cell culture medium in each well) of CellTiter-Glo working solution was added to each well; The culture plate was shaken on an orbital shaker for 2 minutes to induce cell lysis; The plate was then left at room temperature for 10 minutes to stabilize the luminescent signal; The luminescent signal was detected using a SpectraMax Paradigm plate reader; Cell viability assay was conducted using CellTiter-Glo luminescence method.

Data analysis: The corresponding fluorescence value (RLU) for each well was obtained from SpectraMax Paradigm readings. The cell proliferation inhibition rate (Inhibition Rate) data were processed using the formula: Inhibition Rate (Inh%) = 100 - ($RLU_{Drug}$ - $RLU_{Min}$) / ($RLU_{Max}$ - $RLU_{Min}$) * 100%. The inhibition rates corresponding to compounds at different concentrations were calculated in Excel, and the inhibition rate curves were plotted using GraphPad Prism software to calculate relevant parameters, including the maximum and minimum inhibition rates of the cells and the $IC_{50}$ value.
The experimental results are shown in Table 3.

Table 3: Half maximal inhibitory concentration $IC_{50}$ (nM) for cells

| Cancer cell phenotype/ Compound No. | Ba/F3-EML4-ALK-G1202R/L1196 M | Ba/F3-TEL-TRKB | Ba/F3-EML4-ALK-G1202R | Ba/F3-EML4-ALK-G1202R/L1198 F |
|---|---|---|---|---|
| **001** | 280.2 | 1.0 | 306.7 | 60.3 |
| **004** | 3.5 | 0.5 | / | / |
| **005** | 47.5 | 0.7 | 47.4 | 13.8 |
| **006** | 3.3 | 1.1 | / | / |
| **007** | 2.2 | 0.2 | / | / |
| **009** | 7.3 | 7.8 | / | / |
| **010** | 1.2 | 1.7 | / | / |
| **012** | 7.0 | 5.2 | / | / |
| **013** | 1.2 | 0.3 | / | / |
| **014** | 5.3 | 0.2 | / | / |
| **015** | 3.5 | 1.2 | / | / |
| **016** | 12.5 | 0.6 | / | / |
| **018** | 1.1 | 6.9 | / | / |
| **019** | 1.7 | 3.5 | / | / |
| **020** | 1.0 | 14.4 | / | / |
| **022** | 4.2 | 2.3 | / | / |
| **023** | 10.8 | 1.8 | / | / |
| **024** | 1.6 | 0.8 | / | / |

Conclusion: The compounds of the present disclosure show potent inhibitory effect on ALK-mutant cells and TRKB phenotypic cells. Among them, compounds **010, 018, 019, and 020** exhibit stronger inhibition against ALK-mutant cells and relatively weaker inhibition against TRKB phenotypic cells, showing excellent selectivity.

**Experimental example 4: *In vivo* pharmacokinetic testing in mice**

Experimental purpose:

The objective was to study the pharmacokinetic behavior of the compounds of the present disclosure in mice, using male CD-1 mice aged 7 to 9 weeks as test animals. The concentrations of the compounds in plasma at different time points after a single intravenous injection (IV) and oral gavage (PO) administration of the compound were determined using LC/MS/MS.

Experimental methods:

Two groups of healthy mice (fasted) were administered the compounds via intravenous injection (IV) and oral gavage (PO), with 2 mice in each group.

The intravenous solvent for compounds **001, 005, and 006** was 10% dimethyl sulfoxide + 40% polyethylene glycol 400 + 50% water. The test compounds were mixed with the solvent, vortexed, and sonicated to prepare a clear solution with a concentration of 0.5 mg/mL and a pH of 3.3-3.6. The intravenous injection dose for the mice was 1.0 mg/kg. The oral solvent for compounds **001, 005, and 006** was 0.5% sodium carboxymethyl cellulose + 1% polysorbate 80. The test compounds were mixed with the solvent, vortexed, and sonicated to prepare a homogeneous suspension with particles at a concentration of 0.5 mg/mL and a pH of 6.8-7.0. The oral gavage dose for the mice was 5.0 mg/kg.

The intravenous solvent for compound **010** was 10% N,N-dimethylacetamide + 70% polyethylene glycol 400 + 20% (10-hydroxypropyl-β-cyclodextrin aqueous solution). The test compound was mixed with the solvent, vortexed, and sonicated to prepare a clear solution with a concentration of 1.0 mg/mL. The intravenous injection dose for the mice was 1.0 mg/kg. The oral solvent for compound **010** was 5% dimethyl sulfoxide + 60% caprylic/capric triglyceride + 30%

polyethylene glycol 400 + 5% water. The test compound was mixed with the solvent, vortexed, and sonicated to prepare a homogeneous suspension with particles at a concentration of 0.5 mg/mL. The oral gavage dose for the mice was 5.0 mg/kg.

The intravenous solvent for compounds **019 and 020** was 10% dimethyl sulfoxide + 40% polyethylene glycol 400 + 50% water. The test compounds were mixed with the solvent, vortexed, and sonicated to prepare a clear solution with a concentration of 0.1 mg/mL. The intravenous injection dose for the mice was 0.5 mg/kg. The oral solvent for compound **020** was 0.5% sodium carboxymethyl cellulose + 1% polysorbate 80. The test compound was mixed with the solvent, vortexed, and sonicated to prepare a homogeneous suspension with particles at a concentration of 0.5 mg/mL and a pH of 7.08. The oral gavage dose for the mice was 5.0 mg/kg. After administration, the whole blood was collected at the time points of 0.083 hours, 0.25 hours, 0.5 hours, 1.0 hour, 2.0 hours, 4.0 hours, 8.0 hours, 12.0 hours, and 24.0 hours. Plasma was prepared from the whole blood and analyzed for drug concentration by LC-MS/MS. The pharmacokinetic parameters were calculated using Phoenix WinNonlin 6.3. The experimental results are shown in Table 4.

Table 4: Results of pharmacokinetic testing in mice

| Parameter | Compound | 001 | 005 | 006 | 010 | 019 | 020 |
|---|---|---|---|---|---|---|---|
| IV | Half-life $T_{1/2}$ (h) | 6.79 | 2.78 | 1.41 | 0.974 | 1.28 | 2.00 |
| | Apparent volume of distribution Vd (L/kg) | 5.50 | 4.10 | 0.69 | 0.523 | 0.274 | 2.82 |
| | Apparent clearance Cl (mL/min/kg) | 10.4 | 21.8 | 7.4 | 7.10 | 2.40 | 18.5 |
| | Area under the curve $AUC_{0-last}$ (nM. hr) | 3284.0 | 1492.4 | 4776.0 | 5272.8 | 15188.1 | 1704.0 |
| PO | Peak concentration $C_{max}$ (nM) | 900 | 954 | 8764 | 4200 | / | 1890 |
| | Half-life $T_{1/2}$ (h) | 7.79 | 3.82 | 3.44 | 5.56 | / | 2.55 |
| | Area under the curve $AUC_{0-last}$ (nM. hr) | 8395 | 4926.5 | 21116.0 | 20967.5 | / | 11852 |
| | Bioavailability F | 51.1% | 66.0% | 88.9% | 79.5% | / | 139.1% |

Conclusion: The compounds of the present disclosure exhibit a long half-life, high exposure, and high bioavailability in the pharmacokinetics (PK) of mice, demonstrating excellent pharmacokinetic properties.

**Experimental example 5: HMS $CL_{int}$ (liver) test**

Experimental purpose:

The metabolic stability of the compounds of the present disclosure was tested in human and mouse hepatocytes.

Experimental materials:

(1) Test compound (10 mM); Control: 7-ethoxycoumarin (30 mM), 7-hydroxycoumarin (reference substance, 30 mM);

Mouse hepatocytes with cell viability of 73.8%, supplier Cat No.: BioreclamationIVTM005052;
Human hepatocytes with cell viability of 94.6%, supplier Cat No.: Bioreclamation IVTX008001.

(2) Buffer system:
Thawing medium: Williams' medium E containing 5% fetal bovine serum, 30% Percoll solution, and other auxiliary materials.

Incubation medium: Williams' medium E (without phenol red) containing 2 mM L-glutamine and 25 mM 4-(2-hydroxyethyl)-1-piperazineethanesulfonic acid.
Stop solution: acetonitrile containing 200 ng/mL tolbutamide and labetalol as internal standard.
Dilution solution: ultrapure water.

**Experimental method:**

1) An accurate amount of the positive control compound was dissolved in dimethyl sulfoxide (DMSO) to form a 30 mM solution.
2) 10 mM of the test compound and 30 mM of the control compound were diluted into 1 mM and 3 mM, respectively, in a 96-well plate using DMSO.
3) 1 mM of the test compound and 3 mM of the positive control compound were diluted into 100 μM and 300 μM quantitative solutions using acetonitrile.
4) The frozen cells were thawed, separated, and suspended in culture medium, and then diluted to $0.5 \times 10^6$ cells/mL using preheated culture medium.
5) 198 μL of preheated cell suspension was added to a 96-well plate.
6) 100 μL of stop solution (acetonitrile containing 200 ng/mL of tolbutamide and 200 ng/mL of labetalol as internal standards) was transfered in a set of pre-labeled 96-well plates.
7) 2 μL of 100 μM test compound or 300 μM positive control quantification solution was added, in duplicate, to each well of the 96-well plate.
8) T0 samples were mixed for about 1 minute to achieve a uniform suspension, then 20 μL of each sample was immediately transferred into wells containing 100μL of ice-cold stop solution, followed by mixing.
9) All plates were incubated at 37°C in a 95% humidified incubator in 5% $CO_2$, and the reaction was started with a constant shaking at about 600 rpm.
10) At the time points of 15 minutes, 30 minutes, 60 minutes, and 90 minutes, the samples were mixed, and then at each time point, 20 μL of each sample was transferred to the wells containing 100 μL of ice-cold stop solution and mixed.
11) Medium control (MC) sample plates were prepared at T0 and T90 (labeled T0-MC and T90-MC) by adding the same ingredients except the cell suspension to each well. A final concentration table was generated.
12) At each corresponding time point, the reaction was terminated by removing the plate from the incubator and mixing it with 100 μL of ice-cold stop solution.
13) The plate was immediately vortexed and shaken on a plate shaker for 10 minutes at 500 rpm. Then, all sample plates were centrifuged at 3220× g for 20 minutes at 4°C.
14) After centrifugation, the supernatant from the 35 μL/well sample plate was transferred to another set of pre-labeled 96-well plates that contained 70 μL of ultrapure water according to the plate diagram.
15) The analytical plate was sealed and stored at 4°C until LC-MS-MS analysis.

The residual rates of the test compound and the control compound were determined using the following formula:

$$\text{Residual rate (\%)} = \frac{\text{Peak area ratio of compound to internal standard at any time point}}{\text{Peak area ratio of compound to internal standard at 0 minutes}} \times 100\%$$

The elimination rate constant (*k*) of the test compound and the control compound in hepatocytes was calculated by plotting the logarithm of the residual rate against time. The half-life ($T_{1/2}$) and the *in vitro* intrinsic clearance rate (CLint) were obtained from the elimination rate constant *k*, using the following formulas:

$$T_{1/2} = 0.693/k;$$

$$CL_{int\ (hep)} = k \text{ / cells per milliliter (million cells / mL)};$$

$CL_{int\ (liver)} = CL_{int\ (hep)} \times$ ratio of liver weight to body weight $\times$ number of hepatocytes per gram of liver.

The parameters and test results of the compounds of the present disclosure in the HMS test are shown in Table 5.

Table 5 Parameters and test results in HMS testing of compounds of the present disclosure

| Compound | Species | Parameters | | | HMS **CLint** (mL/min/kg) |
|---|---|---|---|---|---|
| | | Ratio of liver weight to body weight (g/kg) | Hepatic blood flow (Qh) (mL/min/kg) | Number of hepatocytes (Number of cells per gram of liver) | |
| **018** | Mouse | 88 | 90.0 | $135 \times 10^6$ | 290.9 |
| | Human | 20 | 20.7 | $139 \times 10^6$ | <17.8 |

Conclusion: The compounds of the present disclosure exhibit moderate metabolism in human hepatocytes and faster metabolism in mice, demonstrating good stability.

**Experimental example 6: Cytochrome P450 isozyme inhibitory activity test**

Experimental purpose:

To test the inhibitory activity of the compounds of the present disclosure on different subtypes of human liver microsomal cytochrome P450 isozymes.

Experimental materials:

Mixed human liver microsomes (HLM) purchased from Corning Inc. (Steuben, New York, USA); Diclofenac as the probe substrate (10 μM); Sulfaphenazole as the positive control inhibitor; 4'-Hydroxydiclofenac as the metabolite

Preparation of test system working solution: Human liver microsomes (HLM): HLM stock solution at 20.0 mg/mL, working solution at 0.2 mg/mL, final concentration at 0.1 mg/mL; Potassium phosphate buffer: 100 mM; Cofactor working solution: Magnesium chloride, NADP (nicotinamide adenine dinucleotide phosphate), G6P (glucose-6-phosphate), G6PDH (glucose-6-phosphate dehydrogenase), and phosphate buffer or magnesium chloride, NADPH (reduced nicotinamide adenine dinucleotide phosphate) powder, and potassium phosphate buffer; prepared into cofactor working solution at appropriate ratios;

Stop solution: Isotope-labeled 4'-hydroxydiclofenac-$d_4$ stock solution prepared in methanol, then prepared into stop solution using acetonitrile.

Experimental methods:

In the substrate-free control sample wells of the reaction plate, 100 μL of a mixture of human liver microsome and potassium phosphate buffer was added. At the zero-time point sample wells, 98 μL of a mixture of the human liver microsome and potassium phosphate buffer was added. In the no-inhibitor control (NIC), test compound, and positive control inhibitor sample wells of the reaction plate, 100 μL of a mixture of the human liver microsome and substrate was added. From the dilution plate, 2 μL of blank solvent, test compound working solution (three parallels), and positive control inhibitor working solution (two parallels) were added to the reaction plate. The reaction plate was preheated at 37.0°C for 10 minutes. Then, 98 μL of the cofactor working solution was added to the reaction plate to start the reaction. After the respective CYP enzyme reaction times were reached, 200 μL of stop solution was added to the reaction plate to stop the

reaction. To the zero-time point sample wells, 4 μL of 5.0% substrate solution was added. The sample plate was centrifuged in a centrifuge at 3220× g for 20 minutes. The supernatant was removed and diluted with an appropriate dilution solution in an appropriate proportion, and the plate was shaken to mix evenly. The concentration of specific metabolites in the samples was detected using the liquid chromatography-tandem mass spectrometry (LC-MS/MS) method. Non-linear regression analysis of the average percentage activity of the test substance against its concentration was conducted using SigmaPlot (V.11). The $IC_{50}$ values were calculated using a three-parameter or four-parameter inverse log equation. The experimental results are shown in Table 6.

Table 6. Inhibitory results of the compounds of the present disclosure on P450 isozymes

| Compound | $IC_{50}$ (μM) | | | | |
|---|---|---|---|---|---|
| | CYP1A2 | CYP2C9 | CYP2C19 | CYP2D6 | CYP3A4-M |
| **004** | > 50 | 0.69 | 5.44 | 32.2 | 8.24 |
| **016** | >50 | 3.12 | 22.7 | >50 | >50 |
| **022** | / | 0.46 | / | / | > 50 |
| **024** | / | 2.12 | / | / | > 50 |

Conclusion: The compounds of the present disclosure have no significant inhibition on different subtypes of P450 isozymes.

**Experimental example 7: Plasma protein binding rate (PPB) Test**

Experimental purpose:

To determine the protein binding rate of the compounds of the present disclosure in CD-1 mouse and human plasma using equilibrium dialysis.

Experimental methods:

Plasma samples of the compounds were prepared using CD-1 mouse and human plasma, with a compound concentration of 2.00 μM. The samples were placed in a 96-well equilibrium dialysis device and dialyzed with phosphate buffer solution at 37°C for 6 hours. Warfarin was used as the control compound in this experiment. The concentrations of the test compounds in plasma and dialysis buffer were determined using the LC-MS/MS method, and the unbound rate (% Unbound), binding rate (% Bound), and recovery rate (% Recovery) were calculated. The calculation formulas were as follows: % Unbound = 100 * $F_C$ / $T_C$; % Bound = 100 - % Unbound; % Recovery = 100 * ($F_C$ + $T_C$) / $T_0$, where $F_C$ is the concentration of the compound at the buffer side of the dialysis plate; $T_C$ is the concentration of the compound at the plasma side of the dialysis plate; and $T_0$ is the concentration of the compound in the plasma sample at zero time.

Conclusion of the experiment: The compounds exhibit a high degree of binding rate in both CD-1 mouse and human plasma.

## Claims

1. A compound of formula (II) or a pharmaceutically acceptable salt thereof,

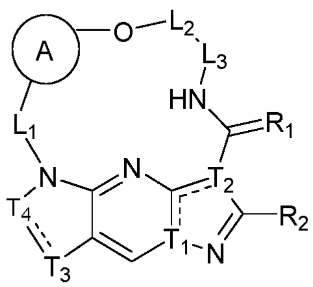

(II) ,

wherein

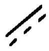 is selected from a single bond and a double bond;
$T_1$ is selected from C, and $T_2$ is selected from N;
or, $T_1$ is selected from N, and $T_2$ is selected from C;
$T_3$ is selected from O, N, NH, C(=O), $C(R_3)_2O$, $C(R_3)$, and $C(R_3)_2$;
$T_4$ is selected from N, NH, C(=O), $C(R_3)_2O$, $C(R_3)$, and $C(R_3)_2$;
$L_1$ is selected from $CH_2$ and C(=O);
$L_2$ and $L_3$ are each independently selected from a single bond and $C_{1-3}$ alkyl, and the $C_{1-3}$ alkyl is optionally substituted with 1, 2, or 3 $R_a$;
$R_1$ is selected from O, S, and $NR_4$;
$R_2$ is selected from H, F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
$R_3$ is selected from H, F, Cl, Br, I, and $C_{1-3}$ alkyl, and the $C_{1-3}$ alkyl is optionally substituted with 1, 2, or 3 $R_b$;
$R_4$ is selected from OH, CN, and $-(OCH_2CH_2)_n-C_{1-3}$ alkoxy;
n is 0 or 1;
ring A is selected from phenyl, 5- to 6-membered heteroaryl, and $C_{5-6}$ cycloalkenyl, and the phenyl, 5- to 6-membered heteroaryl, and $C_{5-6}$ cycloalkenyl are each independently and optionally substituted with 1, 2, or 3 $R_c$;
each $R_a$ and each $R_b$ are independently selected from H, F, Cl, Br, I, and OH;
or, two $R_a$ on the same carbon atom are connected to form a $C_{3-6}$ cycloalkyl group or a 4-to 6-membered heterocycloalkyl group, and the $C_{3-6}$ cycloalkyl and the 4- to 6-membered heterocycloalkyl are each independently and optionally substituted with 1, 2, or 3 halogens;
or, two $R_a$ on different carbon atoms are connected to form a $C_{3-6}$ cycloalkyl group, and the $C_{3-6}$ cycloalkyl is optionally substituted with 1, 2, or 3 halogens;
each $R_c$ is independently selected from H, F, Cl, Br, I, and $C_{1-3}$ alkyl, and the $C_{1-3}$ alkyl is optionally substituted with 1, 2, or 3 halogens;
each $R_a$ is independently selected from H, F, Cl, Br, I, OH, $NH_2$, $C_{1-3}$ alkyl, and $C_{3-6}$ cycloalkyl, and the $C_{1-3}$ alkyl and $C_{3-6}$ cycloalkyl are each independently and optionally substituted with 1, 2, or 3 halogens;
provided that: when $T_4$ is selected from $C(R_3)_2$, $T_3$ is selected from $C(R_3)_2O$, $T_2$ is selected from C, $T_1$ is selected from N, $L_1$ is selected from $CH_2$, ring A is selected from phenyl optionally substituted with 1, 2, or 3 $R_c$ or 5- to 6-membered heteroaryl optionally substituted with 1, 2, or 3 $R_c$, and $R_1$ is selected from O, then $R_2$ is selected from F, Cl, Br, I, OH, $NH_2$, CN, $C_{1-4}$ alkyl, $C_{2-5}$ alkenyl, $C_{2-5}$ alkynyl, and $C_{3-6}$ cycloalkyl, and the $C_{1-4}$ alkyl, $C_{2-5}$ alkenyl, $C_{2-5}$ alkynyl, and $C_{3-6}$ cycloalkyl are each independently and optionally substituted with 1, 2, or 3 $R_d$;
"hetero" in the 5- to 6-membered heteroaryl or 4- to 6-membered heterocycloalkyl represents 1, 2, or 3 heteroatoms or heteroatom groups independently selected from O, NH, S, S(=O), N, and C(=O).

**2.** The compound or the pharmaceutically acceptable salt thereof according to claim 1, wherein each $R_c$ is independently selected from H, F, Cl, Br, I, and $CH_3$, and the $CH_3$ is optionally substituted with 1, 2, or 3 halogens; or each $R_c$ is independently selected from H, F, Cl, Br, I, $CH_3$, and $CF_3$.

**3.** The compound or the pharmaceutically acceptable salt thereof according to claim 1, wherein each $R_d$ is independently selected from H, F, OH, $NH_2$, $CH_3$, and cyclopropyl.

**4.** The compound or the pharmaceutically acceptable salt thereof according to claim 1, wherein $L_2$ and $L_3$ are each independently selected from a single bond, $CH_2$, $CH_2CH_2$, and $C(CH_3)_2$, and the $CH_2$, $CH_2CH_2$, and $C(CH_3)_2$ are each independently and optionally substituted with 1, 2, or 3 $R_a$; or, $L_2$ and $L_3$ are each independently selected from a single bond, $CH_2$, $CF_2$, $CH_2CH_2$, $C(CH_3)_2$, $C(CF_3)_2$,

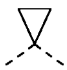, 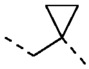, 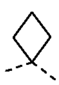, 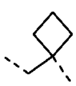, 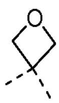, 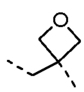, and 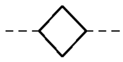.

**5.** The compound or the pharmaceutically acceptable salt thereof according to claim 1, wherein the structural moiety $-L_2-L_3-$ is selected from $-CH_2C(CH_3)_2-$,

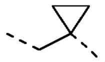, 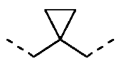,

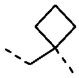, 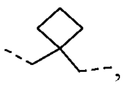, 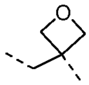, and 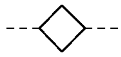.

6. The compound or the pharmaceutically acceptable salt thereof according to claim 1, wherein $R_1$ is selected from O, NOH, NCN, $NOCH_3$, $NOCH_2CH_3$, and $NOCH_2CH_2OCH_3$.

7. The compound or the pharmaceutically acceptable salt thereof according to claim 1, wherein $R_2$ is selected from H, F, Cl, Br, I, OH, $NH_2$, CN, $CH_3$, cyclopropyl, 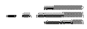, and 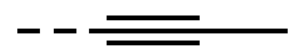, and the $CH_3$, cyclopropyl, 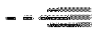, and  are each independently and optionally substituted with 1, 2, or 3 $R_d$; or, $R_2$ is selected from H, F, Cl, Br, I, OH, $NH_2$, CN, $CH_3$, 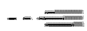,

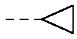, 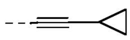,

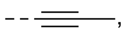, and

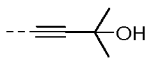.

8. The compound or the pharmaceutically acceptable salt thereof according to claim 1, wherein $R_3$ is selected from H, F, Cl, Br, I, $CH_3$, $CH_2F$, $CHF_2$, $CF_3$, and

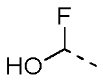;

or $R_3$ is selected from H, $CH_3$, and $CHF_2$.

9. The compound or the pharmaceutically acceptable salt thereof according to claim 1, wherein $T_3$ is selected from O, $CH_2O$, CH, and $C(CH_3)$.

10. The compound or the pharmaceutically acceptable salt thereof according to claim 1, wherein $T_4$ is selected from $CH(CHF_2)$, N, and C(=O).

11. The compound or the pharmaceutically acceptable salt thereof according to claim 1, wherein ring A is selected from phenyl, pyridyl, and cyclohexenyl, and the phenyl, pyridyl, and cyclohexenyl are each independently and optionally substituted with 1, 2, or 3 $R_c$; or ring A is selected from

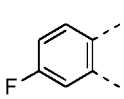, 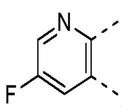, 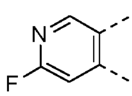, and 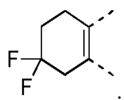.

12. The compound or the pharmaceutically acceptable salt thereof according to claim 1, wherein the structural moiety

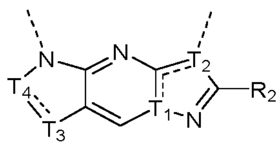

is selected from

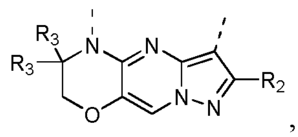,

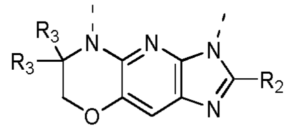, 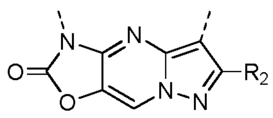, 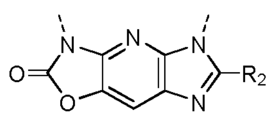, 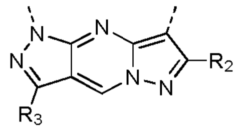,

and

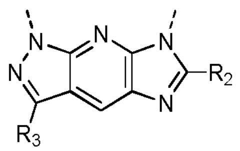.

**13.** The compound or the pharmaceutically acceptable salt thereof according to claim 12, wherein the structural moiety

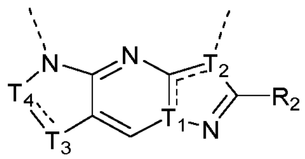

is selected from

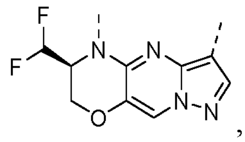,

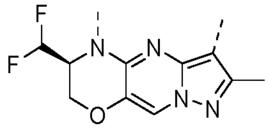, 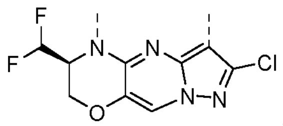, 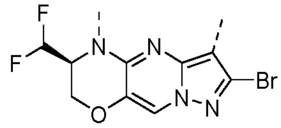, 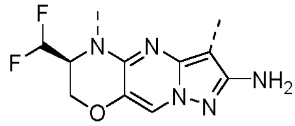,

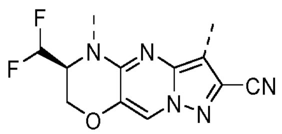, 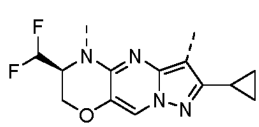, 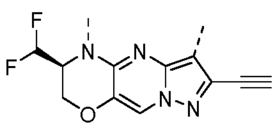, 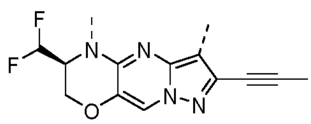,

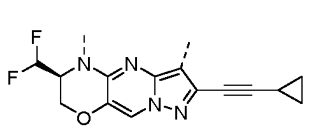, 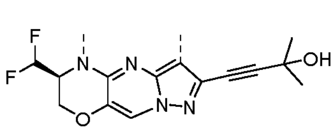, 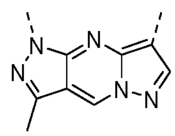, 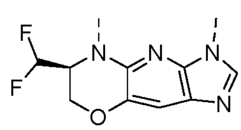,

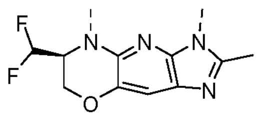, 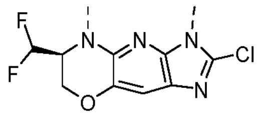, 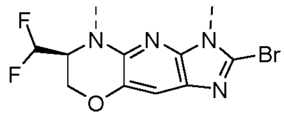, 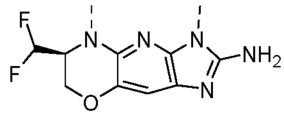,

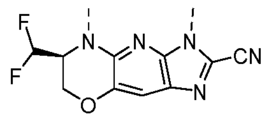, and 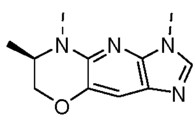.

**14.** The compound or the pharmaceutically acceptable salt thereof according to any one of claims 1 to 13, selected from:

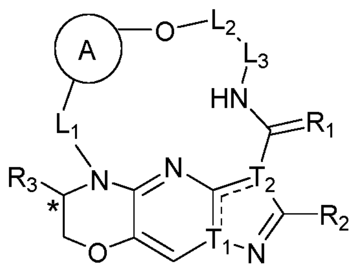

( II-1 ) ,

wherein

$R_1$, $R_2$, $R_3$, $T_1$, $T_2$, $L_1$, $L_2$, $L_3$, ring A, and 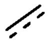 are as defined in any one of claims 1 to 13;
when $R_3$ is not H, the carbon atom with "*" is a chiral carbon atom, which exists in an (R) or (S) single enantiomer form or an (R) or (S) single enantiomer-rich form.

**15.** The compound or the pharmaceutically acceptable salt thereof according to claim 14, selected from:

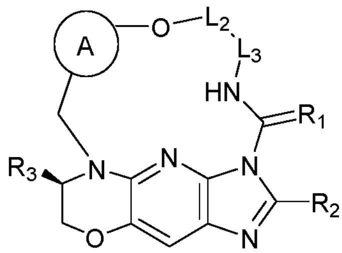

( III-1 ) and

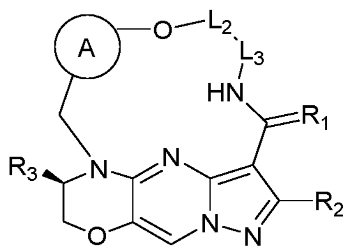

( P-1 ) ,

wherein

$R_3$ is selected from F, Cl, Br, I, and $C_{1-3}$ alkyl, and the $C_{1-3}$ alkyl is optionally substituted with 1, 2, or 3 $R_b$;
$R_1$, $R_2$, $L_2$, $L_3$, ring A, and each $R_b$ are as defined in claim 14.

**16.** A compound as shown below or a pharmaceutically acceptable salt thereof,

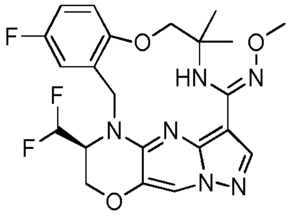
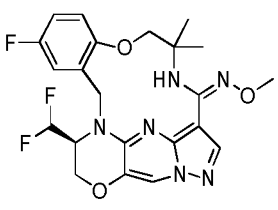
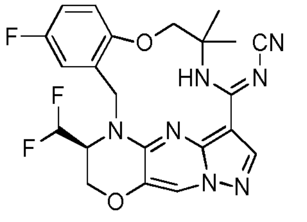
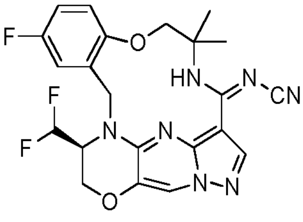

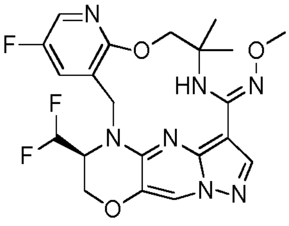
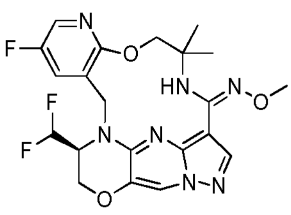
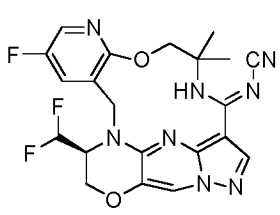
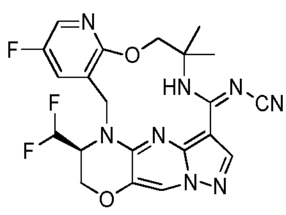

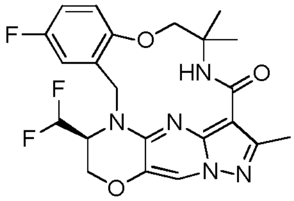
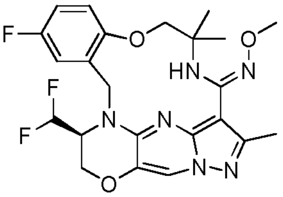
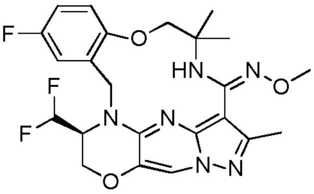
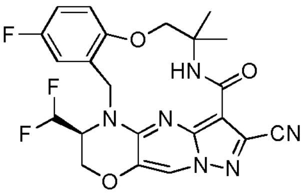

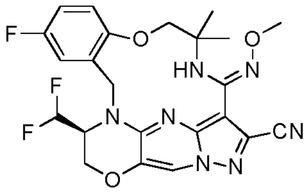
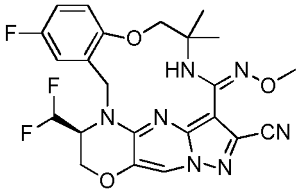
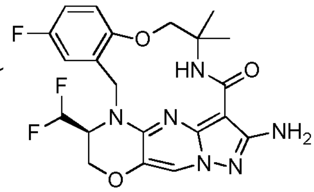
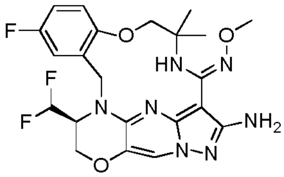

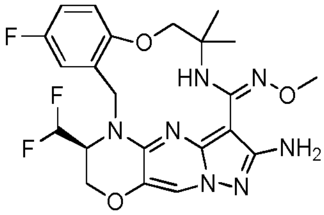
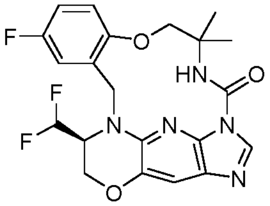
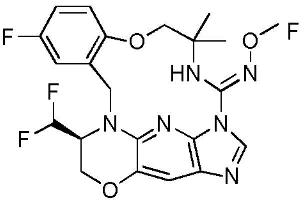
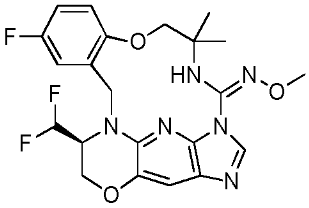

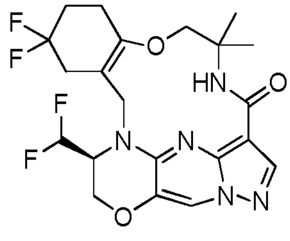
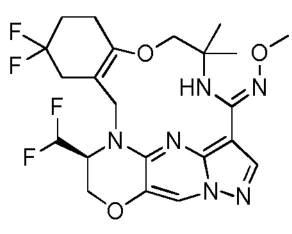
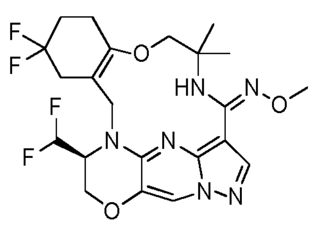
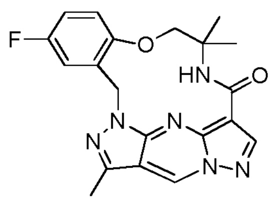

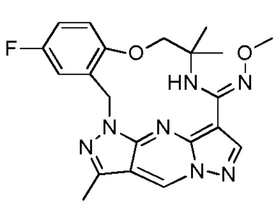
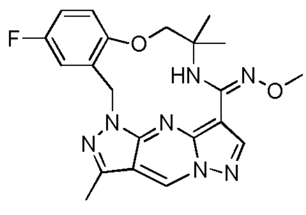
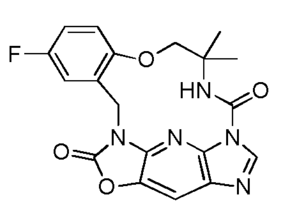
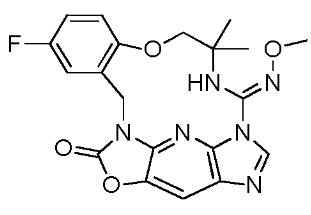

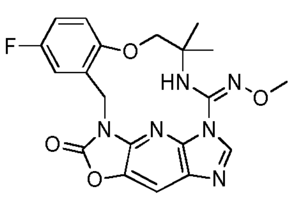
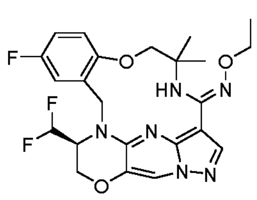
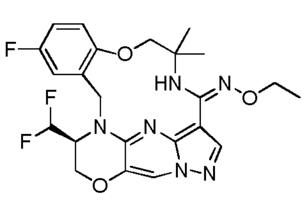
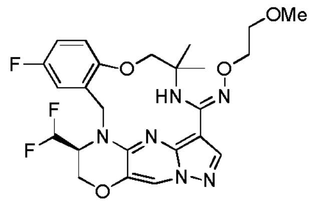

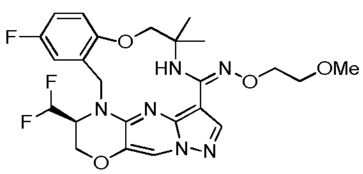
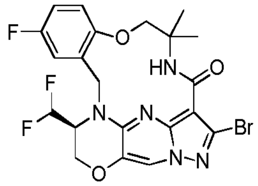
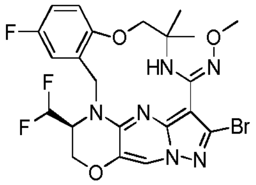
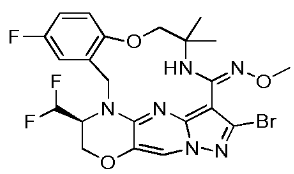

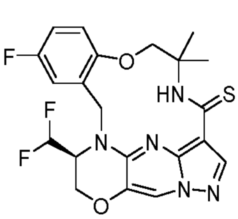
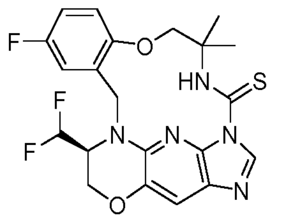
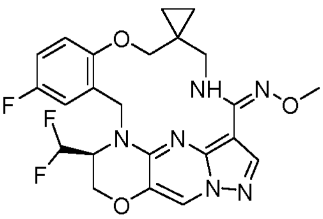
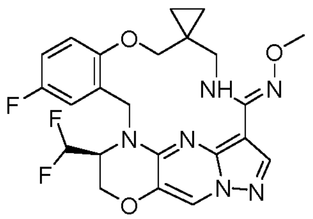

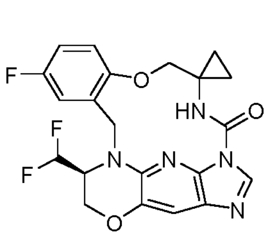
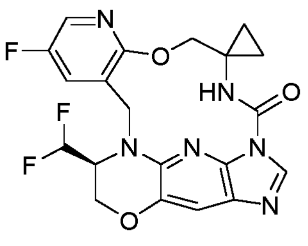
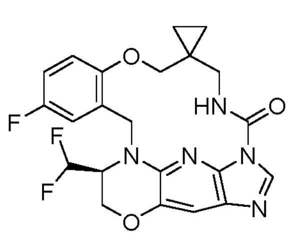
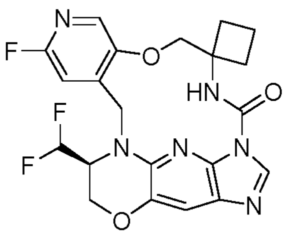

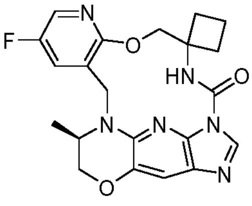
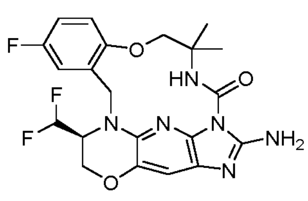
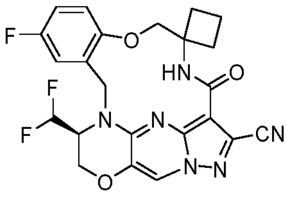
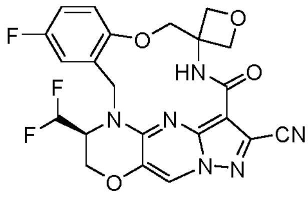

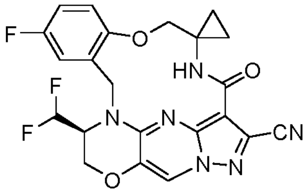
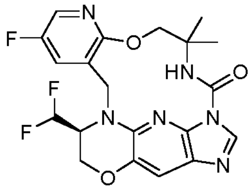
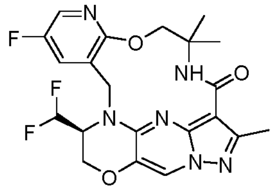
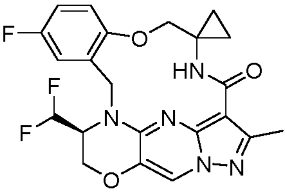

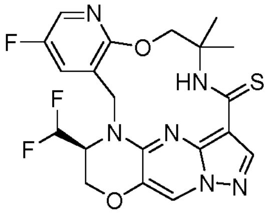
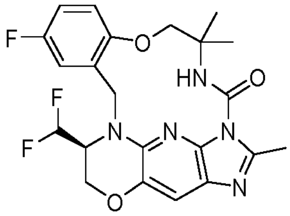
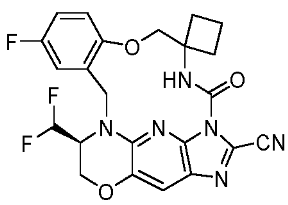
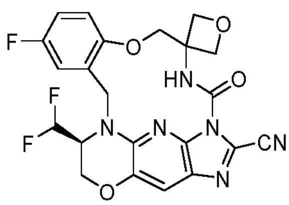

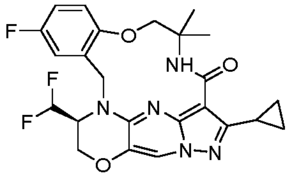
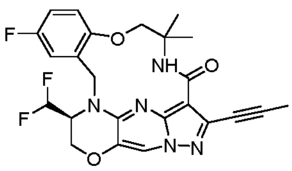
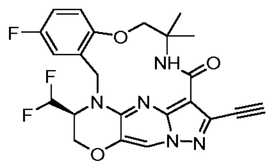
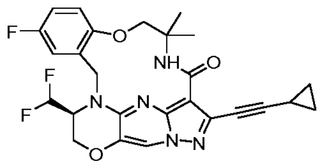
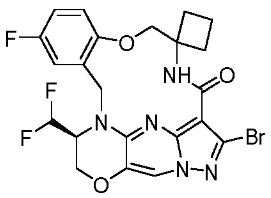
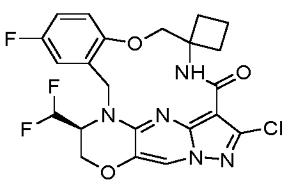
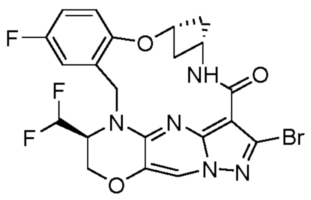
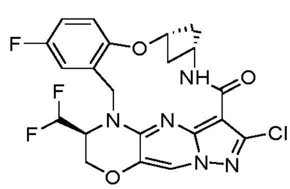
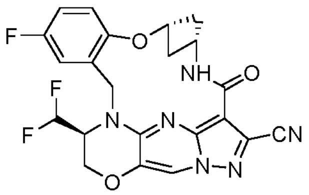
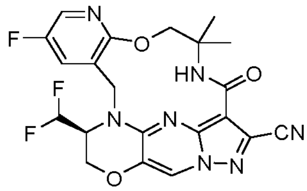
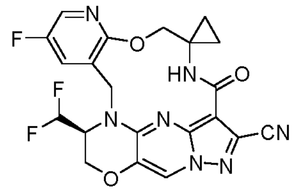
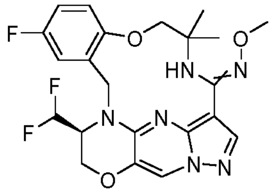
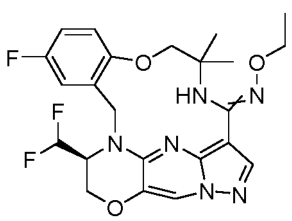
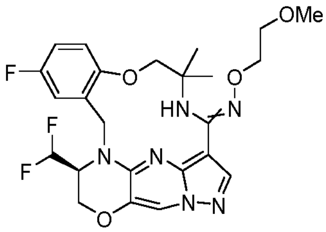
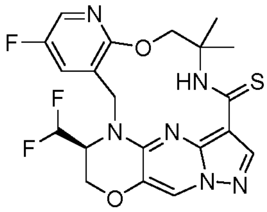
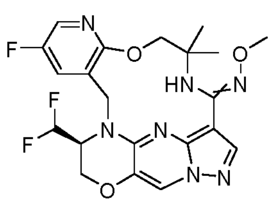
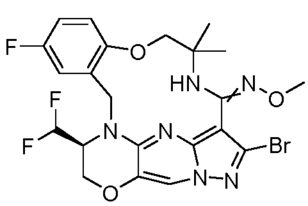
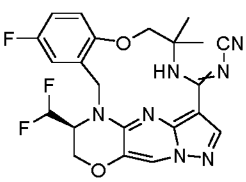
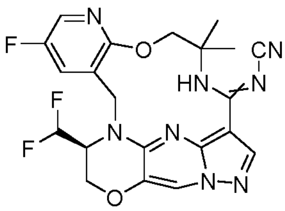
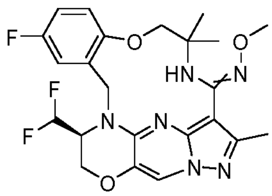
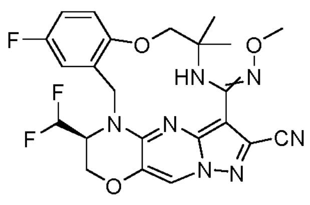
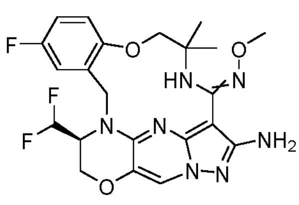
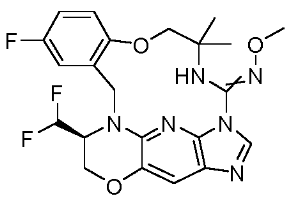
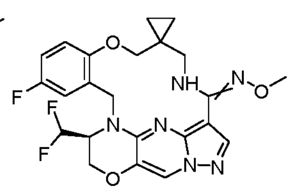
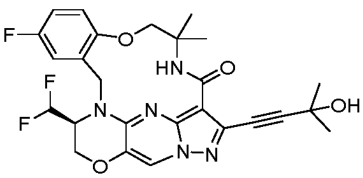

.

**17.** Use of the compound or the pharmaceutically acceptable salt thereof according to any one of claims 1 to 16 in the manufacture of a medicament for treating ALK inhibitor-related diseases.

**INTERNATIONAL SEARCH REPORT**

International application No.
**PCT/CN2023/108708**

**A. CLASSIFICATION OF SUBJECT MATTER**

C07D498/14(2006.01)i; C07D491/18(2006.01)i; A61K31/5383(2006.01)i; A61K31/5386(2006.01)i; A61P35/00(2006.01)n

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNABS, CNTXT, VEN, USTXT, EPTXT, WOTXT, CNKI, Registry, Caplus: 明德新药, 氧杂, 氮杂, 吡唑, 嘧啶, 噁嗪, 咪唑, 酮, ALK激酶, oxa, aza, pyrazol, pyrimid, oxazin, imidazol, ketone, ALK, anaplastic lymphoma kinase.

**C. DOCUMENTS CONSIDERED TO BE RELEVANT**

| Category* | Citation of document, with indication, where appropriate, of the relevant passages | Relevant to claim No. |
|---|---|---|
| X | WO 2021113339 A1 (TURNING POINT THERAPEUTICS, INC.) 10 June 2021 (2021-06-10)<br>description, paragraphs 8-19, 193, and 270-283 | 1-17 |
| X | WO 2019126122 A1 (TP THERAPEUTICS, INC.) 27 June 2019 (2019-06-27)<br>description, paragraphs 60-94, 305, 576, and 580-589 | 1-17 |
| X | CN 111511746 A (TP THERAPEUTICS, INC.) 07 August 2020 (2020-08-07)<br>description, paragraphs 42-55, 157, and 514-549 | 1-17 |
| A | CN 112812128 A (CHIA TAI TIANQING PHARMACEUTICAL GROUP CO., LTD.) 18 May 2021 (2021-05-18)<br>entire document | 1-17 |
| A | US 2022033402 A1 (DANA-FARBER CANCER INSTITUTE, INC.) 03 February 2022 (2022-02-03)<br>entire document | 1-17 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2023** | **30 October 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/108708**

| Patent document cited in search report | Publication date (day/month/year) | Patent family member(s) | Publication date (day/month/year) |
|---|---|---|---|
| WO 2021113339 A1 | 10 June 2021 | CO 2022008817 A2 | 20 September 2022 |
| | | BR 112022010702 A2 | 04 October 2022 |
| | | CA 3163735 A1 | 10 June 2021 |
| | | TW 202134249 A | 16 September 2021 |
| | | AU 2020395136 A1 | 07 July 2022 |
| | | US 2021163499 A1 | 03 June 2021 |
| | | US 11634433 B2 | 25 April 2023 |
| | | KR 20220133869 A | 05 October 2022 |
| | | EP 4069700 A1 | 12 October 2022 |
| | | EP 4069700 A4 | 17 May 2023 |
| | | US 2021246145 A1 | 12 August 2021 |
| | | US 11142533 B2 | 12 October 2021 |
| | | IL 293458 A | 01 July 2022 |
| | | PE 20221485 A1 | 26 September 2022 |
| | | JP 2023504523 A | 03 February 2023 |
| WO 2019126122 A1 | 27 June 2019 | TW 201930313 A | 01 August 2019 |
| | | US 2021087206 A1 | 25 March 2021 |
| | | EP 3728270 A1 | 28 October 2020 |
| | | EP 3728270 A4 | 23 June 2021 |
| CN 111511746 A | 07 August 2020 | PT 3728271 T | 06 October 2022 |
| | | JP 2023027237 A | 01 March 2023 |
| | | US 2020190110 A1 | 18 June 2020 |
| | | US 10745416 B2 | 18 August 2020 |
| | | SI 3728271 T1 | 31 January 2023 |
| | | AU 2018392332 A1 | 11 June 2020 |
| | | AU 2018392332 B2 | 03 August 2023 |
| | | RS 63787 B1 | 30 December 2022 |
| | | HUE 060711 T2 | 28 April 2023 |
| | | KR 20200101358 A | 27 August 2020 |
| | | US 2022306652 A1 | 29 September 2022 |
| | | EP 3728271 A1 | 28 October 2020 |
| | | EP 3728271 A4 | 23 June 2021 |
| | | EP 3728271 B1 | 28 September 2022 |
| | | CA 3083674 A1 | 27 June 2019 |
| | | LT 3728271 T | 12 December 2022 |
| | | WO 2019126121 A1 | 27 June 2019 |
| | | TW 201930312 A | 01 August 2019 |
| | | PH 12020550901 A1 | 17 May 2021 |
| | | PL 3728271 T3 | 23 January 2023 |
| | | DK 3728271 T3 | 19 December 2022 |
| | | IL 275265 A | 30 July 2020 |
| | | IL 275265 B1 | 01 March 2023 |
| | | IL 275265 B2 | 01 July 2023 |
| | | BR 112020012319 A2 | 24 November 2020 |
| | | US 2020392160 A1 | 17 December 2020 |
| | | US 11286265 B2 | 29 March 2022 |
| | | EP 4151641 A1 | 22 March 2023 |
| | | SG 11202005590 PA | 29 July 2020 |
| | | CL 2020001632 A1 | 20 November 2020 |
| | | JOP 20200152 A1 | 16 June 2020 |

Form PCT/ISA/210 (patent family annex) (July 2022)

**INTERNATIONAL SEARCH REPORT**
**Information on patent family members**

International application No.
**PCT/CN2023/108708**

| Patent document cited in search report | Publication date (day/month/year) | Patent family member(s) | Publication date (day/month/year) |
|---|---|---|---|
| | | MX 2020006490 A | 17 August 2020 |
| | | ES 2929467 T3 | 29 November 2022 |
| | | ECSP 20033467 A | 30 September 2020 |
| | | HRP 20221502 T1 | 31 March 2023 |
| | | JP 2021506850 A | 22 February 2021 |
| | | JP 7194188 B2 | 21 December 2022 |
| CN 112812128 A | 18 May 2021 | None | |
| US 2022033402 A1 | 03 February 2022 | WO 2020069118 A1 | 02 April 2020 |
| | | EP 3856745 A1 | 04 August 2021 |
| | | EP 3856745 A4 | 08 June 2022 |
| | | CA 3113065 A1 | 02 April 2020 |
| | | AU 2019346595 A1 | 18 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022108721559
- CN 2022113690989
- CN 2022116017524
- CN 2023102194528
- CN 202310562000X